(19) Europäisches Patentamt
European Patent Office
Office européen des brevets

(11) EP 4 624 513 A2

(12) EUROPEAN PATENT APPLICATION

(43) Date of publication:
**01.10.2025 Bulletin 2025/40**

(21) Application number: **25195866.6**

(22) Date of filing: **29.06.2023**

(51) International Patent Classification (IPC):
***C08G 63/197*** *(2006.01)*

(52) Cooperative Patent Classification (CPC):
(C-Sets available)
**G02B 1/041; C08G 63/183; C08G 63/197; C08G 63/672; C08G 63/6886; C08G 64/06; C08G 64/081; C08G 64/307** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **05.07.2022 KR 20220082381**
**05.07.2022 KR 20220082388**
**05.07.2022 KR 20220082390**
**05.07.2022 KR 20220082391**

(62) Document number(s) of the earlier application(s) in accordance with Art. 76 EPC:
**23835744.6 / 4 382 550**

(71) Applicant: **LG CHEM, LTD.**
**Seoul 07336 (KR)**

(72) Inventors:
- **BAEK, Jonghwa**
  **34122 Daejeon (KR)**
- **CHOI, Il Hwan**
  **34122 Daejeon (KR)**
- **BAE, Jaesoon**
  **34122 Daejeon (KR)**
- **JUNG, Min Suk**
  **34122 Daejeon (KR)**
- **JUNG, Byeongyeon**
  **34122 Daejeon (KR)**

(74) Representative: **Hoffmann Eitle**
**Patent- und Rechtsanwälte PartmbB**
**Arabellastraße 30**
**81925 München (DE)**

Remarks:
This application was filed on 14.08.25 as a divisional application to the application mentioned under INID code 62.

# (54) RESIN AND METHOD FOR MANUFACTURING SAME

(57) The present application relates to a resin comprising a unit of Chemical Formula 1, a method for preparing the same, a resin composition comprising the same, and a molded article comprising the resin composition.

EP 4 624 513 A2

(52) Cooperative Patent Classification (CPC): (Cont.)

C-Sets
**G02B 1/041, C08L 67/02;**
**G02B 1/041, C08L 69/00**

## Description

[Technical Field]

This application claims priority to and the benefit of Korean Patent Application Nos. 10-2022-0082381, 10-2022-0082388, 10-2022-0082390 and 10-2022-0082391 filed in the Korean Intellectual Property Office on July 5, 2022, July 5, 2022, July 5, 2022, and July 5, 2022, respectively, the entire contents of which are incorporated herein by reference.
The present specification relates to a resin and a method for preparing the same.

[Background Art]

The higher the refractive index of an optical material, the thinner the optical lens required to achieve the same level of correction. Accordingly, as the refractive index of the optical material is increased, a thinner and lighter lens can be manufactured, so that it is possible to make various devices, where lenses are used, smaller.
Generally, when the refractive index of an optical material is increased, there is a problem in that the Abbe's Number becomes low, and for use as an optical material, a certain level or higher of transparency is required.

[Detailed Description of the Invention]

[Technical Problem]

An exemplary embodiment of the present specification has been made in an effort to provide a resin having a novel structure and a method for preparing the same.
Another exemplary embodiment of the present specification has been made in an effort to provide a composition comprising a resin having a novel structure and a molded article prepared from the resin composition.

[Technical Solution]

An exemplary embodiment of the present specification provides a resin comprising a unit of the following Chemical Formula 1.

[Chemical Formula 1]

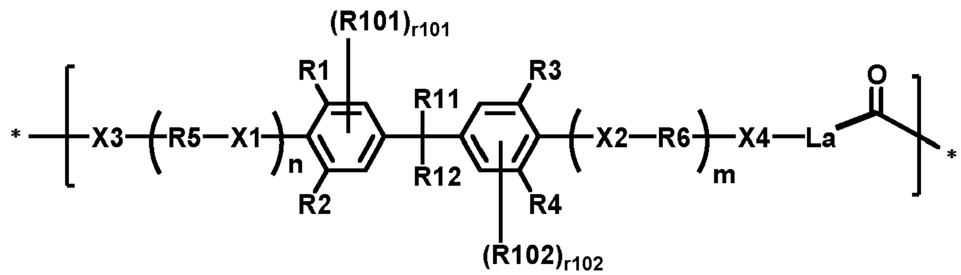

In Chemical Formula 1,

X1 to X4 are the same as or different from each other, and are each independently O or S,
at least one of R1 to R4 is a substituted or unsubstituted alkyl group; a substituted or unsubstituted aryl group; or a substituted or unsubstituted heteroaryl group, and the others are each independently hydrogen; a substituted or unsubstituted alkyl group; a substituted or unsubstituted aryl group; or a substituted or unsubstituted heteroaryl group,
R5 and R6 are the same as or different from each other, and are each independently a substituted or unsubstituted alkylene group; or a substituted or unsubstituted cycloalkylene group,
R11 and R12 are the same as or different from each other, and are each independently a substituted or unsubstituted alkyl group; a substituted or unsubstituted cycloalkyl group; a substituted or unsubstituted aryl group; or a substituted or unsubstituted heteroaryl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,

r101 is 1 or 2, and when r101 is 2, two R101's are the same as or different from each other,
r102 is 1 or 2, and when r102 is 2, two R102's are the same as or different from each other,
La is a direct bond; or -C(=O)-L'-,
L' is a substituted or unsubstituted arylene group,
m and n are each 0 or 1, and
* means a moiety linked to the main chain of the resin.

An exemplary embodiment of the present specification provides the following Chemical Formula 1a.

[Chemical Formula 1a]

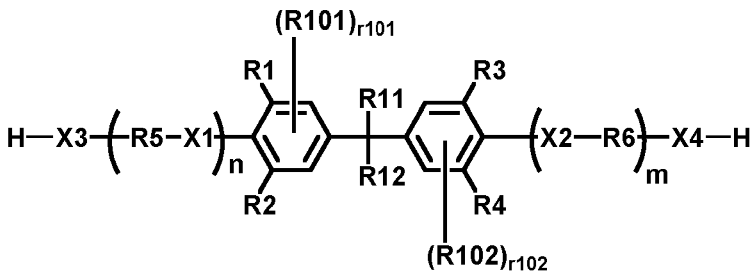

In Chemical Formula 1a,

X1 to X4 are the same as or different from each other, and are each independently O or S,
at least one of R1 to R4 is a substituted or unsubstituted alkyl group; a substituted or unsubstituted aryl group; or a substituted or unsubstituted heteroaryl group, and the others are each independently hydrogen; a substituted or unsubstituted alkyl group; a substituted or unsubstituted aryl group; or a substituted or unsubstituted heteroaryl group,
R5 and R6 are the same as or different from each other, and are each independently a substituted or unsubstituted alkylene group; or a substituted or unsubstituted cycloalkylene group,
R11 and R12 are the same as or different from each other, and are each independently a substituted or unsubstituted alkyl group; a substituted or unsubstituted cycloalkyl group; a substituted or unsubstituted aryl group; or a substituted or unsubstituted heteroaryl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other, and
m and n are each 0 or 1.

Another exemplary embodiment of the present specification provides a method for preparing a resin, the method comprising: polymerizing a composition for preparing a resin, which comprises a compound represented by the following Chemical Formula 1a; and a polyester precursor or a polycarbonate precursor.

[Chemical Formula 1a]

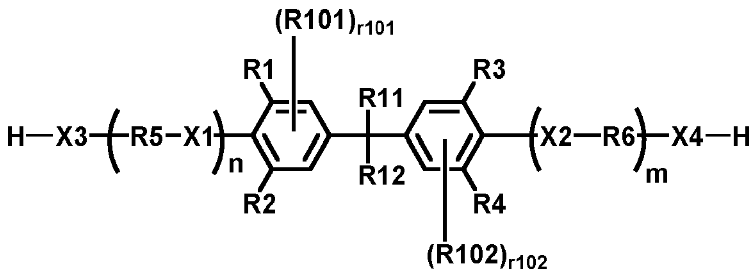

In Chemical Formula 1a,

X1 to X4 are the same as or different from each other, and are each independently O or S,
at least one of R1 to R4 is a substituted or unsubstituted alkyl group; a substituted or unsubstituted aryl group; or a

substituted or unsubstituted heteroaryl group, and the others are each independently hydrogen; a substituted or unsubstituted alkyl group; a substituted or unsubstituted aryl group; or a substituted or unsubstituted heteroaryl group,
R5 and R6 are the same as or different from each other, and are each independently a substituted or unsubstituted alkylene group; or a substituted or unsubstituted cycloalkylene group,
R11 and R12 are the same as or different from each other, and are each independently a substituted or unsubstituted alkyl group; a substituted or unsubstituted cycloalkyl group; a substituted or unsubstituted aryl group; or a substituted or unsubstituted heteroaryl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other, and
m and n are each 0 or 1.

Another exemplary embodiment of the present specification provides a resin composition comprising the resin according to the above-described exemplary embodiment.
Still another exemplary embodiment of the present specification provides a molded article comprising the composition comprising the resin according to the above-described exemplary embodiment.

[Advantageous Effects]

The resin according to the exemplary embodiments of the present specification has a high refractive index and high transparency.
By using the resin according to the exemplary embodiments of the present specification, an excellent optical member, optical lens, optical film, optical thin film, or optical resin having a small thickness can be obtained.

[Best Mode]

Hereinafter, the present specification will be described in more detail.
For a resin comprising the unit of Chemical Formula 1 according to an exemplary embodiment of the present specification, from the relationship formula between the molecular structure and the refractive index, which is known by the Lorentz-Lorenz's formula, it can be seen that the refractive index of a material composed of molecules is increased by increasing the electron density of the molecule and reducing the molecular volume. Further, since the core structure of Chemical Formula 1 is a derivative of bisphenol A (BPA), the molecular volume is small and the ability to pack is excellent, and thus, the refractive index of the resin may be improved. In addition, since R1 to R4 are a substituent rich in electrons such as a substituted or unsubstituted aryl group; or a substituted or unsubstituted heteroaryl group, the refractive index of the resin may be further improved by increasing the electron density of the structure represented by Chemical Formula 1. Therefore, the resin according to an exemplary embodiment of the present specification has a high refractive index and high transparency, and an optical lens, an optical film, or an optical resin using the resin has a small thickness and may exhibit excellent optical characteristics.
In an exemplary embodiment of the present invention, one or more of the units of Chemical Formula 1 can be comprised in the resin, and when two or more are comprised, the units are the same as or different from each other.
Throughout the specification of the present application, the term "combination thereof" comprised in the Markush type expression means a mixture or combination of one or more selected from the group consisting of constituent elements described in the Markush type expression, and means comprising one or more selected from the group consisting of the above-described constituent elements.
Examples of the substituents in the present specification will be described below, but are not limited thereto.
In the present specification,

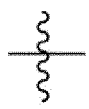

means a moiety to be linked.
In the present specification, the term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more are substituted, the two or more substituents may be the same as or different from each other.

In the present specification, the term "substituted or unsubstituted" means being substituted with one or more substituents selected from the group consisting of deuterium; a halogen group; a hydroxyl group; a cyano group; an alkyl group; a cycloalkyl group; an alkoxy group; an alkenyl group; an aryloxy group; an arylthio group; alkylthio group; a silyl group; an aryl group; a condensed ring group of an aromatic hydrocarbon ring and an aliphatic hydrocarbon ring; and a heteroaryl group, being substituted with a substituent to which two or more substituents among the exemplified substituents are linked, or having no substituent.

In the present specification, the fact that two or more substituents are linked indicates that hydrogen of any one substituent is linked to another substituent. For example, when two substituents are linked to each other, a phenyl group and a naphthyl group may be linked to each other to become a substituent of

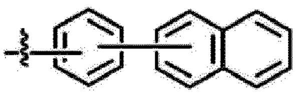

or

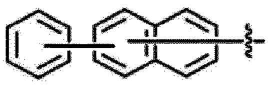

.

Further, the case where three substituents are linked to one another comprises not only a case where (Substituent 1)-(Substituent 2)-(Substituent 3) are consecutively linked to one another, but also a case where (Substituent 2) and (Substituent 3) are linked to (Substituent 1). For example, a phenyl group, a naphthyl group, and an isopropyl group may be linked to one another to form a substituent of

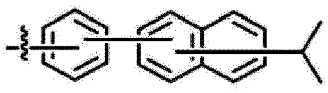

,

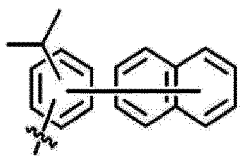

, or

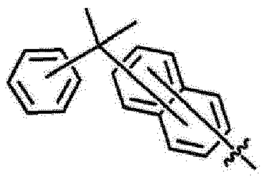

.

The above-described definition also applies equally to the case where four or more substituents are linked to one another.

In the present specification, examples of a halogen group comprise fluorine, chlorine, bromine or iodine.

In the present specification, the alkyl group may be straight-chained or branched, and the number of carbon atoms thereof is not particularly limited, but is preferably 1 to 30. Specific examples thereof comprise a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group, and the like, but are not limited thereto.

In the present specification, a cycloalkyl group is not particularly limited, but has preferably 3 to 30 carbon atoms, and specific examples thereof comprise a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group, an adamantyl group, and the like, but are not limited thereto.

In the present specification, an alkoxy group may be straight-chained, branched, or cyclic. The number of carbon atoms of the alkoxy group is not particularly limited, but is preferably 1 to 30. Specific examples thereof comprise a methoxy group, an ethoxy group, an n-propoxy group, an isopropoxy group, an n-butoxy group, an isobutoxy group, a tert-butoxy group, a sec-butoxy group, an n-pentyloxy group, an neopentyloxy group, an isopentyloxy group, an n-hexyloxy group, a 3,3-dimethylbutyloxy group, a 2-ethylbutyloxy group, an n-octyloxy group, an n-nonyloxy group, an n-decyloxy group, a benzyloxy group, a p-methylbenzyloxy group, and the like, but are not limited thereto.

In the present specification, an alkenyl group may be straight-chained or branched, and the number of carbon atoms thereof is not particularly limited, but is preferably 2 to 30. Specific examples thereof comprise a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group, and the like, but are not limited thereto.

In the present specification, an aryl group is not particularly limited, but has preferably 6 to 30 carbon atoms, and the aryl group may be monocyclic or polycyclic.

When the aryl group is a monocyclic aryl group, the number of carbon atoms is not particularly limited, but is preferably 6 to 50. Specific examples of the monocyclic aryl group comprise a phenyl group, a biphenyl group, a terphenyl group, and the like, but are not limited thereto.

When the aryl group is a polycyclic aryl group, the number of carbon atoms is not particularly limited, but is preferably 10 to 50. Specific examples of the polycyclic aryl group comprise a naphthyl group, an anthracene group, a phenanthrene group, a triphenylene group, a pyrene group, a phenalene group, a perylene group, a chrysene group, a fluorene group, and the like, but are not limited thereto.

In the present specification, the fluorene group may be substituted, and adjacent groups may be bonded to each other to form a ring.

Examples of the case where the fluorene group is substituted comprise a compound having the following structure, but are not limited thereto.

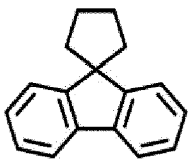 , 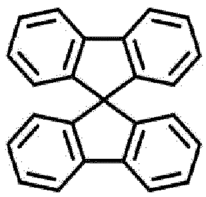 , 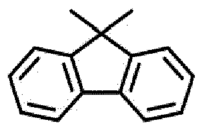 , 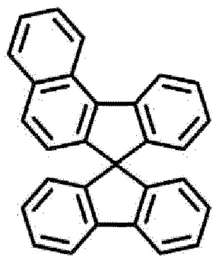 ,

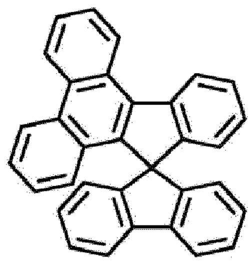 , 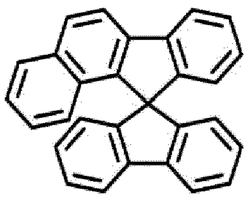 , 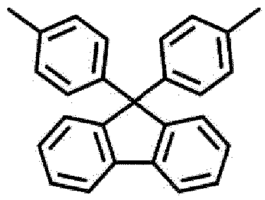 , 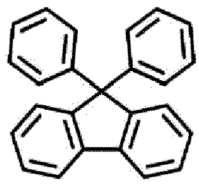

In the present specification, an "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

In the present specification, a heteroaryl group comprises one or more atoms other than carbon, that is, one or more heteroatoms, and specifically, the heteroatom may comprise one or more atoms selected from the group consisting of O, N, Se, S, and the like. The number of carbon atoms thereof is not particularly limited, but is preferably 2 to 30, and the heteroaryl group may be monocyclic or polycyclic. Examples of the heteroaryl group comprise a thiophene group, a furan group, a pyrrole group, an imidazole group, a thiazole group, an oxazole group, an oxadiazole group, a pyridine group, a bipyridine group, a pyrimidine group, a triazine group, a triazole group, an acridine group, a pyridazine group, a pyrazine group, a quinoline group, a quinazoline group, a quinoxaline group, a phthalazine group, a pyridopyrimidine group, a pyridopyrazine group, a pyrazinopyrazine group, an isoquinoline group, an indole group, a carbazole group, a benzoxazole group, a benzimidazole group, a benzothiazole group, a benzocarbazole group, a benzothiophene group, a dibenzothiophene group, a benzofuran group, a phenanthridine group, a phenanthroline group, an isoxazole group, a thiadiazole group, a dibenzofuran group, a dibenzosilole group, a phenoxathiin group, a phenoxazine group, a phenothiazine group, a dihydroindenocarbazole group, a spirofluorenexanthene group, a spirofluorenethioxanthene group, a tetrahydronaphthothiophene group, a tetrahydronaphthofuran group, a tetrahydrobenzothiophene group, a tetrahydrobenzofuran group, and the like, but are not limited thereto.

In the present specification, the silyl group may be an alkylsilyl group, an arylsilyl group, an alkylarylsilyl group, a

heteroarylsilyl group, and the like. The above-described examples of the alkyl group may be applied to the alkyl group in the alkylsilyl group, the above-described examples of the aryl group may be applied to the aryl group in the arylsilyl group, the examples of the alkyl group and the aryl group may be applied to the alkyl group and the aryl group in the alkylarylsilyl group, and the examples of the heterocyclic group may be applied to the heteroaryl group in the heteroarylsilyl group.

In the present specification, a hydrocarbon ring group may be an aromatic hydrocarbon ring group, an aliphatic hydrocarbon ring group, or a fused group of an aromatic hydrocarbon ring and an aliphatic hydrocarbon ring and may be selected among examples of the cycloalkyl group, the aryl group, and a combination thereof, and examples of the hydrocarbon ring group comprise a phenyl group, a cyclohexyl group, an adamantyl group, a bicylo[2.2.1]heptyl group, a bicyclo[2.2.1]octyl group, a tetrahydronaphthalene group, a tetrahydroanthracene group, a 1,2,3,4-tetrahydro-1,4-methanonaphthalene group, a 1,2,3,4-tetrahydro-1,4-ethanonaphthalene group, a spirocyclopentanefluorene group, a spiroadamantanefluorene group, a spirocyclohexanefluorene group, and the like, but are not limited thereto.

In the present specification, a heterocyclic group comprises one or more atoms other than carbon, that is, one or more heteroatoms, and specifically, the heteroatom may comprise one or more atoms selected from the group consisting of O, N, Se, S, and the like. The heterocyclic group may be monocyclic or polycyclic, and may be an aromatic heterocyclic group; an aliphatic heterocyclic group; a condensed ring group of an aromatic hetero ring and an aliphatic hetero ring; a condensed ring group of an aliphatic hydrocarbon ring, an aromatic hydrocarbon ring and an aromatic hetero ring, or a condensed ring group of an aliphatic hydrocarbon ring, an aromatic hydrocarbon ring and an aliphatic hetero ring, and the aromatic heterocyclic group may be selected among the examples of the heteroaryl group.

In the present specification, an aliphatic heterocyclic group means an aliphatic ring group comprising one or more of hetero atoms. An aliphatic heterocyclic group comprises all of a single-bonded aliphatic ring group, an aliphatic ring group comprising a multiple bond, or an aliphatic ring group in a form in which a ring comprising a single bond and a multiple bond is condensed. Examples of the aliphatic hetero ring comprise an epoxy group, an oxirane group, a tetrahydrofuran group, a 1,4-dioxane group, a pyrrolidine group, a piperidine group, a morpholine group, an oxepane group, an azocane group, a thiocane group, a tetrahydronaphthothiophene group, a tetrahydronaphthofuran group, a tetrahydrobenzothiophene group, a tetrahydrobenzofuran group, and the like, but are not limited thereto.

In the present specification, the aryloxy group may be represented by -ORo, and the description on the above-described aryl group is applied to Ro.

In the present specification, the arylthio group may be represented by -SRs1, and the description on the above-described aryl group is applied to Rs1.

In the present specification, the alkylthio group may be represented by -SRs2, and the description on the above-described alkyl group is applied to Rs2.

In the present specification, an alkylene group means a group having two bonding positions in an alkyl group, that is, a divalent group. The above-described description on the alkyl group may be applied to the alkylene group, except for a divalent alkylene group.

In the present specification, the cycloalkylene group means a group having two bonding positions in a cycloalkyl group, that is, a divalent group. The above-described description on the cycloalkyl group may be applied to the cycloalkylene groups, except for a divalent cycloalkylene group.

In the present specification, a condensed ring group of a divalent aromatic hydrocarbon ring and an aliphatic hydrocarbon ring means a group having two bonding positions in the condensed ring group of the aromatic hydrocarbon ring and the aliphatic hydrocarbon ring, that is, a divalent group. The above-described description on the condensed ring group of the aromatic hydrocarbon ring and the aliphatic hydrocarbon ring may be applied, except that the groups are each a divalent group.

In the present specification, an arylene group means a group having two bonding positions in an aryl group, that is, a divalent group. The above-described description on the aryl group may be applied to the arylene group, except for a divalent arylene group.

In the present specification, hydrogen is hydrogen, deuterium, or tritium.

In an exemplary embodiment of the present specification, a portion wherein a substituent is not indicated in Chemical Formula 1 may mean that hydrogen, deuterium, or tritium is substituted.

Hereinafter, preferred exemplary embodiments of the present invention will be described in detail. However, the exemplary embodiments of the present invention may be modified into various other forms, and the scope of the present invention is not limited to the exemplary embodiments which will be described below.

According to an exemplary embodiment of the present specification, one or more of the units of Chemical Formula 1 can be comprised in the resin, and when two or more are comprised, the units are the same as or different from each other.

According to an exemplary embodiment of the present specification, R101 is hydrogen.

According to an exemplary embodiment of the present specification, R102 is hydrogen.

However, in the present specification, the case where all of R1 to R4 are an unsubstituted phenyl group is excluded.

According to an exemplary embodiment of the present specification, Chemical Formula 1 is the following Chemical Formula 1-A.

[Chemical Formula 1-A]

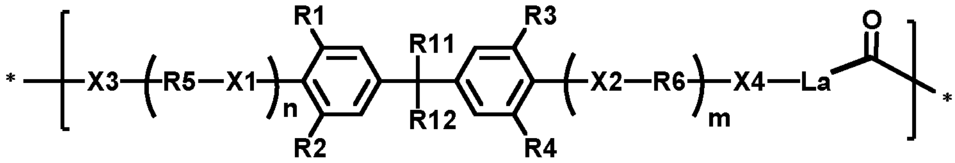

In Chemical Formula 1-A,
the definitions of *, La, R1 to R6, R11, R12 and X1 to X4 are the same as those defined in Chemical Formula 1.

According to an exemplary embodiment of the present specification, Chemical Formula 1 is any one of the following Chemical Formulae 1-1 to 1-4.

[Chemical Formula 1-1]

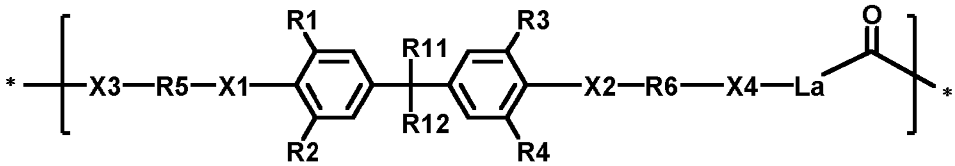

[Chemical Formula 1-2]

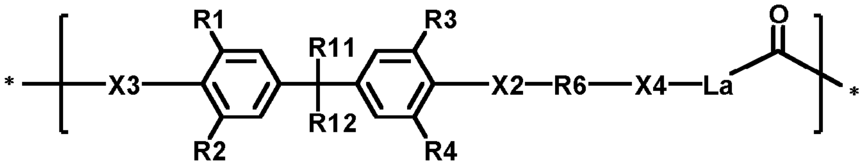

[Chemical Formula 1-3]

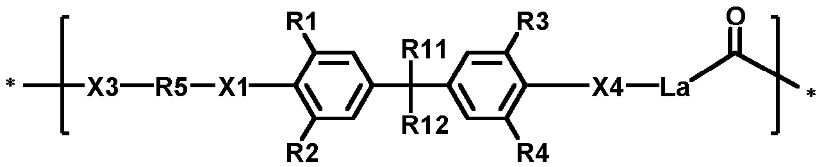

[Chemical Formula 1-4]

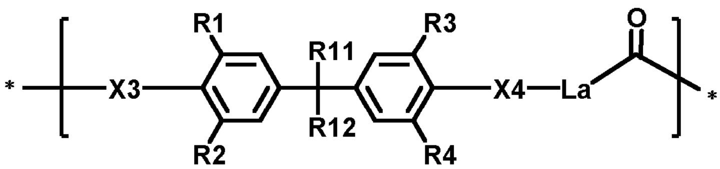

In Chemical Formulae 1-1 to 1-4,
the definitions of *, La, R1 to R6, R11, R12 and X1 to X4 are the same as those defined in Chemical Formula 1.

According to an exemplary embodiment of the present specification, Chemical Formula 1 is the following Chemical Formula 2.

[Chemical Formula 2]

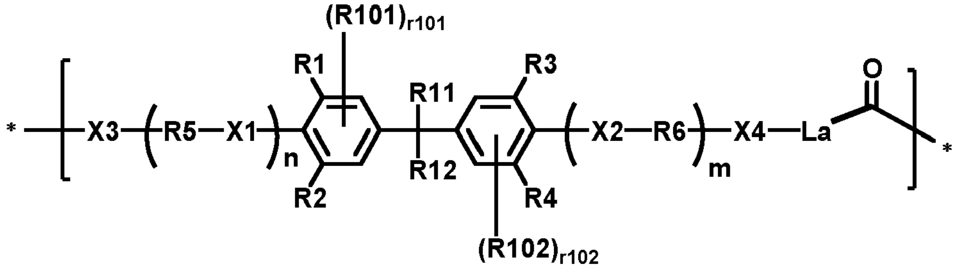

In Chemical Formula 2,

X1 to X4 are the same as or different from each other, and are each independently O or S,
R1 to R4 are the same as or different from each other, and are each independently a substituted or unsubstituted aryl group; or a substituted or unsubstituted heteroaryl group,
R5 and R6 are the same as or different from each other, and are each independently a substituted or unsubstituted alkylene group; or a substituted or unsubstituted cycloalkylene group,
R11 and R12 are the same as or different from each other, and are each independently a substituted or unsubstituted alkyl group; or a substituted or unsubstituted cycloalkyl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other,
La is a direct bond; or -C(=O)-L'-,
L' is a substituted or unsubstituted arylene group,

m and n are each 0 or 1, and

* means a moiety linked to the main chain of the resin.

According to an exemplary embodiment of the present specification, X1 to X4 are the same as or different from each other, and are each independently O; or S,

R1 to R4 are the same as or different from each other, and are each independently a monocyclic or polycyclic aryl group having 6 to 30 carbon atoms, which is unsubstituted or substituted with one or more of a straight-chained or branched alkyl group having 1 to 30 carbon atoms, a straight-chained or branched alkoxy group having 1 to 30 carbon atoms, a monocyclic or polycyclic aryloxy group having 6 to 30 carbon atoms, a monocyclic or polycyclic aryl group having 6 to 30 carbon atoms, a polycyclic heteroaryl group having 6 to 30 carbon atoms, and a combination thereof; or a polycyclic heteroaryl group having 6 to 30 carbon atoms, which is unsubstituted or substituted with a monocyclic or polycyclic aryl group having 6 to 30 carbon atoms,
R5 and R6 are the same as or different from each other, and are each independently a straight-chained or branched alkylene group having 2 to 30 carbon atoms; or a monocyclic or polycyclic cycloalkylene group having 6 to 30 carbon atoms,
R11 and R12 are the same as or different from each other, and are each independently a straight-chained or branched alkyl group having 1 to 30 carbon atoms,
R101 and R102 are hydrogen,
La is a direct bond; or -C(=O)-L'-, and
L' is a monocyclic or polycyclic arylene group having 6 to 30 carbon atoms.

According to an exemplary embodiment of the present specification, Chemical Formula 1 is the following Chemical Formula 3.

[Chemical Formula 3]

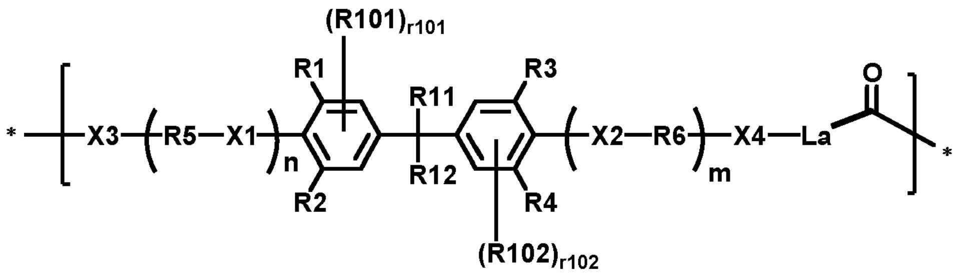

In Chemical Formula 3,

X1 to X4 are the same as or different from each other, and are each independently O or S,
R1 to R4 are the same as or different from each other, and are each independently a substituted or unsubstituted aryl group; or a substituted or unsubstituted heteroaryl group,
R5 and R6 are the same as or different from each other, and are each independently a substituted or unsubstituted alkylene group; or a substituted or unsubstituted cycloalkylene group,
R11 is a substituted or unsubstituted alkyl group; or a substituted or unsubstituted cycloalkyl group,
R12 is a substituted or unsubstituted aryl group; or a substituted or unsubstituted heteroaryl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other,
La is a direct bond; or -C(=O)-L'-,
L' is a substituted or unsubstituted arylene group,

m and n are each 0 or 1, and

* means a moiety linked to the main chain of the resin.

According to an exemplary embodiment of the present specification, X1 to X4 are the same as or different from each other, and are each independently O; or S,

R1 to R4 are the same as or different from each other, and are each independently a monocyclic or polycyclic aryl group having 6 to 30 carbon atoms, which is unsubstituted or substituted with one or more of a straight-chained or branched alkyl group having 1 to 30 carbon atoms, a straight-chained or branched alkoxy group having 1 to 30 carbon atoms, a monocyclic or polycyclic aryloxy group having 6 to 30 carbon atoms, a monocyclic or polycyclic aryl group having 6 to 30 carbon atoms, a polycyclic heteroaryl group having 6 to 30 carbon atoms, and a combination thereof; or a polycyclic heteroaryl group having 6 to 30 carbon atoms, which is unsubstituted or substituted with a monocyclic or polycyclic aryl group having 6 to 30 carbon atoms,
R5 and R6 are the same as or different from each other, and are each independently a straight-chained or branched alkylene group having 2 to 30 carbon atoms; or a monocyclic or polycyclic cycloalkylene group having 6 to 30 carbon atoms,
R11 is a straight-chained or branched alkyl group having 1 to 30 carbon atoms,
R12 is a monocyclic or polycyclic aryl group having 6 to 30 carbon atoms, which is unsubstituted or substituted with one or more of a monocyclic or polycyclic aryl group having 6 to 30 carbon atoms, a polycyclic heteroaryl group having 6 to 30 carbon atoms, and a combination thereof,
R101 and R102 are hydrogen,
La is a direct bond; or -C(=O)-L'-, and
L' is a monocyclic or polycyclic arylene group having 6 to 30 carbon atoms.

According to an exemplary embodiment of the present specification, Chemical Formula 1 is the following Chemical Formula 4.

[Chemical Formula 4]

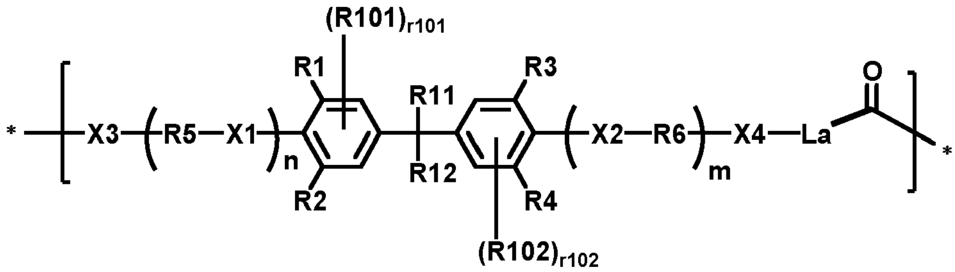

In Chemical Formula 4,

X1 to X4 are the same as or different from each other, and are each independently O or S,
R1 and R3 are the same as or different from each other, and are each independently a phenyl group which is substituted with one or more of a substituted or unsubstituted aryl group having 10 or more carbon atoms, a substituted or unsubstituted heteroaryl group, and a combination thereof; a substituted or unsubstituted aryl group having 10 or more carbon atoms; or a substituted or unsubstituted heteroaryl group,
R2 and R4 are the same as or different from each other, and are each independently hydrogen; or a substituted or unsubstituted alkyl group,
R5 and R6 are the same as or different from each other, and are each independently a substituted or unsubstituted alkylene group; or a substituted or unsubstituted cycloalkylene group,
R11 and R12 are the same as or different from each other, and are each independently a substituted or unsubstituted alkyl group; or a substituted or unsubstituted cycloalkyl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other,
La is a direct bond; or -C(=O)-L'-,
L' is a substituted or unsubstituted arylene group,
m and n are each 0 or 1, and
* means a moiety linked to the main chain of the resin.

According to an exemplary embodiment of the present specification, X1 to X4 are the same as or different from each other, and are each independently O; or S,

R1 and R3 are the same as or different from each other, and are each independently a polycyclic aryl group having 10 to 30 carbon atoms, which is unsubstituted or substituted with a straight-chained or branched alkyl group having 1 to 30 carbon atoms, a straight-chained or branched alkoxy group having 1 to 30 carbon atoms, or a monocyclic or polycyclic aryl group having 6 to 30 carbon atoms; a phenyl group which is substituted with one or more of a monocyclic or polycyclic aryl group having 10 to 30 carbon atoms, a polycyclic heteroaryl group having 6 to 30 carbon atoms, which is unsubstituted or substituted with a monocyclic or polycyclic aryl group having 6 to 30 carbon atoms, and a combination thereof; or a polycyclic heteroaryl group having 6 to 30 carbon atoms, which is unsubstituted or substituted with a monocyclic or polycyclic aryl group having 6 to 30 carbon atoms,
R2 and R4 are the same as or different from each other, and are each independently hydrogen; or a substituted or unsubstituted straight-chained or branched alkyl group having 1 to 30 carbon atoms, which is unsubstituted or substituted with a monocyclic or polycyclic aryl group having 6 to 30 carbon atoms,
R5 and R6 are the same as or different from each other, and are each independently a straight-chained or branched alkylene group having 2 to 30 carbon atoms; or a monocyclic or polycyclic cycloalkylene group having 6 to 30 carbon atoms,
R11 and R12 are the same as or different from each other, and are each independently a straight-chained or branched alkyl group having 1 to 30 carbon atoms,
R101 and R102 are hydrogen,
La is a direct bond; or -C(=O)-L'-, and
L' is a monocyclic or polycyclic arylene group having 6 to 30 carbon atoms.

According to an exemplary embodiment of the present specification, Chemical Formula 1 is the following Chemical Formula 5.

[Chemical Formula 5]

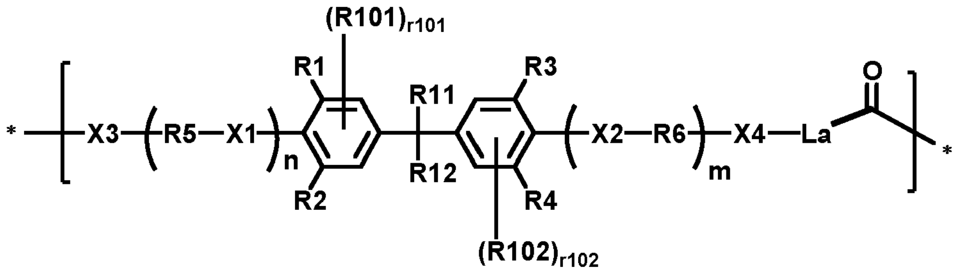

In Chemical Formula 5,

X1 to X4 are the same as or different from each other, and are each independently O or S,
R1 and R3 are the same as or different from each other, and are each independently a substituted or unsubstituted aryl group; or a substituted or unsubstituted heteroaryl group,
R2 and R4 are the same as or different from each other, and are each independently hydrogen; a substituted or unsubstituted alkyl group; or a substituted or unsubstituted cycloalkyl group,
R5 and R6 are the same as or different from each other, and are each independently a substituted or unsubstituted alkylene group; or a substituted or unsubstituted cycloalkylene group,
R11 is a substituted or unsubstituted alkyl group; or a substituted or unsubstituted cycloalkyl group,
R12 is a substituted or unsubstituted aryl group; or a substituted or unsubstituted heteroaryl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other,
La is a direct bond; or -C(=O)-L'-,
L' is a substituted or unsubstituted arylene group,

m and n are each 0 or 1, and

* means a moiety linked to the main chain of the resin.

According to an exemplary embodiment of the present specification, X1 to X4 are the same as or different from each other, and are each independently O; or S,

R1 and R3 are the same as or different from each other, and are each independently a monocyclic or polycyclic aryl group having 6 to 30 carbon atoms, which is unsubstituted or substituted with one or more of a straight-chained or branched alkyl group having 1 to 30 carbon atoms, a straight-chained or branched alkoxy group having 1 to 30 carbon atoms, a monocyclic or polycyclic aryl group having 6 to 30 carbon atoms, a polycyclic heteroaryl group having 6 to 30 carbon atoms, and a combination thereof; or a polycyclic heteroaryl group having 6 to 30 carbon atoms, which is unsubstituted or substituted with a monocyclic or polycyclic aryl group having 6 to 30 carbon atoms,
R2 and R4 are the same as or different from each other, and are each independently hydrogen; or a straight-chained or branched alkyl group having 1 to 30 carbon atoms,
R5 and R6 are the same as or different from each other, and are each independently a straight-chained or branched alkylene group having 2 to 30 carbon atoms; or a monocyclic or polycyclic cycloalkylene group having 6 to 30 carbon atoms,
R11 is a straight-chained or branched alkyl group having 1 to 30 carbon atoms,
R12 is a monocyclic or polycyclic aryl group having 6 to 30 carbon atoms, which is unsubstituted or substituted with a monocyclic or polycyclic aryl group having 6 to 30 carbon atoms; or a polycyclic heteroaryl group having 6 to 30 carbon atoms,
R101 and R102 are hydrogen,
La is a direct bond; or -C(=O)-L'-, and

L' is a monocyclic or polycyclic arylene group having 6 to 30 carbon atoms.

According to an exemplary embodiment of the present specification, R1 to R4 are the same as or different from each other, and are each independently any one of the following structures.

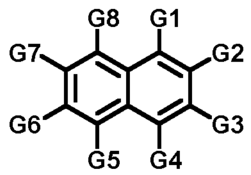
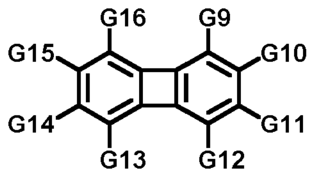
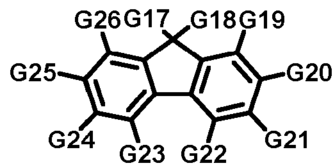
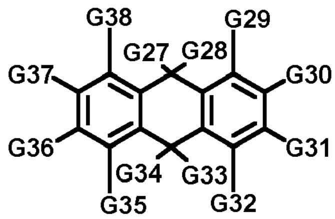
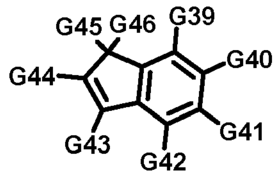
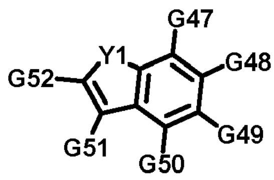
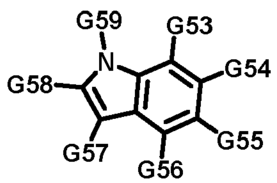
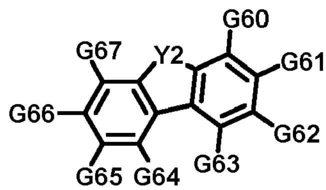
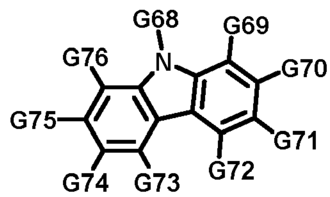

In the structures,

Y1 and Y2 are each independently O or S, any one of G1 to G8 is a moiety bonded to Chemical Formula 1, and the others of G1 to G8, which are not bonded to Chemical Formula 1, are the same as or different from each other, and are each independently hydrogen; a substituted or unsubstituted alkyl group; a substituted or unsubstituted alkenyl group; a substituted or unsubstituted hydrocarbon ring group; or a substituted or unsubstituted heterocyclic group,

any one of G9 to G16 is a moiety bonded to Chemical Formula 1, and the others of G9 to G16, which are not bonded to Chemical Formula 1, are the same as or different from each other, and are each independently hydrogen; a substituted or unsubstituted alkyl group; a substituted or unsubstituted alkenyl group; a substituted or unsubstituted hydrocarbon ring group; or a substituted or unsubstituted heterocyclic group,

any one of G17 to G26 is a moiety bonded to Chemical Formula 1, and the others of G17 to G26, which are not bonded to Chemical Formula 1, are the same as or different from each other, and are each independently hydrogen; a substituted or unsubstituted alkyl group; a substituted or unsubstituted alkenyl group; a substituted or unsubstituted hydrocarbon ring group; or a substituted or unsubstituted heterocyclic group,

any one of G27 to G38 is a moiety bonded to Chemical Formula 1, and the others of G27 to G38, which are not bonded to Chemical Formula 1, are the same as or different from each other, and are each independently hydrogen; a substituted or unsubstituted alkyl group; a substituted or unsubstituted alkenyl group; a substituted or unsubstituted hydrocarbon ring group; or a substituted or unsubstituted heterocyclic group,

any one of G39 to G46 is a moiety bonded to Chemical Formula 1, and the others of G39 to G46, which are not bonded to Chemical Formula 1, are the same as or different from each other, and are each independently hydrogen; a substituted or unsubstituted alkyl group; a substituted or unsubstituted alkenyl group; a substituted or unsubstituted hydrocarbon ring group; or a substituted or unsubstituted heterocyclic group,

any one of G47 to G52 is a moiety bonded to Chemical Formula 1, and the others of G47 to G52, which are not bonded to Chemical Formula 1, are the same as or different from each other, and are each independently hydrogen; a substituted or unsubstituted alkyl group; a substituted or unsubstituted alkenyl group; a substituted or unsubstituted hydrocarbon ring group; or a substituted or unsubstituted heterocyclic group,

any one of G53 to G59 is a moiety bonded to Chemical Formula 1, and the others of G53 to G59, which are not bonded to Chemical Formula 1, are the same as or different from each other, and are each independently hydrogen; a substituted or unsubstituted alkyl group; a substituted or unsubstituted alkenyl group; a substituted or unsubstituted

hydrocarbon ring group; or a substituted or unsubstituted heterocyclic group,

any one of G60 to G67 is a moiety bonded to Chemical Formula 1, and the others of G60 to G67, which are not bonded to Chemical Formula 1, are the same as or different from each other, and are each independently hydrogen; a substituted or unsubstituted alkyl group; a substituted or unsubstituted alkenyl group; a substituted or unsubstituted hydrocarbon ring group; or a substituted or unsubstituted heterocyclic group, and

any one of G68 to G76 is a moiety bonded to Chemical Formula 1, and the others of G68 to G73, which are not bonded to Chemical Formula 1, are the same as or different from each other, and are each independently hydrogen; a substituted or unsubstituted alkyl group; a substituted or unsubstituted alkenyl group; a substituted or unsubstituted hydrocarbon ring group; or a substituted or unsubstituted heterocyclic group.

According to an exemplary embodiment of the present specification, R12 is any one selected from the following structures.

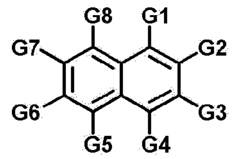
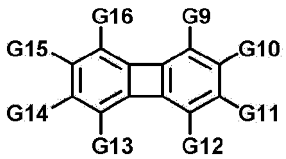
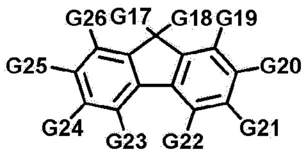

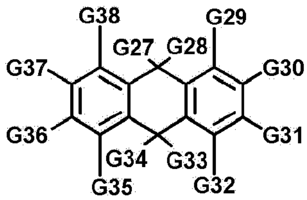
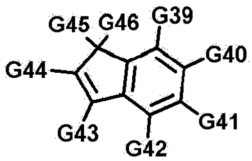
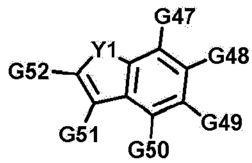

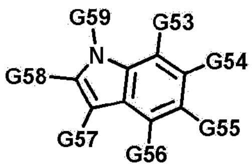
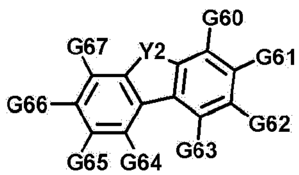
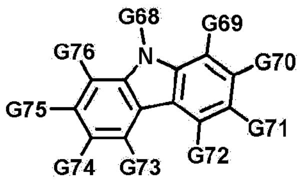

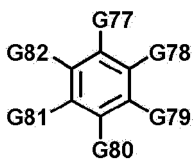

In the structures,

the definitions of Y1, Y2 and G1 to G76 are the same as those described above, and

any one of G77 to G82 is a moiety bonded to Chemical Formula 1, and the others of G77 to G82, which are not bonded to Chemical Formula 1, are the same as or different from each other, and are each independently hydrogen; a substituted or unsubstituted alkyl group; a substituted or unsubstituted alkenyl group; a substituted or unsubstituted hydrocarbon ring group; or a substituted or unsubstituted heterocyclic group.

According to an exemplary embodiment of the present specification, X1 to X4 are O.

According to an exemplary embodiment of the present specification, X1 to X4 are S.

According to an exemplary embodiment of the present specification, X1 and X2 are S, and X3 and X4 are O.

According to an exemplary embodiment of the present specification, X1 and X2 are O, and X3 and X4 are S.

According to an exemplary embodiment of the present specification, X1 is O.

According to an exemplary embodiment of the present specification, X2 is O.

According to an exemplary embodiment of the present specification, X3 is O.

According to an exemplary embodiment of the present specification, X4 is O.

According to an exemplary embodiment of the present specification, X1 is S.
According to an exemplary embodiment of the present specification, X2 is S.
According to an exemplary embodiment of the present specification, X3 is S.
According to an exemplary embodiment of the present specification, X4 is S.
According to an exemplary embodiment of the present specification, R1 to R4 are the same as or different from each other, and are each independently a monocyclic or polycyclic aryl group having 6 to 30 carbon atoms, which is unsubstituted or substituted with one or more of a straight-chained or branched alkyl group having 1 to 30 carbon atoms, a straight-chained or branched alkoxy group having 1 to 30 carbon atoms, a monocyclic or polycyclic aryloxy group having 6 to 30 carbon atoms, a monocyclic or polycyclic aryl group having 6 to 30 carbon atoms, a polycyclic heteroaryl group having 6 to 30 carbon atoms, and a combination thereof; or a polycyclic heteroaryl group having 6 to 30 carbon atoms, which is unsubstituted or substituted with a monocyclic or polycyclic aryl group having 6 to 30 carbon atoms.
According to an exemplary embodiment of the present specification, R1 to R4 are the same as or different from each other, and are each independently a phenyl group which is substituted with one or more of a monocyclic or polycyclic aryloxy group having 6 to 30 carbon atoms, a monocyclic or polycyclic aryl group having 6 to 30 carbon atoms, a polycyclic heteroaryl group having 6 to 30 carbon atoms, and a combination thereof; a naphthyl group which is unsubstituted or substituted with a straight-chained or branched alkyl group having 1 to 30 carbon atoms, a straight-chained or branched alkoxy group having 1 to 30 carbon atoms, or a monocyclic or polycyclic aryl group having 6 to 30 carbon atoms; a carbazole group which is unsubstituted or substituted with a monocyclic or polycyclic aryl group having 6 to 30 carbon atoms; a benzofuran group; or a dibenzofuran group.
According to an exemplary embodiment of the present specification, R1 to R4 are the same as or different from each other, and are each independently a phenyl group which is substituted with one or more of a phenoxy group, a naphthyl group, a phenyl group, an imidazole group, a carbazole group, and a combination thereof; a naphthyl group which is unsubstituted or substituted with a methyl group, a methoxy group, or a phenyl group; a carbazole group which is unsubstituted or substituted with a phenyl group; a benzofuran group; or a dibenzofuran group.
According to an exemplary embodiment of the present specification, R1 to R4 are the same as or different from each other, and are each independently a phenyl group which is substituted with a phenoxy group, a naphthyl group, an imidazole group substituted with a phenyl group, or a carbazole group; a naphthyl group which is unsubstituted or substituted with a methyl group, a methoxy group, or a phenyl group; a carbazole group substituted with a phenyl group; a benzofuran group; or a dibenzofuran group.
According to an exemplary embodiment of the present specification, R1 to R4 are the same as each other.
According to an exemplary embodiment of the present specification, R1 and R3 are the same as or different from each other, and are each independently a polycyclic aryl group having 10 to 30 carbon atoms, which is unsubstituted or substituted with a straight-chained or branched alkyl group having 1 to 30 carbon atoms, a straight-chained or branched alkoxy group having 1 to 30 carbon atoms, or a monocyclic or polycyclic aryl group having 6 to 30 carbon atoms; a phenyl group which is substituted with one or more of a monocyclic or polycyclic aryl group having 10 to 30 carbon atoms, a polycyclic heteroaryl group having 6 to 30 carbon atoms, and a combination thereof; or a polycyclic heteroaryl group having 6 to 30 carbon atoms, which is unsubstituted or substituted with a monocyclic or polycyclic aryl group having 6 to 30 carbon atoms.
According to an exemplary embodiment of the present specification, R1 and R3 are the same as or different from each other, and are each independently a phenyl group which is substituted with one or more of a monocyclic or polycyclic aryl group having 10 to 30 carbon atoms, a polycyclic heteroaryl group having 6 to 30 carbon atoms, which is unsubstituted or substituted with a monocyclic or polycyclic aryl group having 6 to 30 carbon atoms, and a combination thereof; a naphthyl group which is unsubstituted or substituted with a straight-chained or branched alkyl group having 1 to 30 carbon atoms, a straight-chained or branched alkoxy group having 1 to 30 carbon atoms, or a monocyclic or polycyclic aryl group having 6 to 30 carbon atoms; a carbazole group which is unsubstituted or substituted with a monocyclic or polycyclic aryl group having 6 to 30 carbon atoms; a benzofuran group; or a dibenzofuran group.
According to an exemplary embodiment of the present specification, R1 and R3 are the same as or different from each other, and are each independently a phenyl group which is substituted with one or more of a phenoxy group, a naphthyl group, an imidazole group which is unsubstituted or substituted with a phenyl group, a carbazole group, and a combination thereof; a naphthyl group which is unsubstituted or substituted with a methyl group, a methoxy group, or a phenyl group; a carbazole group which is unsubstituted or substituted with a phenyl group; a benzofuran group; or a dibenzofuran group.
According to an exemplary embodiment of the present specification, R1 and R3 are the same as or different from each other, and are each independently a phenyl group which is substituted with a naphthyl group, an imidazole group substituted with a phenyl group, or a carbazole group; a naphthyl group which is unsubstituted or substituted with a methyl group, a methoxy group, or a phenyl group; a carbazole group substituted with a phenyl group; a benzofuran group; or a dibenzofuran group.
According to an exemplary embodiment of the present specification, R1 and R3 are the same as or different from

each other, and are each independently a phenyl group which is substituted with a phenoxy group, a naphthyl group, an imidazole group substituted with a phenyl group, or a carbazole group; a naphthyl group which is unsubstituted or substituted with a methyl group, a methoxy group, or a phenyl group; a carbazole group substituted with a phenyl group; a benzofuran group; or a dibenzofuran group.

According to an exemplary embodiment of the present specification, R1 and R3 are the same as each other.

According to an exemplary embodiment of the present specification, R2 and R4 are the same as or different from each other, and are each independently hydrogen; or a substituted or unsubstituted straight-chained or branched alkyl group having 1 to 30 carbon atoms, which is unsubstituted or substituted with a monocyclic or polycyclic aryl group having 6 to 30 carbon atoms.

According to an exemplary embodiment of the present specification, R2 and R4 are the same as or different from each other, and are each independently hydrogen; a substituted or unsubstituted methyl group which is unsubstituted or substituted with a monocyclic or polycyclic aryl group having 6 to 30 carbon atoms; an ethyl group; an isopropyl group; or a tert-butyl group.

According to an exemplary embodiment of the present specification, R2 and R4 are the same as or different from each other, and are each independently hydrogen; a substituted or unsubstituted methyl group which is unsubstituted or substituted with a phenyl group; an ethyl group; an isopropyl group; or a tert-butyl group.

According to an exemplary embodiment of the present specification, R5 and R6 are the same as or different from each other, and are each independently a straight-chained or branched alkylene group having 2 to 30 carbon atoms; or a monocyclic or polycyclic cycloalkylene group having 6 to 30 carbon atoms.

According to an exemplary embodiment of the present specification, R5 and R6 are the same as or different from each other, and are each independently a straight-chained or branched alkylene group having 2 to 10 carbon atoms; or a monocyclic or polycyclic cycloalkylene group having 6 to 10 carbon atoms.

According to an exemplary embodiment of the present specification, R5 and R6 are the same as or different from each other, and are each independently an ethylene group; an isopropylene group; an isobutylene group; or a cyclohexylene group.

According to an exemplary embodiment of the present specification, R5 and R6 are the same as each other.

According to an exemplary embodiment of the present specification, R11 and R12 are the same as or different from each other, and are each independently a straight-chained or branched alkyl group having 1 to 30 carbon atoms.

According to an exemplary embodiment of the present specification, R11 and R12 are the same as or different from each other, and are each independently a straight-chained or branched alkyl group having 1 to 10 carbon atoms.

According to an exemplary embodiment of the present specification, R11 and R12 are the same as or different from each other, and are each independently a methyl group; an ethyl group; or an isopropyl group.

According to an exemplary embodiment of the present specification, R11 is a straight-chained or branched alkyl group having 1 to 30 carbon atoms.

According to an exemplary embodiment of the present specification, R11 is a straight-chained or branched alkyl group having 1 to 10 carbon atoms.

According to an exemplary embodiment of the present specification, R11 is a methyl group; an ethyl group; or an isopropyl group.

According to an exemplary embodiment of the present specification, R11 is a methyl group.

According to an exemplary embodiment of the present specification, R12 is a monocyclic or polycyclic aryl group having 6 to 30 carbon atoms, which is unsubstituted or substituted with one or more of a monocyclic or polycyclic aryl group having 6 to 30 carbon atoms, a polycyclic heteroaryl group having 6 to 30 carbon atoms, and a combination thereof.

According to an exemplary embodiment of the present specification, R12 is a phenyl group which is unsubstituted or substituted with one or more of a monocyclic or polycyclic aryl group having 6 to 30 carbon atoms, a polycyclic heteroaryl group having 6 to 30 carbon atoms, and a combination thereof; or a biphenyl group which is unsubstituted or substituted with a monocyclic or polycyclic aryl group having 6 to 30 carbon atoms.

According to an exemplary embodiment of the present specification, R12 is a phenyl group which is unsubstituted or substituted with one or more of a phenyl group, a naphthyl group, a benzofuran group, an indole group, and a combination thereof; or a biphenyl group which is unsubstituted or substituted with a naphthyl group.

According to an exemplary embodiment of the present specification, R12 is a phenyl group which is unsubstituted or substituted with a naphthyl group, a benzofuran group, or an indole group substituted with a phenyl group; or a biphenyl group substituted with a naphthyl group.

According to an exemplary embodiment of the present specification, La is a direct bond; or -C(=O)-L'-.

According to an exemplary embodiment of the present specification, La is a direct bond.

According to an exemplary embodiment of the present specification, La is - C(=O)-L'-.

According to an exemplary embodiment of the present specification, L' is a monocyclic or polycyclic arylene group having 6 to 30 carbon atoms.

According to an exemplary embodiment of the present specification, L' is a monocyclic or polycyclic arylene

group having 6 to 10 carbon atoms.

According to an exemplary embodiment of the present specification, L' is a phenylene group.

According to an exemplary embodiment of the present specification, the resin may have -OH; -SH; $-CO_2CH_3$; or $-OC_6H_5$ as an end group.

In an exemplary embodiment of the present invention, the resin has a weight average molecular weight of 10,000 g/mol to 200,000 g/mol, preferably 15,000 g/mol to 100,000 g/mol, more preferably 20,000 g/mol to 50,000 g/mol, and 25,000 g/mol to 40,000 g/mol.

When the resin satisfies the above-described weight average molecular weight range, the resin may have optimum fluidity and processability.

In an exemplary embodiment of the present invention, the resin has a number average molecular weight of 10,000 g/mol to 100,000 g/mol, 10,000 g/mol to 50,000 g/mol, 10,000 g/mol to 30,000 g/mol, 11,000 g/mol to 28,000 g/mol, preferably 12,000 g/mol to 25,000 g/mol.

In the present specification, the weight average molecular weights (Mws) of the resin and the oligomer used in the preparation thereof may be measured by gel permeation chromatography (GPC) using a polystyrene (PS) standard using Agilent 1200 series. Specifically, the weight average molecular weights may be measured using an Agilent 1200 series device using a Polymer Laboratories PLgel MIX-B 300 mm length column, and in this case, the measurement temperature is 40°C, the used solvent is tetrahydrofuran (THF), and the flow rate is 1 mL/min. The sample of the resin or oligomer is each prepared at a concentration of 10 mg/10 mL, and then fed in an amount of 10 μL, and the weight average molecular weight (Mw) value is induced using a calibration curve formed using a polystyrene standard. In this case, nine types of polystyrene standard products with a molecular weight (g/mol) of 2,000 / 10,000 / 30,000 / 70,000 / 200,000 / 700,000 / 2,000,000 / 4,000,000 / 10,000,000 are used.

In an exemplary embodiment of the present specification, the resin may have a glass transition temperature (Tg) of 100°C to 260°C. The resin may have a glass transition temperature of 100°C to 250°C, 100°C to 240°C, 110°C to 230°C, 120°C to 220°C, 130°C to 210°C, 133°C to 236°C, 120°C to 250°C, 130°C to 240°C, 145°C to 237°C, 105°C to 240°C, 107°C to 209°C, 110°C to 250°C, 110°C to 240°C, or 121°C to 215°C.

When the resin satisfies the above glass transition temperature range, the glass transition temperature is easily adjusted when a resin composition is prepared by mixing with a resin having excellent heat resistance and injectability and having a glass transition temperature different from the above-described range, so that the physical properties desired in the present specification may be satisfied.

The glass transition temperature (Tg) may be measured by a differential scanning calorimeter (DSC). Specifically, the glass transition temperature may be measured from a graph obtained by heating 5.5 mg to 8.5 mg of the resin sample to 270°C under a nitrogen atmosphere, and then scanning the resin sample while heating the resin sample at a heating rate of 10°C/min during the second heating after cooling.

In an exemplary embodiment of the present specification, a refractive index of the resin, which is measured at a wavelength of 589 nm, is 1.60 to 1.80. The refractive index may be 1.64 to 1.74, 1.65 to 1.75, 1.66 to 1.76, 1.667 to 1.757, 1.665 to 1.755, 1.688 to 1.752, 1.655 to 1.745, 1.656 to 1.731, 1.645 to 1.735, or 1.657 to 1.725. When the resin satisfies the above refractive index, a thin and light optical lens can be manufactured when the resin is applied to a molded article such as an optical lens.

In an exemplary embodiment of the present specification, the Abbe's Number of the resin, which is measured and calculated at a wavelength of 486 nm, 589 nm, and 656 nm may be 8 to 25. The Abbe's Number may be 12.2 to 20.2, 12.0 to 20.0, 12.5 to 17.6, 13.5 to 20.3, or 13.5 to 21.5. When the resin satisfies the above Abbe's Number range, there is an effect that the dispersion is decreased and the sharpness is increased when the resin is applied to a molded article such as an optical lens.

The Abbe's Number may be specifically obtained by the following Equation by measuring the refractive index ($n_D$, $n_F$, and $n_C$) at a wavelength of D (589 nm), F (486 nm), and C (656 nm), respectively at 20°C.

$$\text{Abbe's Number} = (n_D - 1)/(n_F - n_C)$$

The refractive index and the Abbe's Number may be measured from a film prepared by applying a solution prepared by dissolving the resin in a solvent to a silicon wafer by spin-coating, and may be measured by obtaining the result value according to the wavelength of light using an ellipsometer at 20°C for the applied film. The solution may be applied by the spin-coating at a rotation speed of 150 rpm to 300 rpm, and the applied film may have a thickness of 5 μm to 20 μm. The silicon wafer is not particularly limited, and any silicon wafer that can measure the refractive index and the Abbe's Number of the resin composition according to the present specification may be appropriately adopted. The solvent may be dimethylacetamide or 1,2-dichlorobenzene, and the solution may be prepared by dissolving the resin sample in an amount of 10 wt% based on the total weight of the solution.

An exemplary embodiment of the present specification provides the following Chemical Formula 1a.

[Chemical Formula 1a]

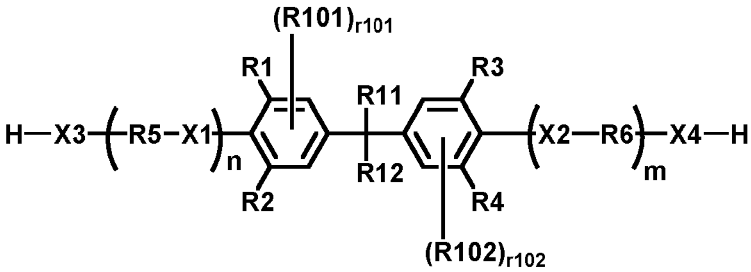

In Chemical Formula 1a,

X1 to X4 are the same as or different from each other, and are each independently O or S,
at least one of R1 to R4 is a substituted or unsubstituted alkyl group; a substituted or unsubstituted aryl group; or a substituted or unsubstituted heteroaryl group, and the others are each independently hydrogen; a substituted or unsubstituted alkyl group; a substituted or unsubstituted aryl group; or a substituted or unsubstituted heteroaryl group,
R5 and R6 are the same as or different from each other, and are each independently a substituted or unsubstituted alkylene group; or a substituted or unsubstituted cycloalkylene group,
R11 and R12 are the same as or different from each other, and are each independently a substituted or unsubstituted alkyl group; a substituted or unsubstituted cycloalkyl group; a substituted or unsubstituted aryl group; or a substituted or unsubstituted heteroaryl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other, and
m and n are each 0 or 1.

However, in the present specification, the case where all of R1 to R4 are an unsubstituted phenyl group is excluded.

According to an exemplary embodiment of the present specification, Chemical Formula 1a is the following Chemical Formula 1a-1.

[Chemical Formula 1a-1]

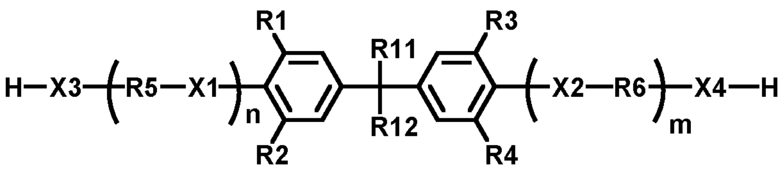

In Chemical Formula 1a-1, the definitions of X1 to X4, R1 to R6, R11, R12, m and n are the same as those defined in Chemical Formula 1a.

According to an exemplary embodiment of the present specification, Chemical Formula 1a is any one of the following Chemical Formulae 1a-2 to 1a-5.

[Chemical Formula 1a-2]

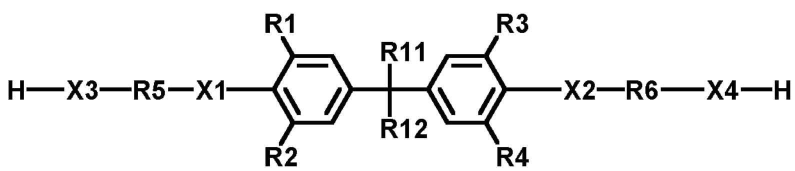

[Chemical Formula 1a-3]

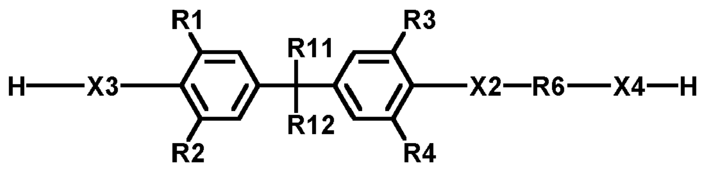

[Chemical Formula 1a-4]

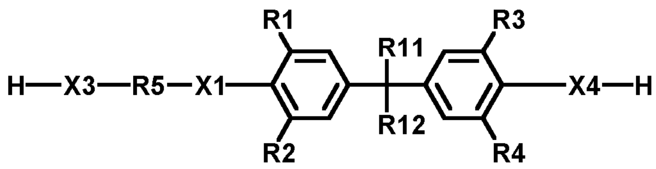

[Chemical Formula 1a-5]

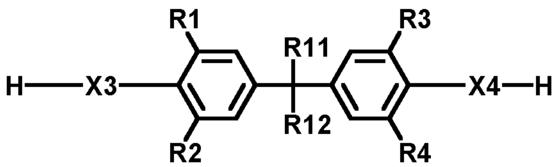

In Chemical Formulae 1a-2 to 1a-5, the definitions of X1 to X4, R1 to R6, R11, R12, m and n are the same as those defined in Chemical Formula 1a.

According to an exemplary embodiment of the present specification, Chemical Formula 1a is the following Chemical Formula 2a.

[Chemical Formula 2a]

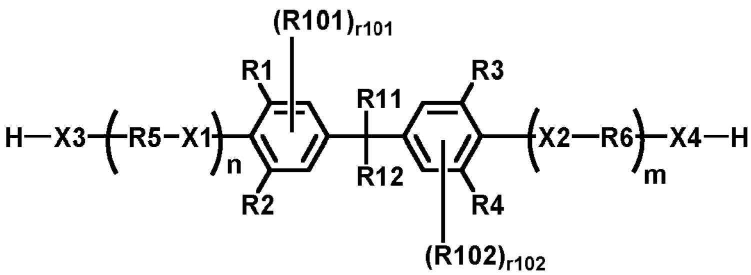

In Chemical Formula 2a,

X1 to X4 are the same as or different from each other, and are each independently O or S,
R1 to R4 are the same as or different from each other, and are each independently a substituted or unsubstituted aryl group; or a substituted or unsubstituted heteroaryl group,
R5 and R6 are the same as or different from each other, and are each independently a substituted or unsubstituted alkylene group; or a substituted or unsubstituted cycloalkylene group,
R11 and R12 are the same as or different from each other, and are each independently a substituted or unsubstituted alkyl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other, and
m and n are each 0 or 1.

In an exemplary embodiment of the present specification, the compound of Chemical Formula 2a may be any one of the following compounds, but is not limited thereto.

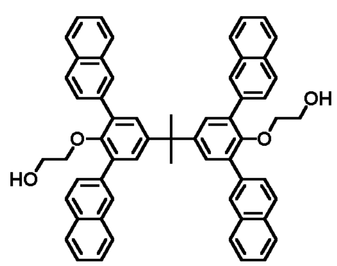

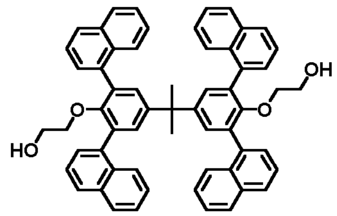

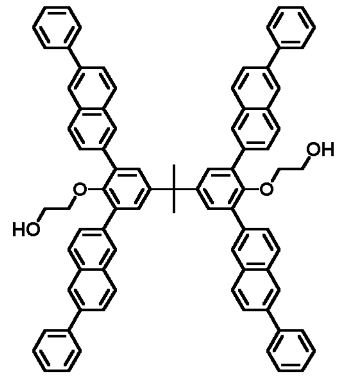

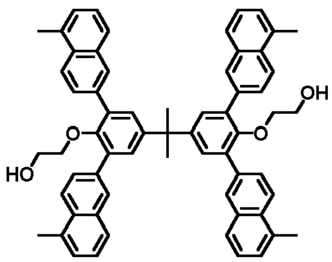

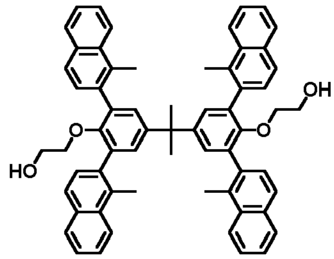

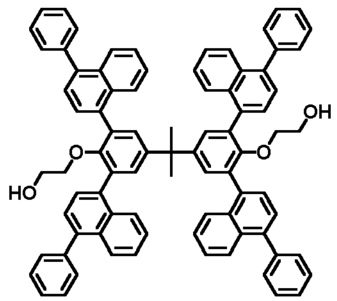

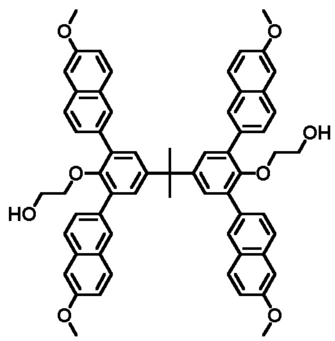

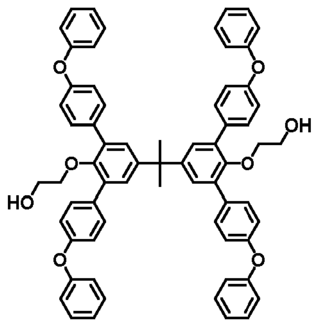

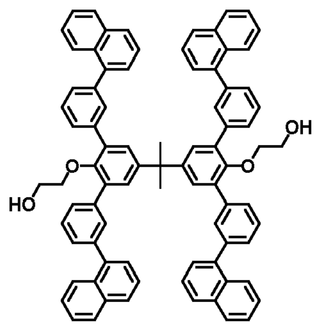

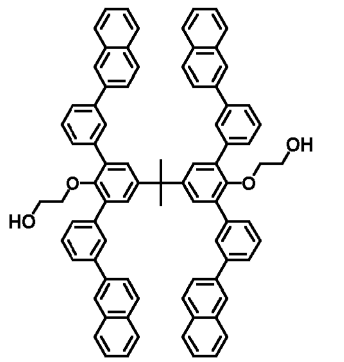

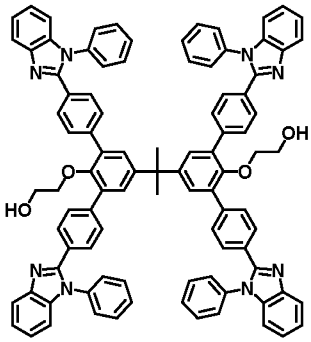

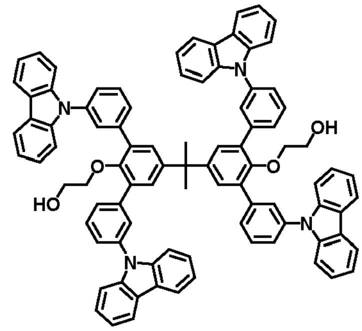

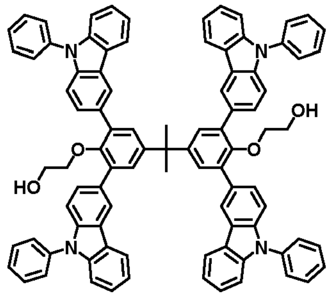

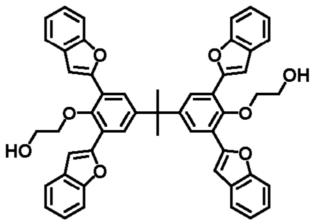

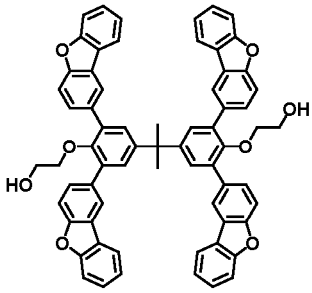

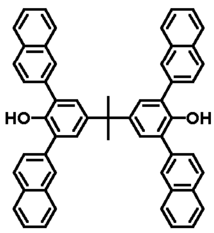

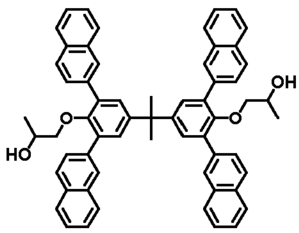

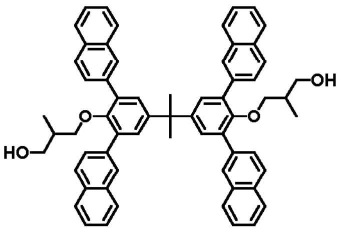

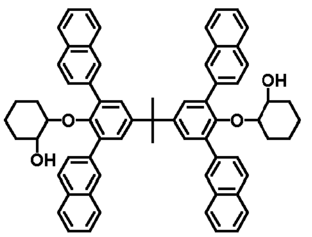

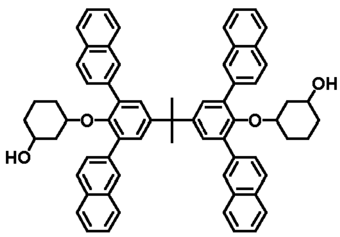

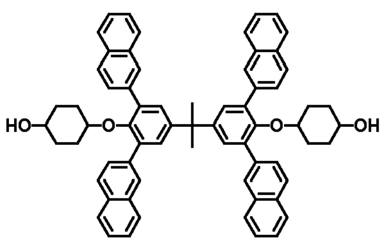

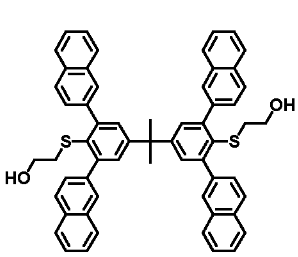

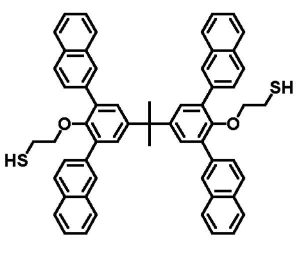

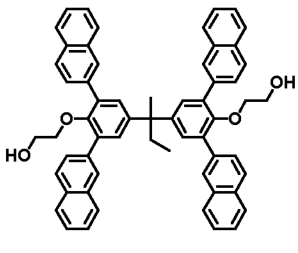

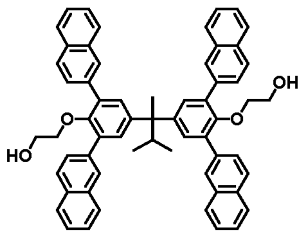

According to an exemplary embodiment of the present specification, Chemical Formula 1a is the following Chemical Formula 3a.

[Chemical Formula 3a]

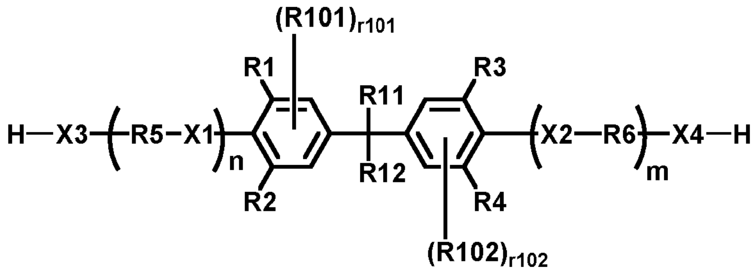

In Chemical Formula 3a,

X1 to X4 are the same as or different from each other, and are each independently O or S,
R1 to R4 are the same as or different from each other, and are each independently a substituted or unsubstituted aryl group; or a substituted or unsubstituted heteroaryl group,
R5 and R6 are the same as or different from each other, and are each independently a substituted or unsubstituted alkylene group; or a substituted or unsubstituted cycloalkylene group,
R11 is a substituted or unsubstituted alkyl group; or a substituted or unsubstituted cycloalkyl group,
R12 is a substituted or unsubstituted aryl group; or a substituted or unsubstituted heteroaryl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other, and
m and n are each 0 or 1.

In an exemplary embodiment of the present specification, the compound of Chemical Formula 3a may be any one of the following compounds, but is not limited thereto.

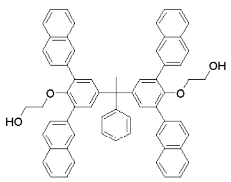

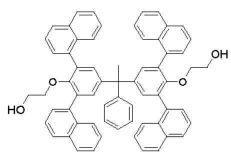

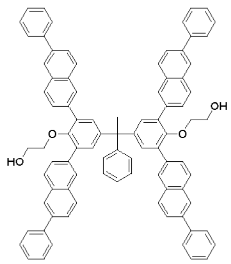

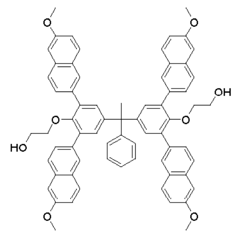

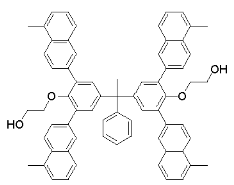

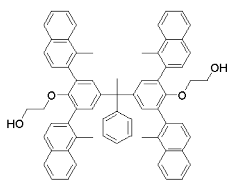

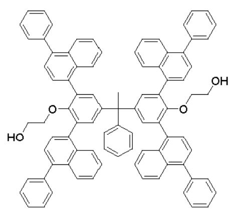

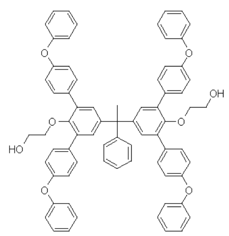
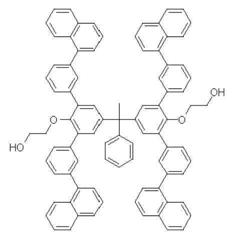
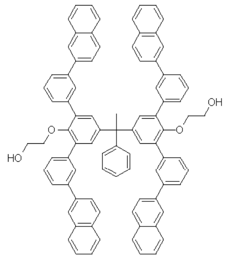
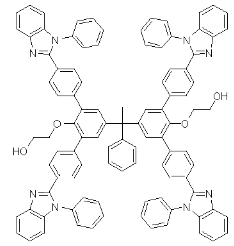

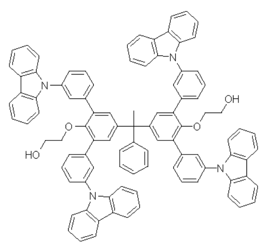
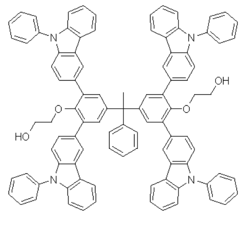
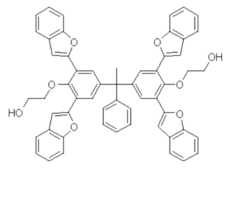
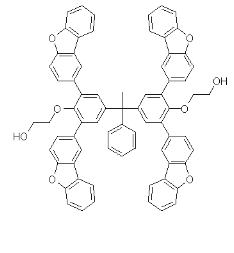

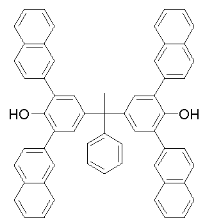
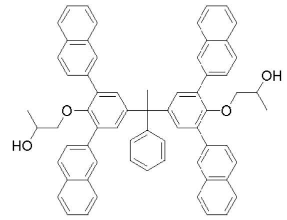
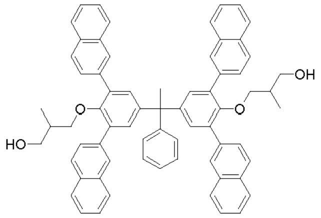

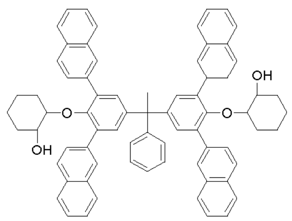
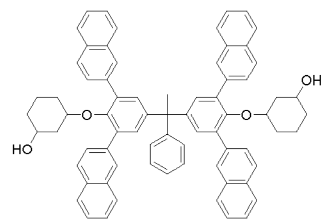
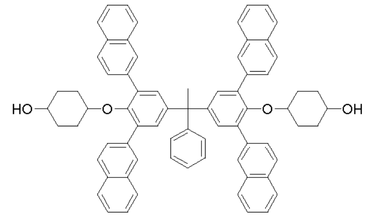

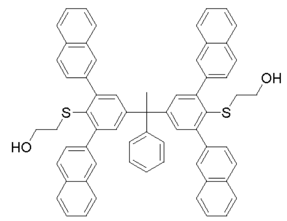
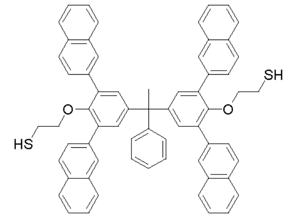

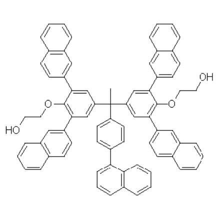
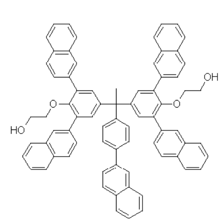
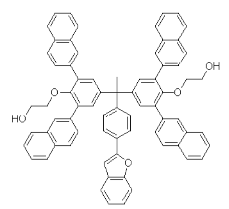
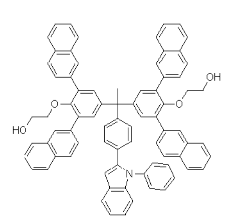

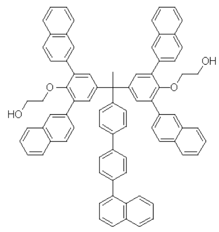
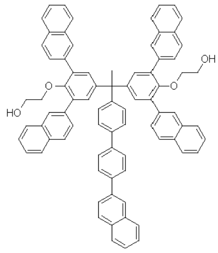
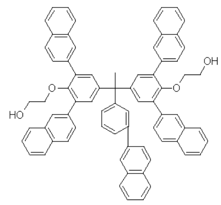

According to an exemplary embodiment of the present specification, Chemical Formula 1a is the following Chemical Formula 4a.

[Chemical Formula 4a]

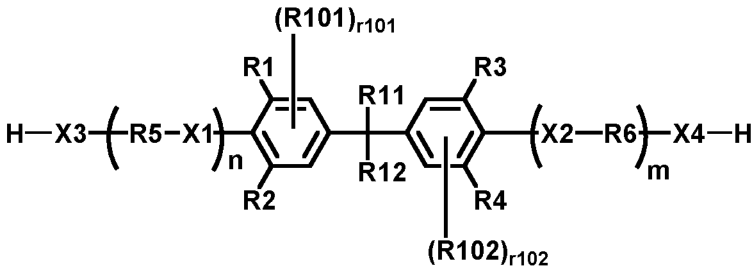

In Chemical Formula 4a,

X1 to X4 are the same as or different from each other, and are each independently O or S,
R1 and R3 are the same as or different from each other, and are each independently a phenyl group which is substituted with one or more of a substituted or unsubstituted aryl group having 10 or more carbon atoms, a substituted or unsubstituted heteroaryl group, and a combination thereof; a substituted or unsubstituted aryl group having 10 or more carbon atoms; or a substituted or unsubstituted heteroaryl group,
R2 and R4 are the same as or different from each other, and are each independently hydrogen; or a substituted or unsubstituted alkyl group,
R5 and R6 are the same as or different from each other, and are each independently a substituted or unsubstituted alkylene group; or a substituted or unsubstituted cycloalkylene group,
R11 and R12 are the same as or different from each other, and are each independently a substituted or unsubstituted alkyl group; or a substituted or unsubstituted cycloalkyl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other, and
m and n are each 0 or 1.

In an exemplary embodiment of the present specification, the compound of Chemical Formula 4a may be any one of the following compounds, but is not limited thereto.

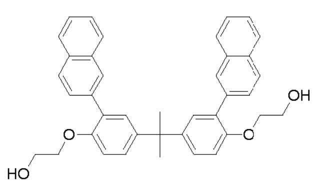
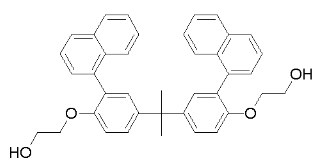
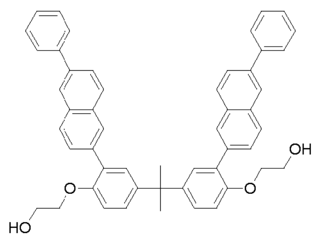

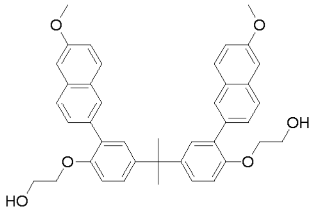
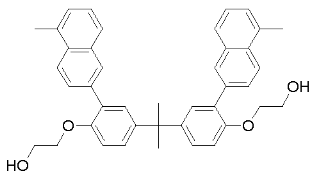
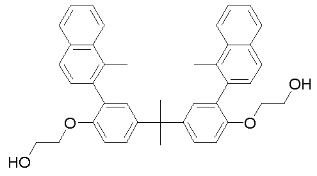

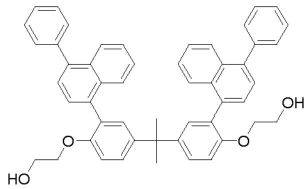
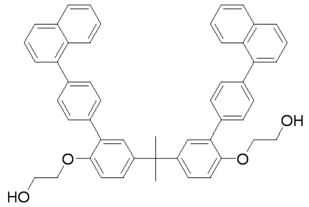
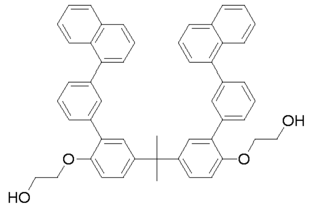

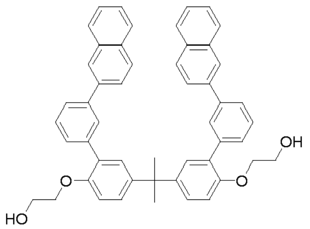
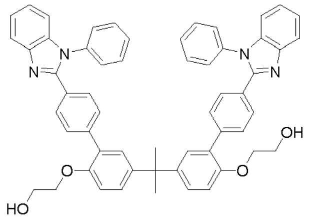
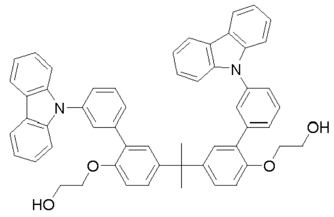

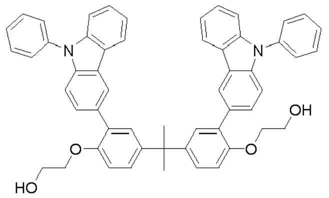
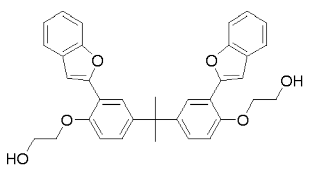
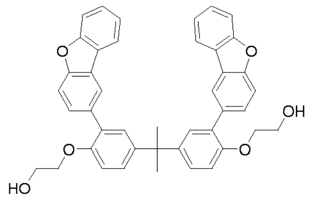

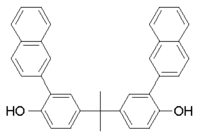
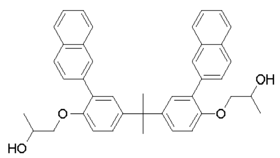
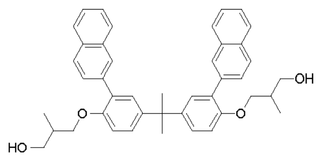

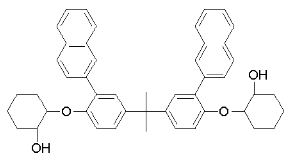
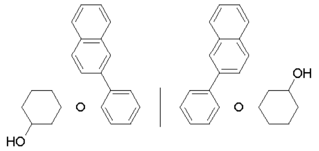
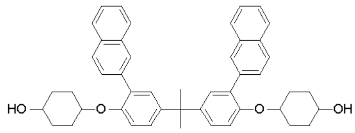

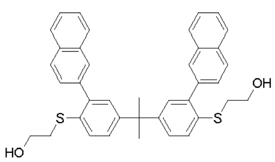
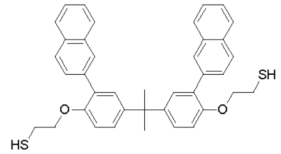

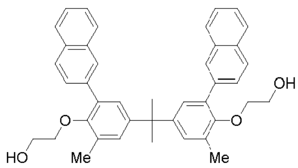
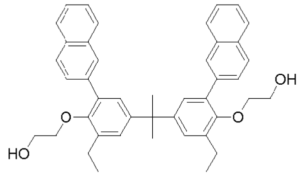
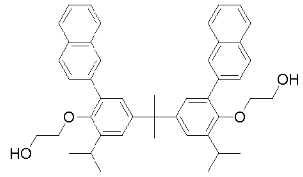

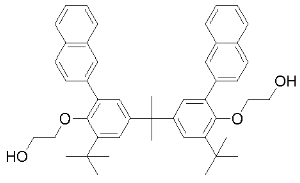
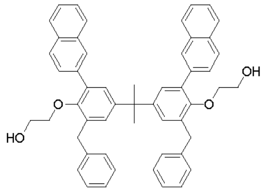

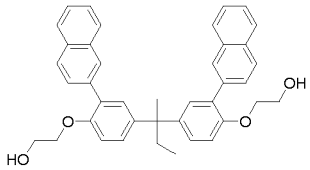
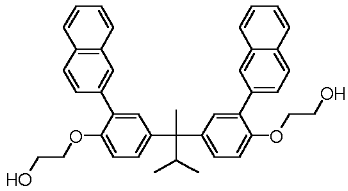
.

According to an exemplary embodiment of the present specification, Chemical Formula 1a is the following Chemical Formula 5a.

[Chemical Formula 5a]

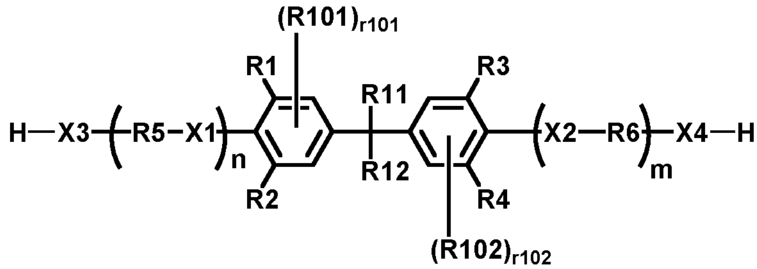

In Chemical Formula 5a,

X1 to X4 are the same as or different from each other, and are each independently O or S,
R1 and R3 are the same as or different from each other, and are each independently a substituted or unsubstituted aryl group; or a substituted or unsubstituted heteroaryl group,
R2 and R4 are the same as or different from each other, and are each independently hydrogen; a substituted or unsubstituted alkyl group; or a substituted or unsubstituted cycloalkyl group,
R5 and R6 are the same as or different from each other, and are each independently a substituted or unsubstituted alkylene group; or a substituted or unsubstituted cycloalkylene group,
R11 is a substituted or unsubstituted alkyl group; or a substituted or unsubstituted cycloalkyl group,
R12 is a substituted or unsubstituted aryl group; or a substituted or unsubstituted heteroaryl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other, and
m and n are each 0 or 1.

In an exemplary embodiment of the present specification, the compound of Chemical Formula 5a may be any one of the following compounds, but is not limited thereto.

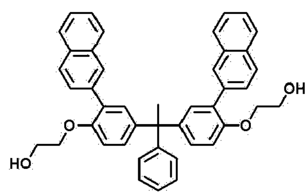
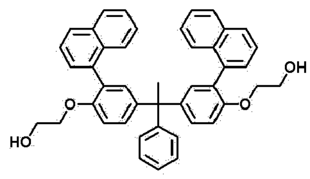
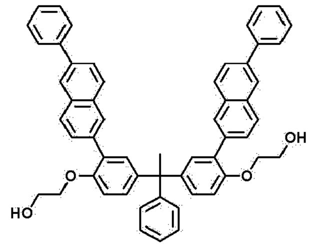
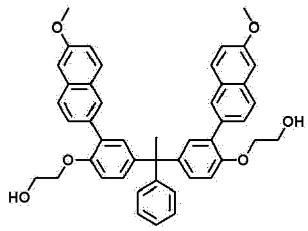
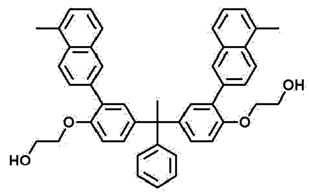
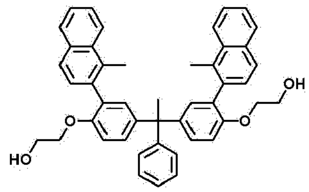
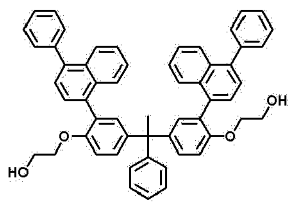
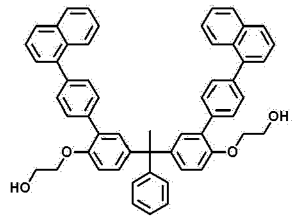
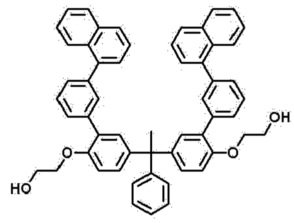
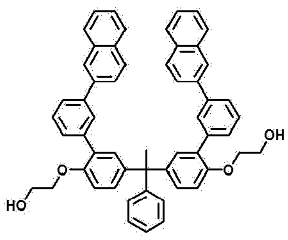
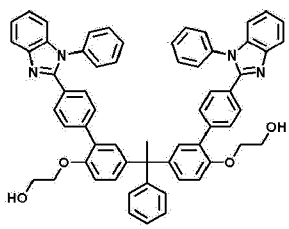
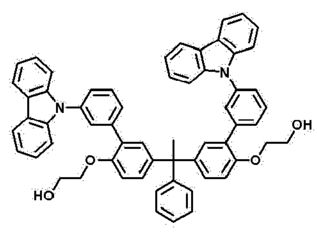
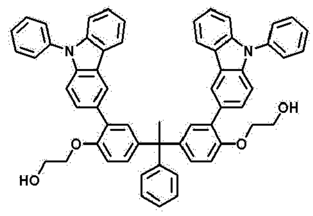
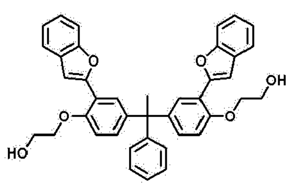
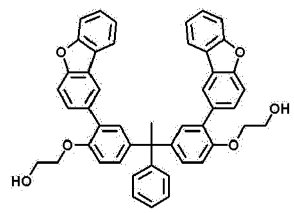
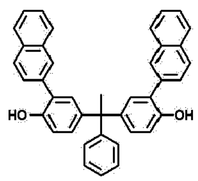
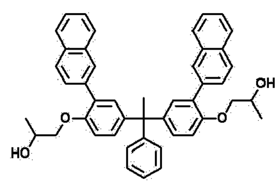
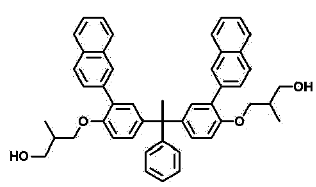
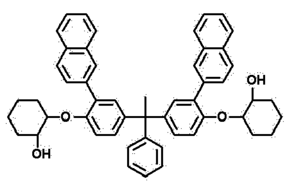
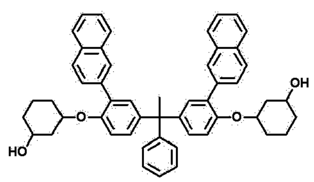
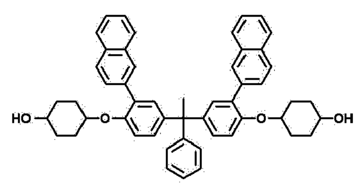

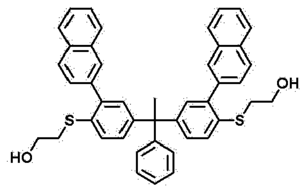
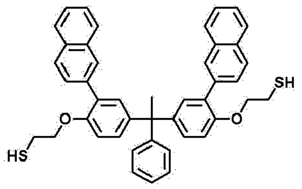
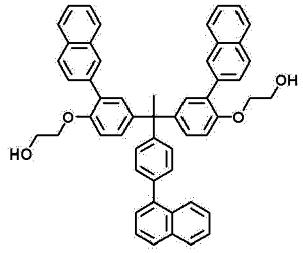

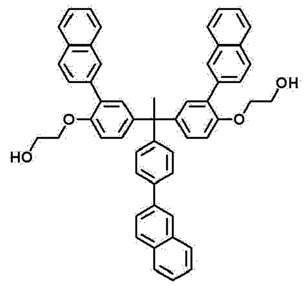
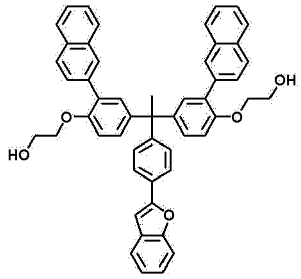
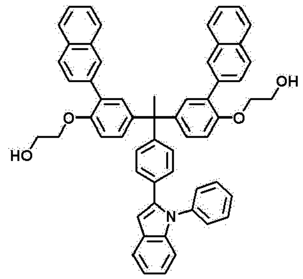

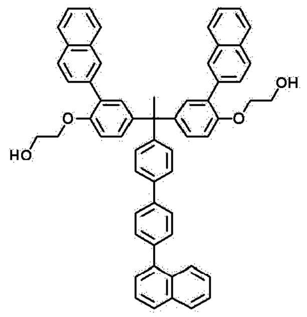
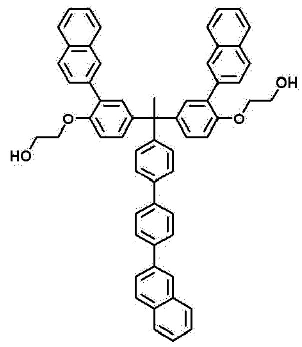
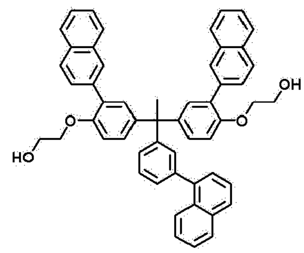

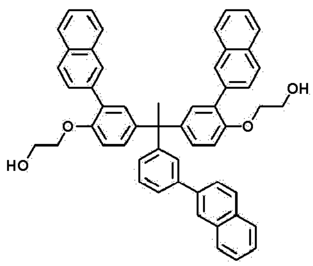
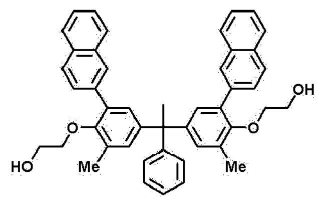
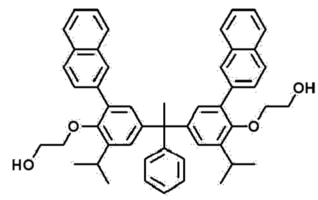

.

An exemplary embodiment of the present specification provides a method for preparing the resin, the method comprising: polymerizing a composition for preparing a resin, which comprises a compound of the following Chemical Formula 1a; and a polyester precursor or a polycarbonate precursor.

[Chemical Formula 1a]

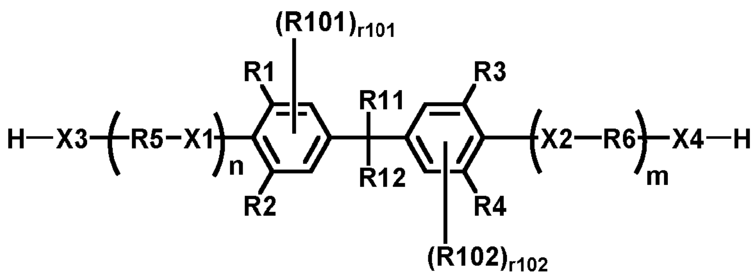

In Chemical Formula 1a,

X1 to X4 are the same as or different from each other, and are each independently O or S,
at least one of R1 to R4 is a substituted or unsubstituted alkyl group; a substituted or unsubstituted aryl group; or a substituted or unsubstituted heteroaryl group, and the others are each independently hydrogen; a substituted or unsubstituted alkyl group; a substituted or unsubstituted aryl group; or a substituted or unsubstituted heteroaryl group,
R5 and R6 are the same as or different from each other, and are each independently a substituted or unsubstituted alkylene group; or a substituted or unsubstituted cycloalkylene group,
R11 and R12 are the same as or different from each other, and are each independently a substituted or unsubstituted alkyl group; a substituted or unsubstituted cycloalkyl group; a substituted or unsubstituted aryl group; or a substituted or unsubstituted heteroaryl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other, and
m and n are each 0 or 1.

When the compound of Chemical Formula 1a is comprised, the compounds are easily polymerized, have a wide range of refractive indices or a high refractive index depending on the substituent, and have a wide range of glass transition temperatures.

The composition for preparing a resin may further comprise a solvent.

The solvent may be, for example, diphenyl ether, dimethylacetamide or methanol, but is not limited thereto, and any solvent applied in the art may be appropriately adopted.

The solvent may be comprised in an amount of 5 parts by weight to 60 parts by weight with respect to 100 parts by weight of the composition for preparing a resin.

The solvent may be comprised in an amount of preferably 5 parts by weight to 50 parts by weight, 7 parts by weight to 45 parts by weight or 8 parts by weight to 40 parts by weight with respect to 100 parts by weight of the composition for preparing a resin.

According to an exemplary embodiment of the present specification, two or more of Chemical Formula 1a may be comprised. Two or more of Chemical Formula 1a are the same as or different from each other.

**In** an exemplary embodiment of the present specification, the compound of Chemical Formula 1a may be comprised in an amount of 1 part by weight to 100 parts by weight or 1 part by weight to 99 parts by weight with respect to 100 parts by weight of the composition for preparing a resin.

Even more specifically, the compound of Chemical Formula 1a may be comprised in an amount of preferably 1 part by weight to 60 parts by weight, 1 part by weight to 50 parts by weight, 1 part by weight to 40 parts by weight, 1 part by weight to 30 parts by weight, 1 part by weight to 20 parts by weight or 1 part by weight to 10 parts by weight with respect to 100 parts by weight of the composition for preparing a resin.

In an exemplary embodiment of the present specification, the polyester precursor or the polycarbonate precursor may be comprised in an amount of 1 part by weight to 60 parts by weight with respect to 100 parts by weight of the composition for preparing a resin.

The polyester precursor or the polycarbonate precursor may be comprised in an amount of preferably 1 part by weight to 60 parts by weight, 1 part by weight to 55 parts by weight, 1 part by weight to 50 parts by weight, 1 part by weight to 45 parts by weight or 1 part by weight to 40 parts by weight with respect to 100 parts by weight of the composition for preparing a resin.

According to an exemplary embodiment of the present specification, the polyester precursor is the following Chemical Formula A, and the polycarbonate precursor is the following Chemical Formula B.

[Chemical Formula A]

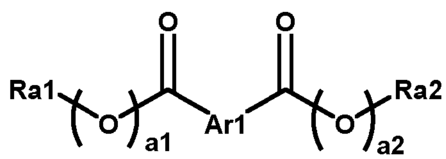

[Chemical Formula B]

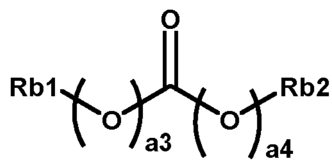

In Chemical Formulae A and B,

Ra1, Ra2, Rb1, and Rb2 are the same as or different from each other, and are each independently a halogen group; a substituted or unsubstituted alkyl group; or a substituted or unsubstituted aryl group,
Ar1 is a substituted or unsubstituted arylene group, and
a1 to a4 are each 0 or 1.

According to an exemplary embodiment of the present specification, Ra1, Ra2, Rb1 and Rb2 are the same as or different from each other, and are each independently a halogen group; a substituted or unsubstituted straight-chained or branched alkyl group having 1 to 30 carbon atoms; or a substituted or unsubstituted monocyclic or polycyclic aryl group having 6 to 30 carbon atoms.
According to an exemplary embodiment of the present specification, Ra1, Ra2, Rb1 and Rb2 are the same as or different from each other, and are each independently a halogen group; a substituted or unsubstituted straight-chained or branched alkyl group having 1 to 20 carbon atoms; or a substituted or unsubstituted monocyclic or polycyclic aryl group having 6 to 20 carbon atoms.
According to an exemplary embodiment of the present specification, Ra1, Ra2, Rb1 and Rb2 are the same as or different from each other, and are each independently a halogen group; a straight-chained or branched alkyl group having 1 to 30 carbon atoms, which is unsubstituted or substituted with a hydroxyl group; or a monocyclic or polycyclic aryl group having 6 to 30 carbon atoms.
According to an exemplary embodiment of the present specification, Ra1, Ra2, Rb1 and Rb2 are the same as or different from each other, and are each independently a halogen group; a straight-chained or branched alkyl group having 1 to 20 carbon atoms, which is unsubstituted or substituted with a hydroxyl group; or a monocyclic or polycyclic aryl group having 6 to 20 carbon atoms.
According to an exemplary embodiment of the present specification, Ra1, Ra2, Rb1 and Rb2 are the same as or different from each other, and are each independently -Cl; a methyl group; an ethyl group which is unsubstituted or substituted with a hydroxyl group; an n-propyl group; an n-butyl group; or a phenyl group.
In an exemplary embodiment of the present specification, Ra1 and Ra2 are the same as or different from each other, and are each independently -Cl; or an ethyl group substituted with a hydroxyl group.
In an exemplary embodiment of the present specification, Ra1 and Ra2 are -Cl.
In an exemplary embodiment of the present specification, Ra1 and Ra2 are a methyl group.
In an exemplary embodiment of the present specification, Ra1 and Ra2 are an ethyl group substituted with a hydroxyl group.
According to an exemplary embodiment of the present specification, Rb1 and Rb2 are the same as or different from each other, and are each independently -Cl; a methyl group; an ethyl group; an n-propyl group; an n-butyl group; or a phenyl group.
In an exemplary embodiment of the present invention, Ar1 is a substituted or unsubstituted arylene group having 6 to 30 carbon atoms.
In an exemplary embodiment of the present invention, Ar1 is a substituted or unsubstituted arylene group having 6 to 20 carbon atoms.
In an exemplary embodiment of the present invention, Ar1 is a substituted or unsubstituted arylene group having 6 to 12 carbon atoms.
In an exemplary embodiment of the present invention, Ar1 is a substituted or unsubstituted phenylene group.
In an exemplary embodiment of the present invention, Ar1 is a phenylene group.
According to an exemplary embodiment of the present specification, Chemical Formula A is the following compound.

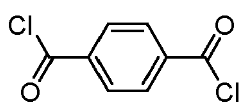

According to an exemplary embodiment of the present specification, Chemical Formula B is any one selected among the following compounds.

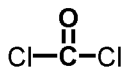
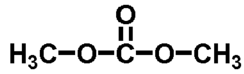
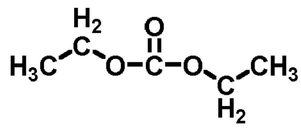
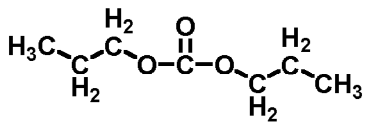
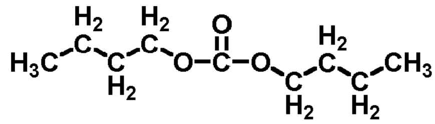
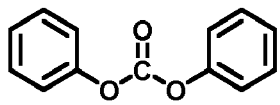

The polycarbonate precursor serves to link an additional comonomer, if necessary, and other specific examples thereof which may be applied in addition to the compound of Chemical Formula B comprise phosgene, triphosgene, diphosgene, bromophosgene, dimethyl carbonate, diethyl carbonate, dibutyl carbonate, dicyclohexyl carbonate, ditolyl carbonate, bis(chlorophenyl) carbonate, m-cresyl carbonate, dinaphthyl carbonate, bis(diphenyl) carbonate, bishaloformate, or the like, and any one of them or a mixture of two or more thereof may be used.

The unit of the above-described Chemical Formula 1 may be formed by polymerizing the compound of Chemical Formula 1a with the polyester precursor of Chemical Formula A or the polycarbonate precursor of Chemical Formula B.

In an exemplary embodiment of the present specification, it is preferred that the resin is polymerized from the compound of Chemical Formula 1a and the polyester precursor of Chemical Formula A.

In an exemplary embodiment of the present specification, it is preferred that the resin is polymerized from the compound of Chemical Formula 1a and the polycarbonate precursor of Chemical Formula B.

The compound of Chemical Formula 1a may be used in an amount of 1 part by mol to 100 parts by mol, and 1 part by mol to 99 parts by mol with respect to 100 parts by mol of the entire monomer constituting the resin comprising the unit of Chemical Formula 1.

The polyester precursor of Chemical Formula A or the polycarbonate precursor of Chemical Formula B may be used in an amount of 50 parts by mol to 150 parts by mol with respect to 100 parts by mol of the entire monomer of the compound of Chemical Formula 1a, which constitutes the resin.

For the polymerization of the resin according to the present specification, methods known in the art may be used.

It is preferred that the polymerization is performed by a melt polycondensation method.

In the melt polycondensation method, a catalyst may be further applied as needed, and melt polycondensation may be performed using the composition for preparing a resin under heating and further under normal pressure or reduced pressure while removing byproducts by an ester exchange reaction. As the catalyst, a material generally applied in the art may be adopted.

Specifically, in the melt polycondensation method, it is preferred that the compound of Chemical Formula 1a; and the polyester precursor or polycarbonate precursor are melted in a reaction vessel, and then a reaction is performed in a state where a by-product compound is allowed to stay.

In order to allow the by-product compound to stay, the reaction device may be closed, or pressure may be controlled by reducing pressure or increasing pressure.

The reaction time of this process is 20 minutes or more and 600 minutes or less, preferably 40 minutes or more and 450 minutes or less, and more preferably 60 minutes or more and 300 minutes or less.

In this case, when the by-product compound is distilled off immediately after being produced, a resin to be finally obtained has a small content of high molecular weight materials. However, when the by-product compound is allowed to stay in the reaction vessel for a certain period of time, the finally obtained resin is obtained to have a large content of high molecular weight materials.

The melt polycondensation method may be performed continuously or in a batch manner. The reaction device used for performing the reaction may be a vertical type equipped with an anchor type impeller, a Maxblend impeller, a helical ribbon type impeller or the like, may be a horizontal type equipped with a paddle blade, a lattice blade, a spectacle-shaped blade or the like, and may be an extruder type equipped with a screw. **In** addition, it is desirably performed to use a reaction device in which these reaction devices are appropriately combined in consideration of the viscosity of the polymer.

In the method for preparing a resin used in the present specification, the catalyst may be removed or deactivated in order to maintain heat stability and hydrolysis stability after the completion of the polymerization reaction. A method of deactivating the catalyst by adding a known acidic material in the art may be preferably performed.

As the acidic material, for example, esters such as butyl benzoate, aromatic sulfonic acids such as p-toluenesulfonic acid; aromatic sulfonic acid esters such as butyl p-toluenesulfonate and hexyl p-toluenesulfonate; phosphoric acids such as phosphorous acid, phosphoric acid and phosphonic acid; phosphorous acid esters such as triphenyl phosphite, monophenyl phosphite, diphenyl phosphite, diethyl phosphite, di-n-propyl phosphite, di-n-butyl phosphite, di-n-hexyl phosphite, dioctyl phosphite and monooctyl phosphite; phosphoric acid esters such as triphenyl phosphate, diphenyl phosphate, monophenyl phosphate, dibutyl phosphate, dioctyl phosphate and monooctyl phosphate; phosphonic acids such as diphenylphosphonic acid, dioctylphosphonic acid and dibutylphosphonic acid; phosphonic acid esters such as diethyl phenylphosphonate; phosphines such as triphenylphosphine and bis(diphenylphosphino)ethane; boric acids such as boric acid and phenylboric acid; aromatic sulfonic acid salts such as dodecylbenzenesulfonic acid tetrabutylphosphonium salts; organic halides such as stearic acid chloride, benzoyl chloride and p-toluenesulfonic acid chloride; alkylsulfuric acids such as dimethylsulfuric acid; organic halides such as benzyl chloride, and the like are preferably used.

The acidic material may be used in an amount of 0.1 parts by mol to 5 parts by mol, preferably 0.1 parts by mol to 1 part by mol with respect to 100 parts by mol of the catalyst.

When the amount of the acidic material is smaller than 0.1 parts by mol, the deactivation effect becomes insufficient, which is not preferred. Further, when the amount exceeds 5 parts by mol, the heat resistance of the resin deteriorates and the molded article is easily colored, which is not preferred.

After the catalyst is deactivated, a process of devolatilizing a low boiling point compound in the resin may be further performed under a pressure of 0.1 mmHg to 1 mmHg and at a temperature of 200°C to 350°C. In this process, a horizontal-type apparatus equipped with a stirring blade having excellent surface renewal ability such as a paddle blade, a lattice blade, and a spectacle-shaped blade, or a thin film evaporator is preferably used.

It is preferred that the content of foreign materials in the resin of the present specification is as small as possible, and filtration of a melting raw material, filtration of a catalyst solution, and the like are preferably performed.

The mesh of the filter used in the filtration is preferably 5 μm or less, and more preferably 1 μm or less. In addition, filtration of the produced resin using a polymer filter is preferably performed. The mesh of the polymer filter is preferably 100 μm or less, and more preferably 30 μm or less. Furthermore, a process of obtaining a resin pellet needs to be performed in a low-dust environment, and the environment is preferably Class 6 or lower, and more preferably Class 5 or lower.

Further, examples of a method of molding a molded article comprising the resin comprise compression molding, molds, roll processing, extrusion molding, stretching, and the like in addition to injection molding, but are not limited thereto.

Another exemplary embodiment of the present specification provides a resin composition comprising the resin according to the above-described exemplary embodiments.

In an exemplary embodiment of the present specification, the resin may be comprised in an amount of 1 part by weight to 80 parts by weight based on 100 parts by weight of the resin composition.

In an exemplary embodiment of the present specification, the resin composition may further comprise a solvent. The solvent may be, for example, dimethylacetamide or 1,2-dichlorobenzene.

The solvent may be comprised in an amount of 20 parts by weight to 99 parts by weight based on 100 parts by weight of the resin composition.

The resin composition may further comprise an additional monomer in addition to the compound of Chemical Formula 1a. The additional monomer is not particularly limited, and a monomer generally applied in the art related to polyester or polycarbonate may be appropriately adopted as long as the main physical properties of the resin composition are not changed. The additional monomer may be used in an amount of 1 part by mol to 50 parts by mol with respect to 100 parts by mol of the entire monomer constituting the resin comprising the unit of Chemical Formula 1.

The resin composition may further comprise one or more selected from the group consisting of an additive, for example, an antioxidant, a plasticizer, an anti-static agent, a nucleating agent, a flame retardant, a lubricant, an impact modifier, a fluorescent brightener, a UV absorber, a pigment and a dye, if necessary, in addition to a resin comprising the unit of Chemical Formula 1.

The additive may be comprised in an amount of 1 part by weight to 99 parts by weight based on 100 parts by weight of the resin composition.

The type of antioxidant, plasticizer, anti-static agent, nucleating agent, flame retardant, lubricant, impact modifier, fluorescent brightener, UV absorber, pigment or dye is not particularly limited, and those applied in the art may be appropriately adopted.

Still another exemplary embodiment of the present specification provides a molded article comprising the resin composition according to the above-described exemplary embodiments.

**In** an exemplary embodiment of the present specification, the molded article may be prepared from the resin

composition or a cured product thereof.

As an example of a method of preparing the molded article, it is possible to comprise mixing a resin comprising the unit of Chemical Formula 1 and the additive well using a mixer, preparing the resulting mixture as a pellet by extrusion molding the mixture using an extruder, drying the pellet, and then injecting the pellet using an injection molding machine.

In an exemplary embodiment of the present specification, the molded article is an optical member.

In an exemplary embodiment of the present specification, the molded article is an optical lens.

An optical lens according to an exemplary embodiment of the present specification has a high refractive index, and thus may implement an optical lens with a small thickness.

The optical lens is manufactured using the resin, has a small thickness, a high refractive index and high transparency, and may be preferably applied to a camera.

[Mode for Invention]

Hereinafter, the present specification will be exemplified in more detail through Examples.

**<Preparation Examples>**

**<Synthesis of Monomer 1-1>**

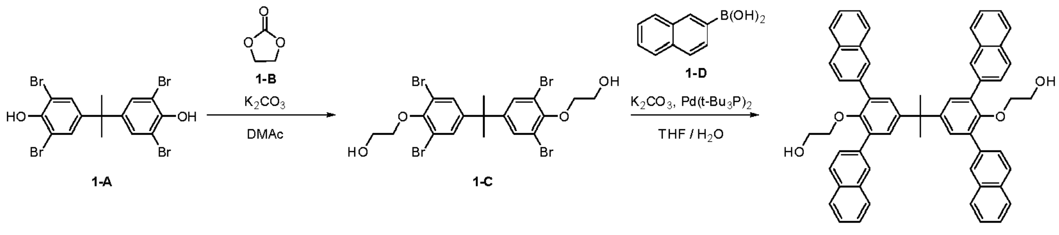

1-A 1-C Monomer 1-1

1) Synthesis of Compound 1-C

100.0 g (183 mmol, 1.0 eq) of Compound 1-A, 48.57 g (552 mmol, 3.0 eq) of Compound 1-B, and 20.33g (147 mmol, 0.80 eq) of $K_2CO_3$ were dissolved in 400 g of dimethylacetamide (DMAc), and the resulting solution was stirred in an oil bath at 120°C for 2 hours. After cooling, a solid was precipitated by adding water thereto, and then filtered. The obtained solid was purified by column chromatography through ethyl acetate (EA) and dichloromethane (DCM), and then precipitated in n-hexane (n-Hex) to obtain 60 g of Compound 1-C as a white solid.

2) Synthesis of Monomer 1-1

50.0 g (79 mmol, 1.0 eq) of Compound 1-C and 56.06 g (326 mmol, 4.12 eq) of Compound 1-D were dissolved in 510 g of tetrahydrofuran (THF), and the resulting solution was stirred in an oil bath at 80°C for 30 minutes. After 56.86 g (411 mmol, 5.20 eq) of $K_2CO_3$ was dissolved in 489 mL of water, the solution was added dropwise thereto for 10 minutes while maintaining the internal temperature of the solution at 50°C or higher. 0.20 g (0.4 mmol, 0.005 eq) of a Pd(t-$Bu_3P)_2$ catalyst was added thereto at an internal temperature of 60°C. After being stirred for 1 hour, the mixture was washed with ethyl acetate (EA)/$H_2O$ to separate the organic layer, and the solvent was concentrated under vacuum. Purification was performed by column chromatography through n-hexane (n-Hex) and dichloromethane (DCM), and then precipitated in n-hexane (n-Hex) to obtain 58 g of Monomer 1-1 as a white solid.

MS: $[M+H]^+$=820

**<Synthesis of Monomer 1-2>**

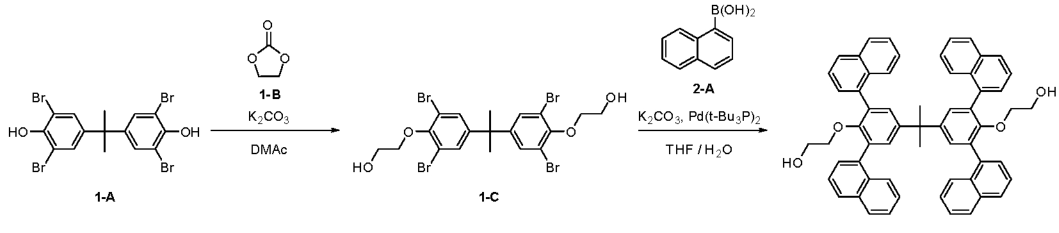


Monomer 1-2

Monomer 1-2 was obtained in the same manner as in the synthesis of Monomer 1-1, except that Compound 2-A was used instead of Compound 1-D.
MS: $[M+H]^+$=664

## <Synthesis of Monomer 1-3>

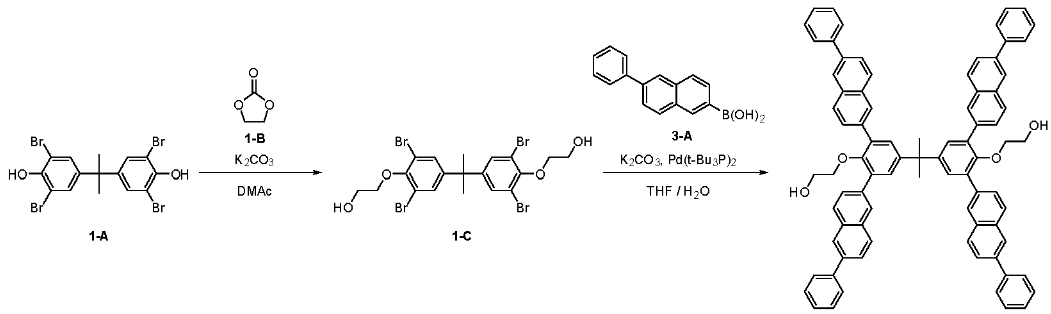


Monomer 1-3

Monomer 1-3 was obtained in the same manner as in the synthesis of Monomer 1-1, except that Compound 3-A was used instead of Compound 1-D.
MS: $[M+H]^+$=1124

## <Synthesis of Monomer 1-4>

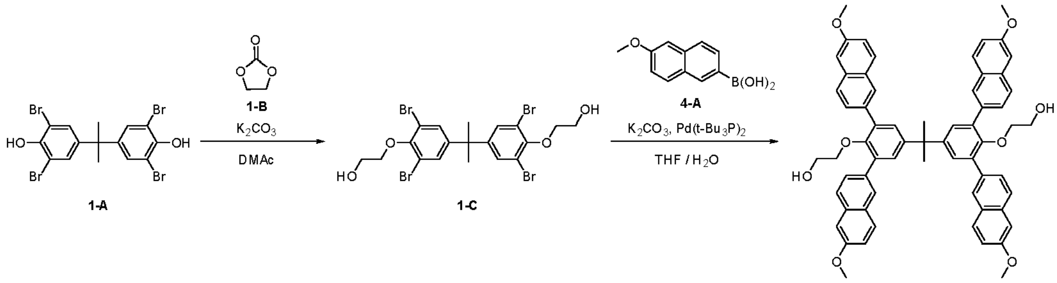


Monomer 1-4

Monomer 1-4 was obtained in the same manner as in the synthesis of Monomer 1-1, except that Compound 4-A was used instead of Compound 1-D.
MS: $[M+H]^+$=940

## <Synthesis of Monomer 1-5>

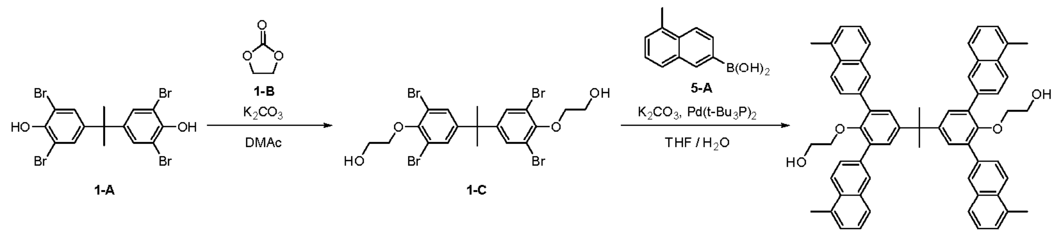

Monomer 1-5

Monomer 1-5 was obtained in the same manner as in the synthesis of Monomer 1-1, except that Compound 5-A was used instead of Compound 1-D.
MS: $[M+H]^+$=876

**<Synthesis of Monomer 1-6>**

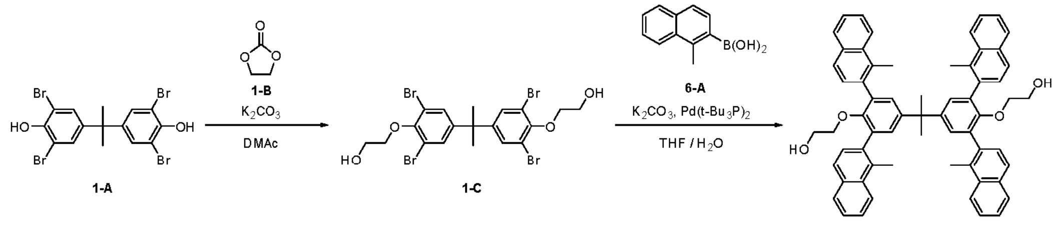

Monomer 1-6

Monomer 1-6 was obtained in the same manner as in the synthesis of Monomer 1-1, except that Compound 6-A was used instead of Compound 1-D.
MS: $[M+H]^+$=876

**<Synthesis of Monomer 1-7>**

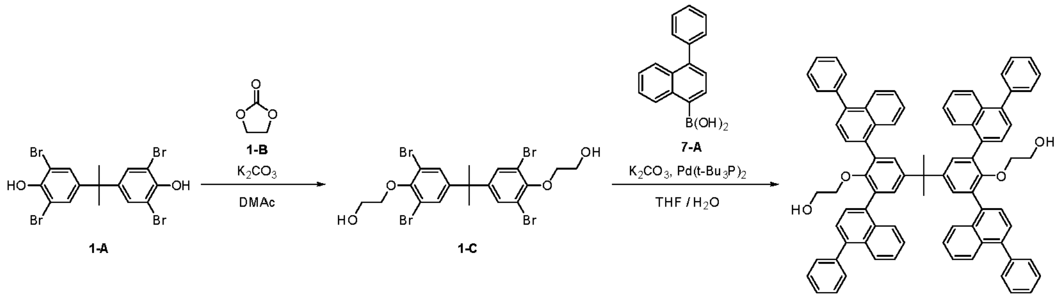

Monomer 1-7

Monomer 1-7 was obtained in the same manner as in the synthesis of Monomer 1-1, except that Compound 7-A was used instead of Compound 1-D.
MS: $[M+H]^+$=1124

**<Synthesis of Monomer 1-8>**

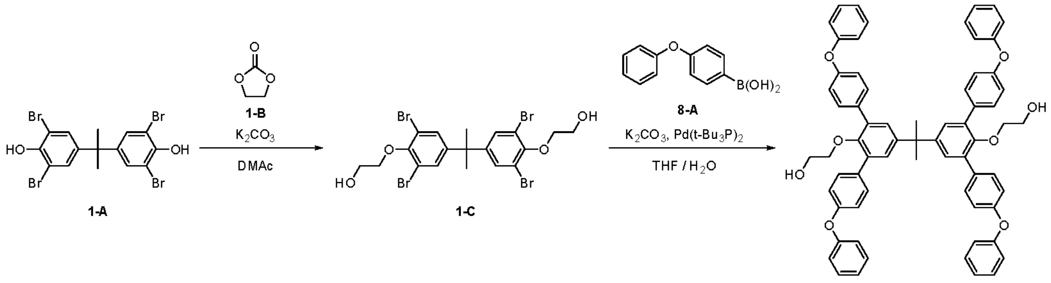


Monomer 1-8

Monomer 1-8 was obtained in the same manner as in the synthesis of Monomer 1-1, except that Compound 8-A was used instead of Compound 1-D.
MS: $[M+H]^+$=988

**<Synthesis of Monomer 1-9>**

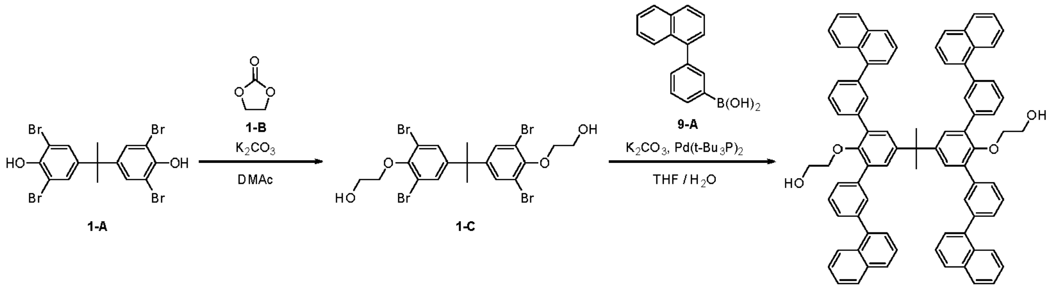


Monomer 1-9

Monomer 1-9 was obtained in the same manner as in the synthesis of Monomer 1-1, except that Compound 9-A was used instead of Compound 1-D.
MS: $[M+H]^+$=1124

**<Synthesis of Monomer 1-10>**

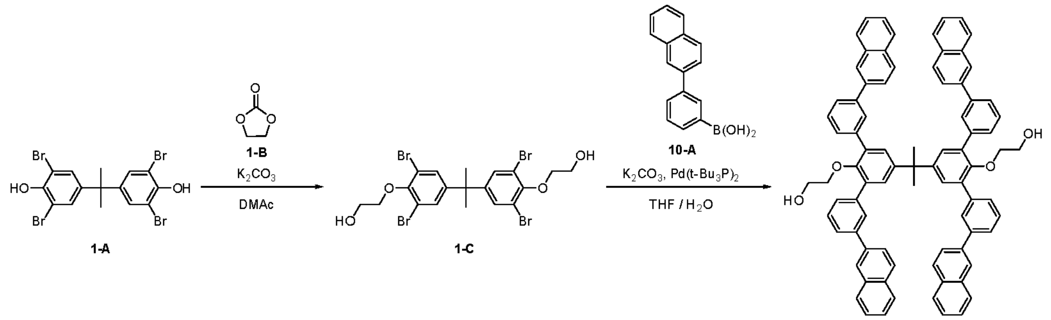


Monomer 1-10

Monomer 1-10 was obtained in the same manner as in the synthesis of Monomer 1-1, except that Compound 10-A was used instead of Compound 1-D.
MS: $[M+H]^+$=1124

**<Synthesis of Monomer 1-11>**

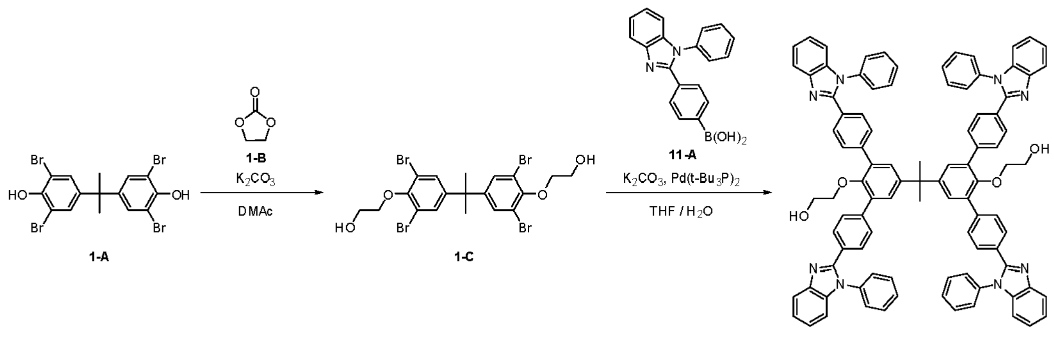


Monomer 1-11

Monomer 1-11 was obtained in the same manner as in the synthesis of Monomer 1-1, except that Compound 11-A was used instead of Compound 1-D.
MS: $[M+H]^+$=1388

**<Synthesis of Monomer 1-12>**

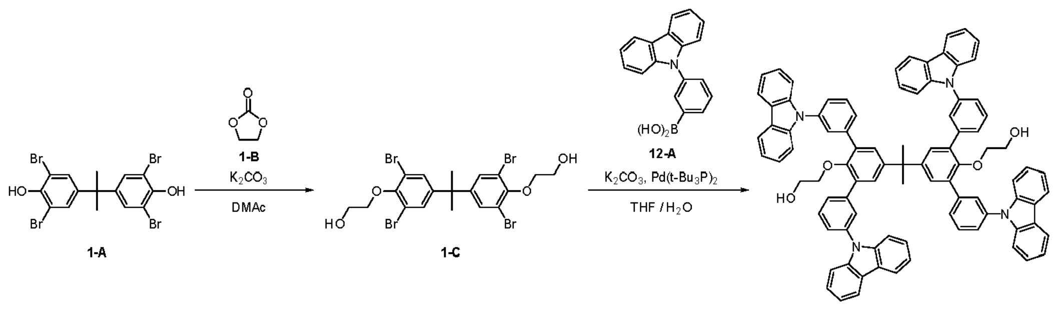


Monomer 1-12

Monomer 1-12 was obtained in the same manner as in the synthesis of Monomer 1-1, except that Compound 12-A was used instead of Compound 1-D.
MS: $[M+H]^+$=1280

**<Synthesis of Monomer 1-13>**

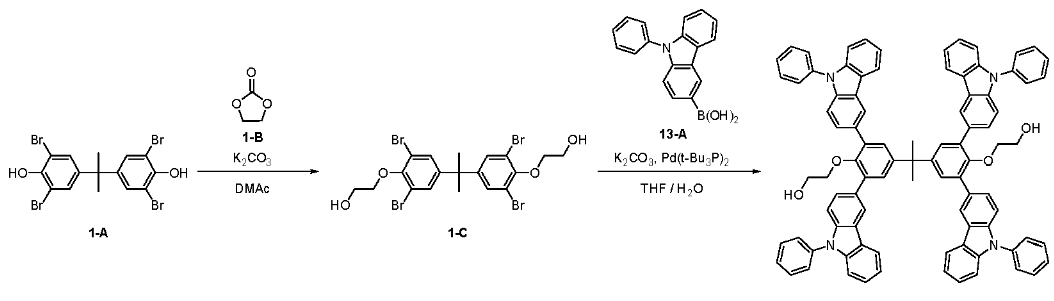


Monomer 1-13

Monomer 1-13 was obtained in the same manner as in the synthesis of Monomer 1-1, except that Compound 13-A was used instead of Compound 1-D.
MS: $[M+H]^+$=1280

**<Synthesis of Monomer 1-14>**

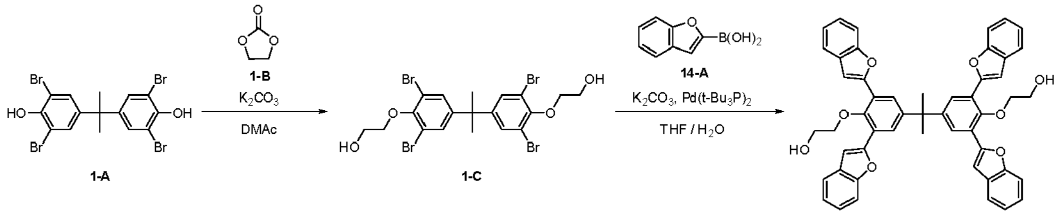


Monomer 1-14

Monomer 1-14 was obtained in the same manner as in the synthesis of Monomer 1-1, except that Compound 14-A was used instead of Compound 1-D.
MS: $[M+H]^+$=780

**<Synthesis of Monomer 1-15>**

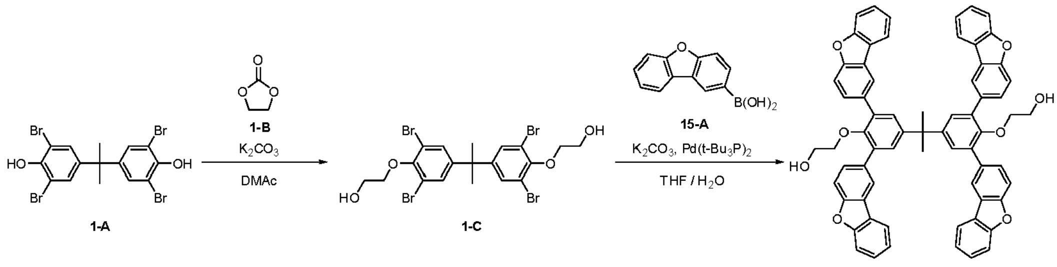


Monomer 1-15

Monomer 1-15 was obtained in the same manner as in the synthesis of Monomer 1-1, except that Compound 15-A was used instead of Compound 1-D.
MS: $[M+H]^+$=980

**<Synthesis of Monomer 1-16>**

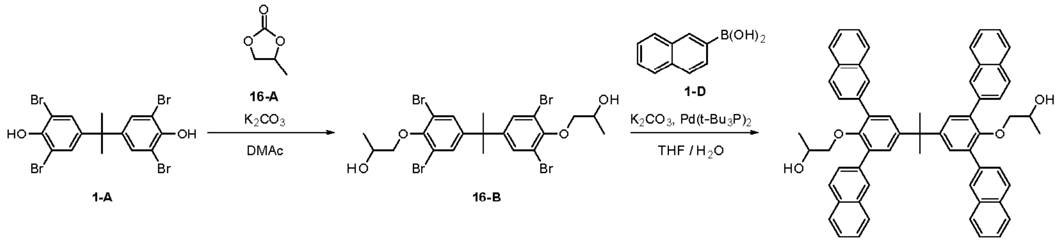


Monomer 1-16

Monomer 1-16 was obtained in the same manner as in the synthesis of Monomer 1-1, except that Compound 16-A was used instead of Compound 1-D.
MS: $[M+H]^+$=848

**<Synthesis of Monomer 1-17>**

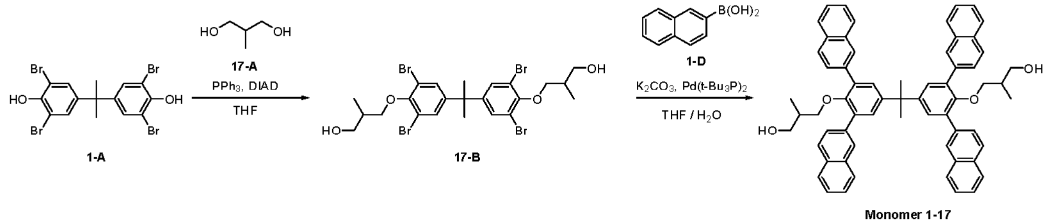

1-A 17-B Monomer 1-17

1) Synthesis of Compound 17-B

50.0 g (91.5 mmol, 1.0 eq) of Compound 1-A, 17.32 g (192 mmol, 2.1 eq) of Compound 17-A, and 50.36 g (192 mmol, 2.1 eq) of $PPh_3$ were dissolved in 2,000 g of tetrahydrofuran (THF), and 38.82 g (192 mmol, 2.1 eq) of diisopropyl azodicarboxylate was added thereto for 70 minutes. After the addition, the resulting mixture was stirred at room temperature for 12 hours. Thereafter, the mixture was purified by column chromatography through ethyl acetate (EA) and dichloromethane (DCM) after concentration, and then precipitated in n-hexane (n-Hex) to obtain 48 g of Compound 17-B as a white solid.

2) Synthesis of Monomer 1-17

Monomer 1-17 was obtained in the same manner as in the synthesis of Monomer 1-1, except that Compound 17-B was used instead of Compound 1-C.
MS: $[M+H]^+$=876

**<Synthesis of Monomer 1-18>**

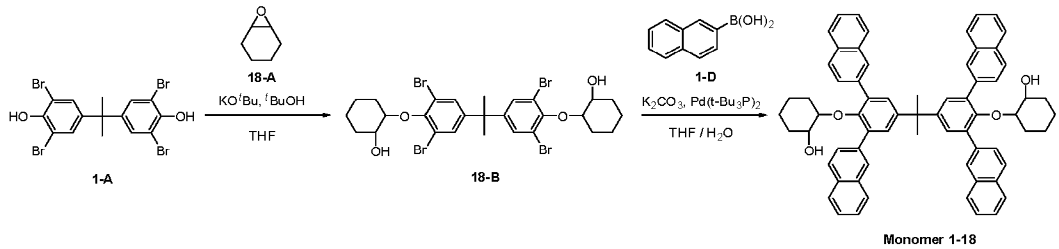

1-A 18-B Monomer 1-18

1) Synthesis of Compound 18-B

50 g (91.5 mmol, 1.0 eq) of Compound 1-A was dissolved in 500 g of tetrahydrofuran (THF) and 500 g of tert-BuOH, and 21.56 g (192 mmol, 2.1 eq) of KOtBu was added thereto. After the addition, 18.84 g (192 mmol, 2.0 eq) of Compound 18-A was slowly added thereto. Thereafter, the temperature was raised and the mixture was stirred under reflux for 72 hours. After cooling, the mixture was purified by column chromatography through ethyl acetate (EA) and dichloromethane (DCM) after concentration, and then precipitated in n-hexane (n-Hex) to obtain 45 g of Compound 18-B as a white solid.

2) Synthesis of Monomer 1-18

Monomer 1-18 was obtained in the same manner as in the synthesis of Monomer 1-1, except that Compound 18-B was used instead of Compound 1-C.
MS: $[M+H]^+$=928

**<Synthesis of Monomer 1-19>**

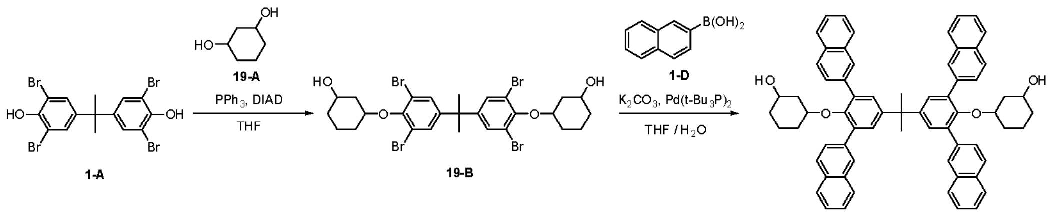


Monomer 1-19

Monomer 1-19 was obtained in the same manner as in the synthesis of Monomer 1-17, except that Compound 19-A was used instead of Compound 17-A.
MS: $[M+H]^+$=928

**<Synthesis of Monomer 1-20>**

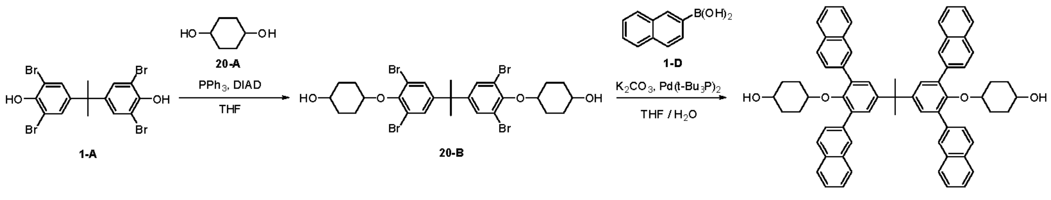


Monomer 1-20

Monomer 1-20 was obtained in the same manner as in the synthesis of Monomer 1-17, except that Compound 20-A was used instead of Compound 17-A.
MS: $[M+H]^+$=928

**<Synthesis of Monomer 1-21>**

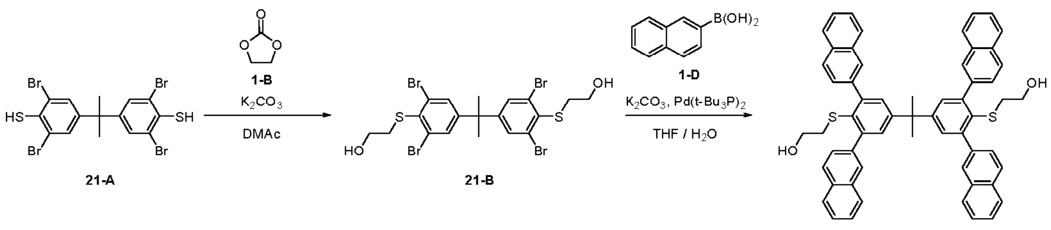


Monomer 1-21

Monomer 1-21 was obtained in the same manner as in the synthesis of Monomer 1-1, except that Compound 21-A was used instead of Compound 1-A.
MS: $[M+H]^+$=852

**<Synthesis of Monomer 1-22>**

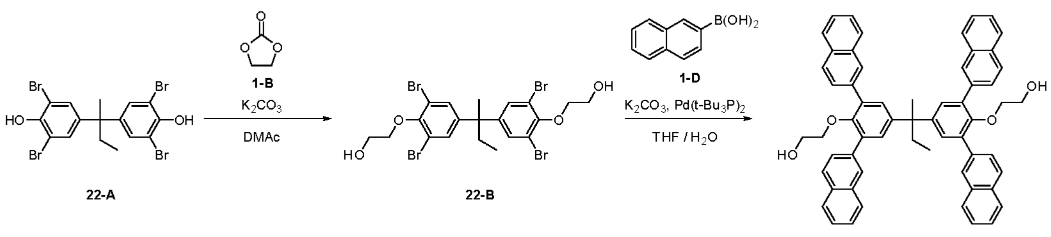


Monomer 1-22

Monomer 1-22 was obtained in the same manner as in the synthesis of Monomer 1-1, except that Compound 22-A was used instead of Compound 1-A.
MS: $[M+H]^+$=834

**<Synthesis of Monomer 1-23>**

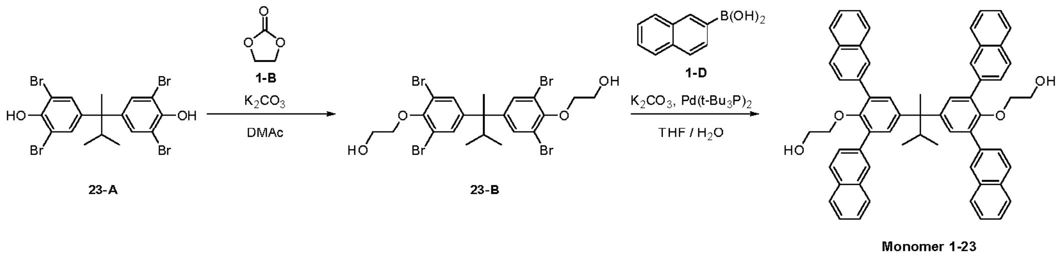


Monomer 1-23 was obtained in the same manner as in the synthesis of Monomer 1-1, except that Compound 23-A was used instead of Compound 1-A.
MS: $[M+H]^+$=848

**<Synthesis of Monomer 2-1>**

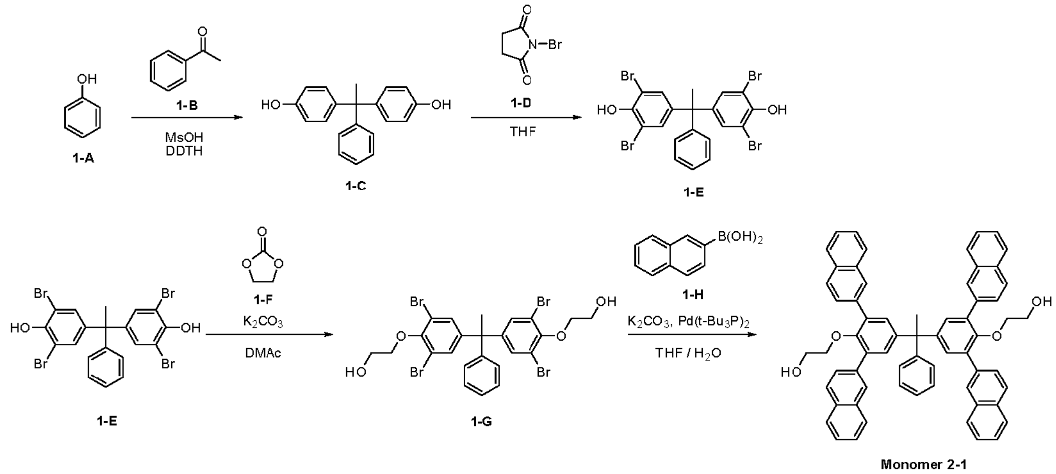


1) Synthesis of Compound 1-C

After 94.0 g (999 mmol, 6.0 eq) of Compound 1-A, 20.0 g (166 mmol, 1.0 eq) of Compound 1-B, 8.0 g (83 mmol, 0.50 eq) of methanesulfonic acid (MsOH), and 4.25 g (21 mmol, 0.126 eq) of dodecyl mercaptan (DDTH) were put into a flask, the resulting mixture was stirred in an oil bath at 35°C for 72 hours. After cooling, the mixture was concentrated under vacuum to remove Compound 1-A, then the residue was washed with dichloromethane (DCM)/$H_2O$ to separate the organic layer, and the solvent was concentrated under vacuum. The obtained solid was purified by column chromatography through ethyl acetate (EA) and dichloromethane (DCM), and then precipitated in n-hexane (n-Hex) to obtain 35 g of Compound 1-C as a white solid.

2) Synthesis of Compound 1-E

20.0 g (68.9 mmol, 1.0 eq) of Compound 1-C and 51.49 g (289 mmol, 4.2 eq) of Compound 1-D were dissolved in 200 g of tetrahydrofuran (THF), and the resulting solution was stirred at room temperature for 5 hours. Thereafter, the solution was concentrated under vacuum to completely remove the solvent, then the residue was washed with dichloromethane (DCM)/$H_2O$ to separate the organic layer, and the solvent was concentrated under vacuum. Thereafter,

the obtained solid was purified by column chromatography through ethyl acetate (EA) and dichloromethane (DCM), and then precipitated in n-hexane (n-Hex) to obtain 28 g of Compound 1-E as a white solid.

3) Synthesis of Compound 1-G

110.9 g (183 mmol, 1.0 eq) of Compound 1-E, 48.57 g (552 mmol, 3.0 eq) of Compound 1-F, and 20.33 g (147 mmol, 0.80 eq) of $K_2CO_3$ were dissolved in 400 g of dimethylacetamide (DMAc), and the resulting solution was stirred in an oil bath at 120°C for 2 hours. After cooling, a solid was precipitated by adding water thereto, and then filtered. The obtained solid was purified by column chromatography through ethyl acetate (EA) and dichloromethane (DCM), and then precipitated in n-hexane (n-Hex) to obtain 76 g of Compound 1-G as a white solid.

4) Synthesis of Monomer 2-1

54.8g (79 mmol, 1.0 eq) of Compound 1-G and 56.06 g (326 mmol, 4.12 eq) of Compound 1-H were dissolved in 510 g of tetrahydrofuran (THF), and the resulting solution was stirred in an oil bath at 80°C for 30 minutes. After 56.86 g (411 mmol, 5.20 eq) of $K_2CO_3$ was dissolved in 489 mL of water, the solution was added dropwise thereto for 10 minutes while maintaining the internal temperature of the solution at 50°C or higher. 0.20 g (0.4 mmol, 0.005 eq) of a $Pd(t\text{-}Bu_3P)_2$ catalyst was added thereto at an internal temperature of 60°C. After being stirred for 1 hour, the mixture was washed with ethyl acetate (EA)/$H_2O$ to separate the organic layer, and the solvent was concentrated under vacuum. Purification was performed by column chromatography through n-hexane (n-Hex) and dichloromethane (DCM), and then precipitated in n-hexane (n-Hex) to obtain 42g of Monomer 2-1 as a white solid.
MS: $[M+H]^+$=820

**<Synthesis of Monomer 2-2>**

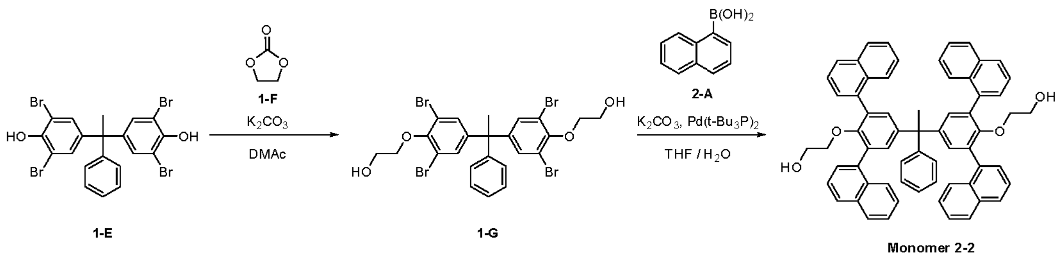


Monomer 2-2 was obtained in the same manner as in the synthesis of Monomer 2-1, except that Compound 2-A was used instead of Compound 1-H.
MS: $[M+H]^+$=882

**<Synthesis of Monomer 2-3>**

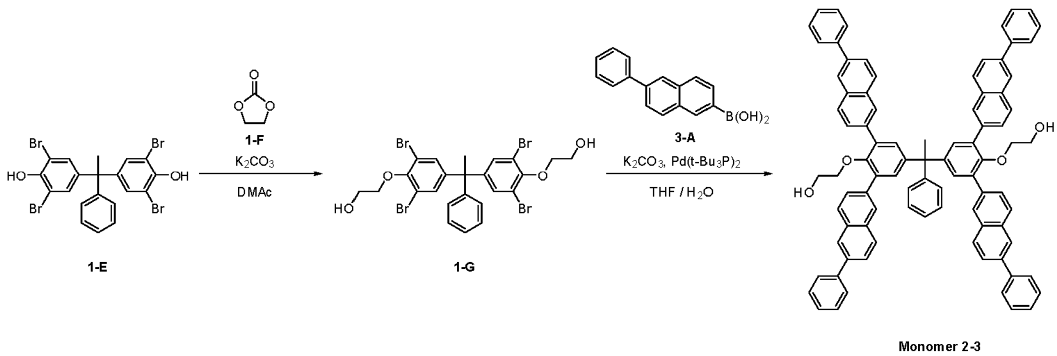


Monomer 2-3 was obtained in the same manner as in the synthesis of Monomer 2-1, except that Compound 3-A

was used instead of Compound 1-H.
MS: $[M+H]^+$=1186

**<Synthesis of Monomer 2-4>**

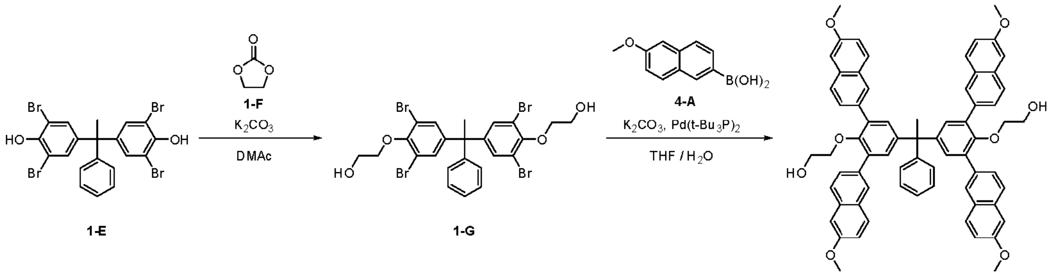


1-E 1-G

Monomer 2-4

Monomer 2-4 was obtained in the same manner as in the synthesis of Monomer 2-1, except that Compound 4-A was used instead of Compound 1-H.
MS: $[M+H]^+$=1002

**<Synthesis of Monomer 2-5>**

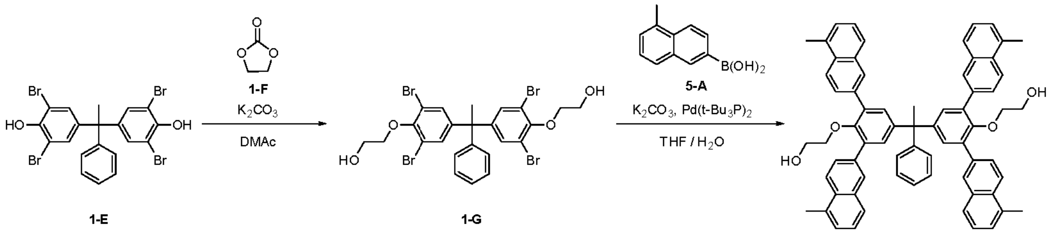


1-E 1-G

Monomer 2-5

Monomer 2-5 was obtained in the same manner as in the synthesis of Monomer 2-1, except that Compound 5-A was used instead of Compound 1-H.
MS: $[M+H]^+$=938

**<Synthesis of Monomer 2-6>**

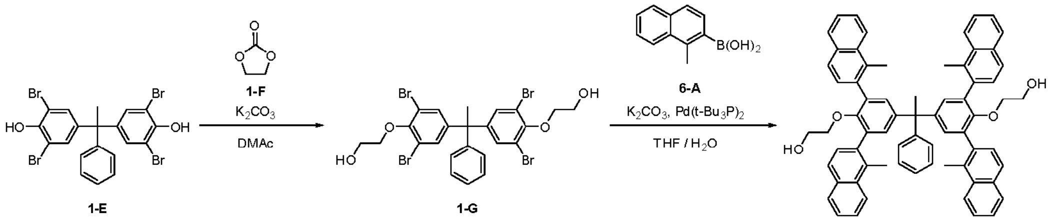


1-E 1-G

Monomer 2-6

Monomer 2-6 was obtained in the same manner as in the synthesis of Monomer 2-1, except that Compound 6-A was used instead of Compound 1-H.
MS: $[M+H]^+$=938

**<Synthesis of Monomer 2-7>**

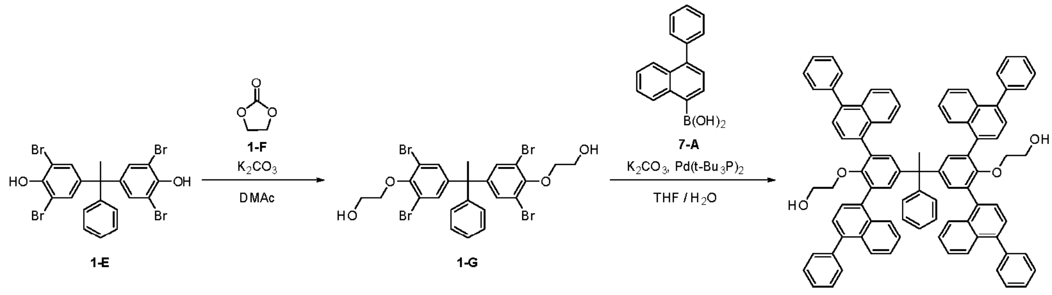


1-E 1-G Monomer 2-7

Monomer 2-7 was obtained in the same manner as in the synthesis of Monomer 2-1, except that Compound 7-A was used instead of Compound 1-H.
MS: $[M+H]^+$=1186

**<Synthesis of Monomer 2-8>**

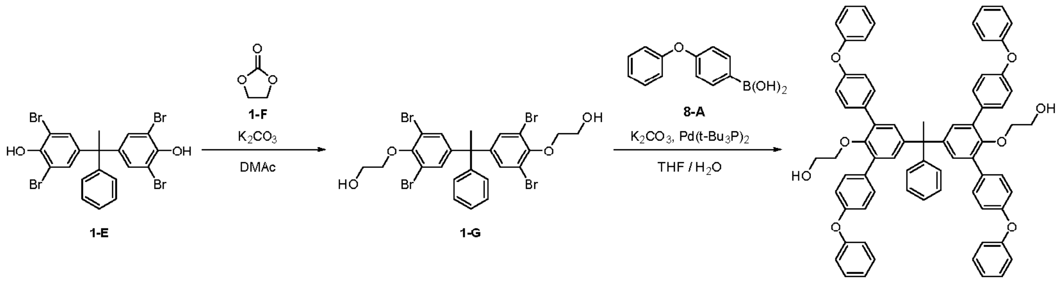


1-E 1-G Monomer 2-8

Monomer 2-8 was obtained in the same manner as in the synthesis of Monomer 2-1, except that Compound 8-A was used instead of Compound 1-H.
MS: $[M+H]^+$=1050

**<Synthesis of Monomer 2-9>**

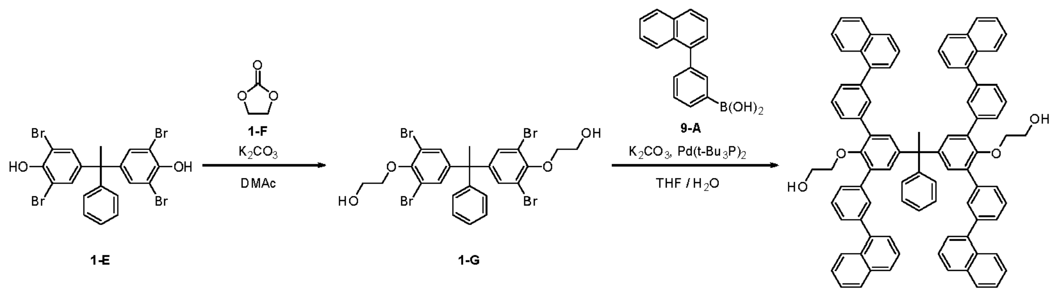


1-E 1-G Monomer 2-9

Monomer 2-9 was obtained in the same manner as in the synthesis of Monomer 2-1, except that Compound 9-A was used instead of Compound 1-H.
MS: $[M+H]^+$=1186

**<Synthesis of Monomer 2-10>**

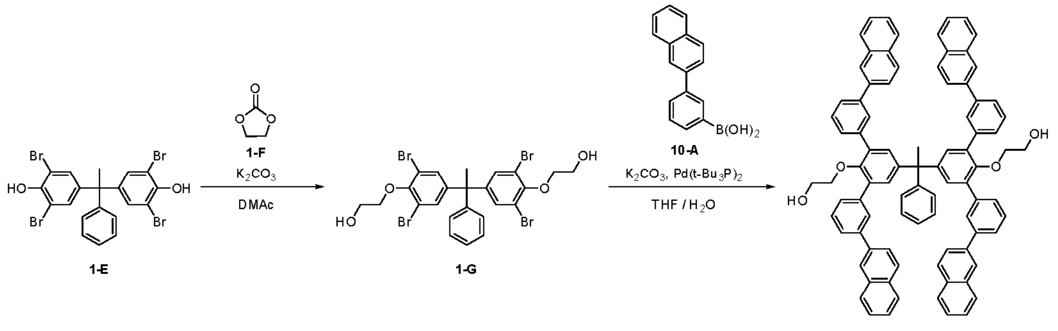


Monomer 2-10

Monomer 2-10 was obtained in the same manner as in the synthesis of Monomer 2-1, except that Compound 10-A was used instead of Compound 1-H.
MS: $[M+H]^+$=1186

**<Synthesis of Monomer 2-11>**

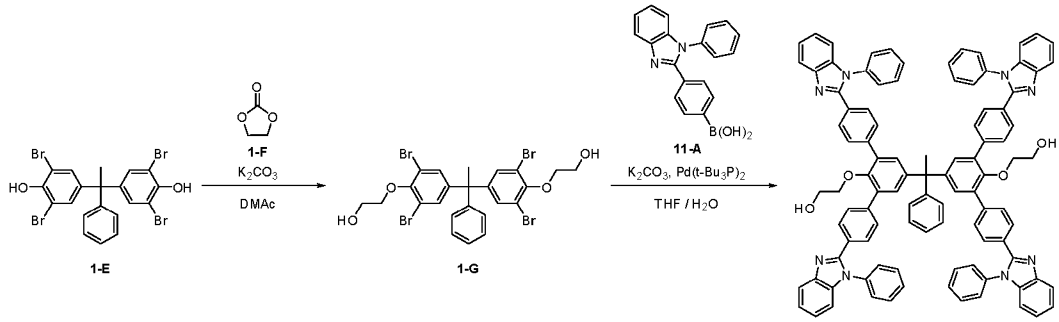


Monomer 2-11

Monomer 2-11 was obtained in the same manner as in the synthesis of Monomer 2-1, except that Compound 11-A was used instead of Compound 1-H.
MS: $[M+H]^+$=1450

**<Synthesis of Monomer 2-12>**

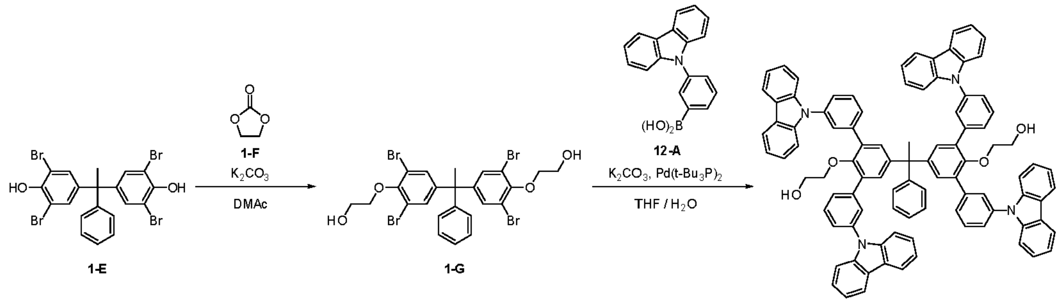


Monomer 2-12

Monomer 2-12 was obtained in the same manner as in the synthesis of Monomer 2-1, except that Compound 12-A was used instead of Compound 1-H.
MS: $[M+H]^+$=1342

**<Synthesis of Monomer 2-13>**

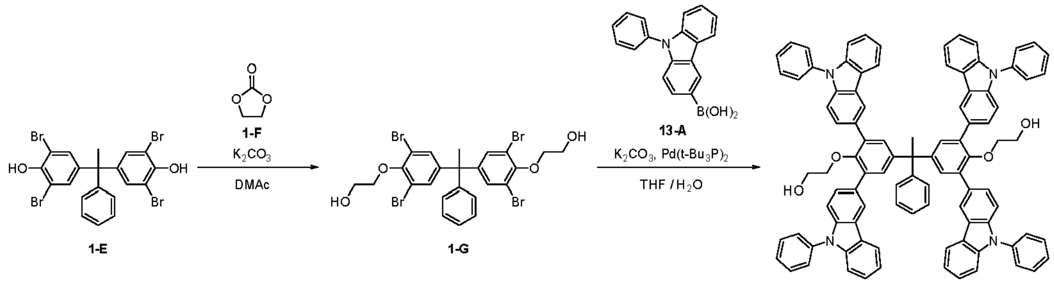

Monomer 2-13

Monomer 2-13 was obtained in the same manner as in the synthesis of Monomer 2-1, except that Compound 13-A was used instead of Compound 1-H.
MS: $[M+H]^+$=1342

**<Synthesis of Monomer 2-14>**

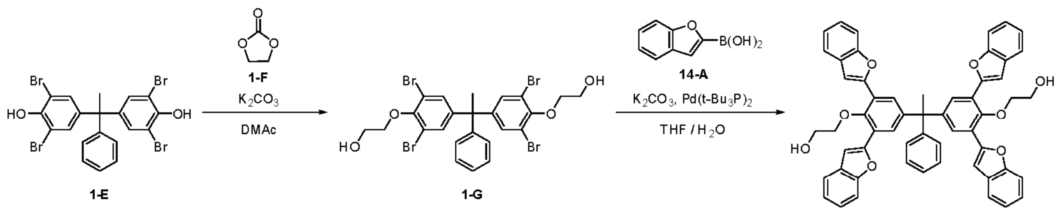

Monomer 2-14

Monomer 2-14 was obtained in the same manner as in the synthesis of Monomer 2-1, except that Compound 14-A was used instead of Compound 1-H.
MS: $[M+H]^+$=842

**<Synthesis of Monomer 2-15>**

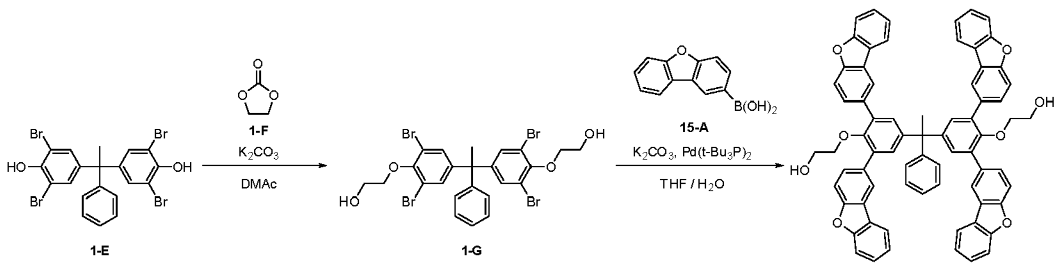

Monomer 2-15

Monomer 2-15 was obtained in the same manner as in the synthesis of Monomer 2-1, except that Compound 15-A was used instead of Compound 1-H.
MS: $[M+H]^+$=1042

**<Synthesis of Monomer 2-16>**

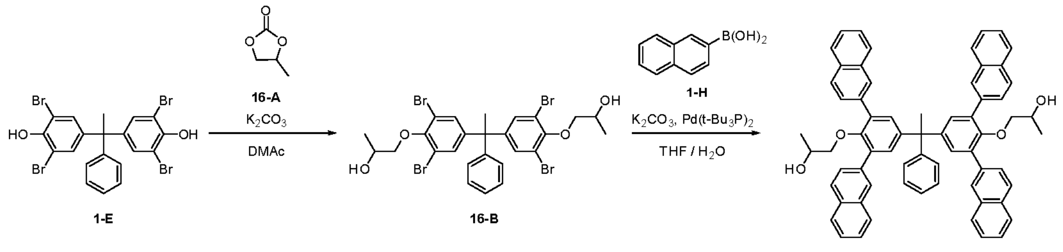


1-E 16-B Monomer 2-16

Monomer 2-16 was obtained in the same manner as in the synthesis of Monomer 2-1, except that Compound 16-A was used instead of Compound 1-F.
MS: $[M+H]^+$=910

**<Synthesis of Monomer 2-17>**

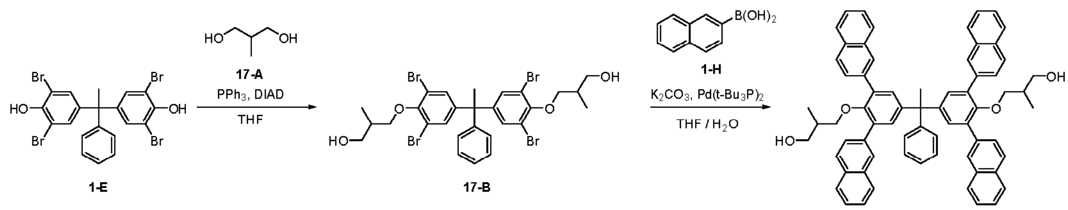


1-E 17-B Monomer 2-17

1) Synthesis of Compound 17-B

55.4 g (91.5 mmol, 1.0 eq) of Compound 1-E, 17.32 g (192 mmol, 2.1 eq) of Compound 17-A, and 50.36 g (192 mmol, 2.1 eq) of $PPh_3$ were dissolved in 2,000 g of tetrahydrofuran (THF), and 38.82 g (192 mmol, 2.1 eq) of diisopropyl azodicarboxylate was added thereto for 70 minutes. After the addition, the resulting mixture was stirred at room temperature for 12 hours. Thereafter, the mixture was purified by column chromatography through ethyl acetate (EA) and dichloromethane (DCM) after concentration, and then precipitated in n-hexane (n-Hex) to obtain 52 g of Compound 17-B as a white solid.

2) Synthesis of Monomer 2-17

Monomer 2-17 was obtained in the same manner as in the synthesis of Monomer 2-1.
MS: $[M+H]^+$=938

**<Synthesis of Monomer 2-18>**

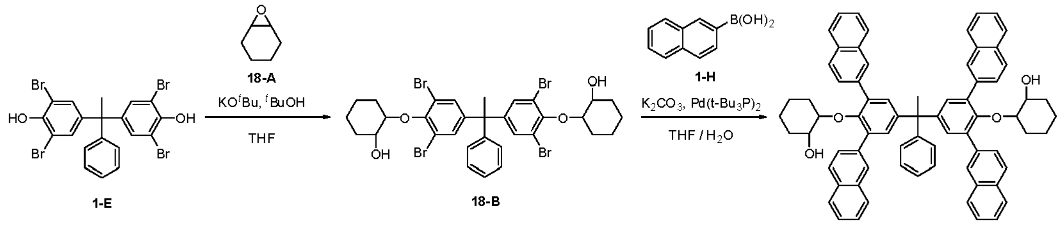


1-E 18-B Monomer 2-18

1) Synthesis of Compound 18-B

55.4 g (91.5 mmol, 1.0 eq) of Compound 1-E was dissolved in 500 g of tetrahydrofuran (THF) and 500 g of $^tBuOH$, and 21.56 g (192 mmol, 2.1 eq) of KOtBu was added thereto. After the addition, 18.84 g (192 mmol, 2.0 eq) of Compound 18-A was slowly added thereto. Thereafter, the temperature was raised and the mixture was stirred under reflux for 72 hours. After cooling, the mixture was purified by column chromatography through ethyl acetate (EA) and dichloromethane (DCM) after concentration, and then precipitated in n-hexane (n-Hex) to obtain 54g of Compound 18-B as a white solid.

2) Synthesis of Monomer 2-18

Monomer 2-18 was obtained in the same manner as in the synthesis of Monomer 2-1.
MS: $[M+H]^+$=990

**<Synthesis of Monomer 2-19>**

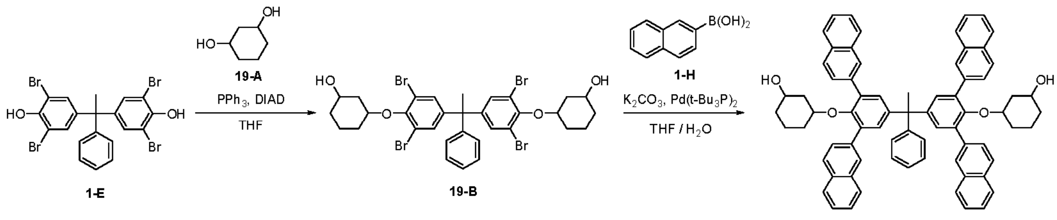


Monomer 2-19

Monomer 2-19 was obtained in the same manner as in the synthesis of Monomer 2-17, except that Compound 19-A was used instead of Compound 17-A.
MS: $[M+H]^+$=990

**<Synthesis of Monomer 2-20>**

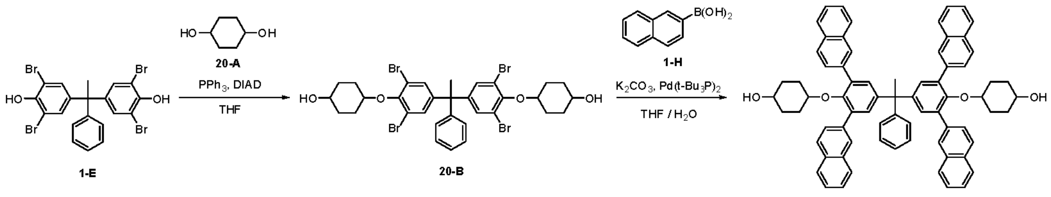


Monomer 2-20

Monomer 2-20 was obtained in the same manner as in the synthesis of Monomer 2-17, except that Compound 20-A was used instead of Compound 17-A.
MS: $[M+H]^+$=990

**<Synthesis of Monomer 2-21>**

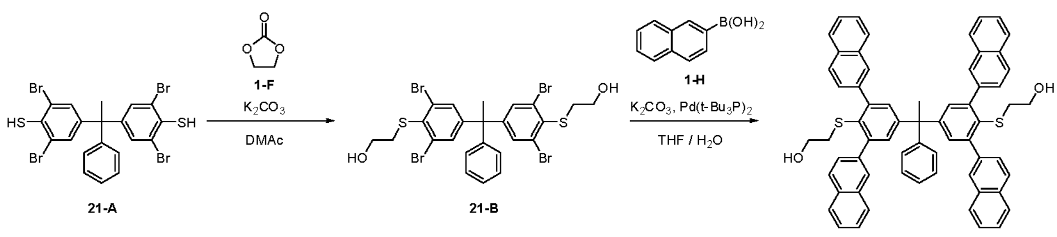


Monomer 2-21

Monomer 2-21 was obtained in the same manner as in the synthesis of Monomer 2-1, except that Compound 21-A was used instead of Compound 1-E.
MS: $[M+H]^+$=914

## <Synthesis of Monomer 2-22>

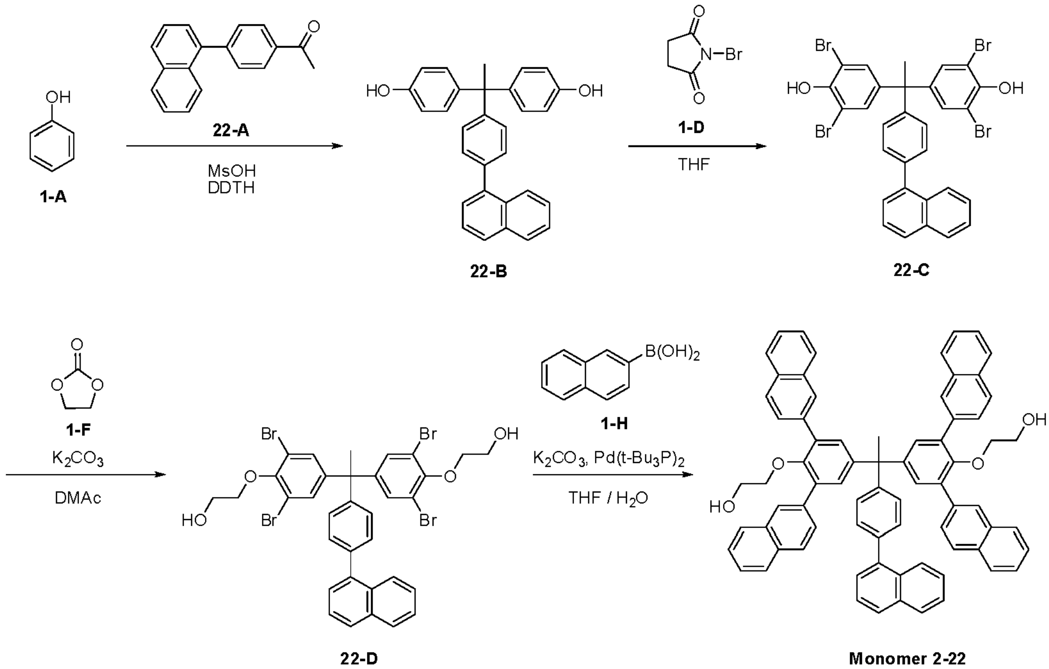


Monomer 2-22 was obtained in the same manner as in the synthesis of Monomer 2-1, except that Compound 22-A was used instead of Compound 1-B.
MS: $[M+H]^+$=1009

## <Synthesis of Monomer 2-23>

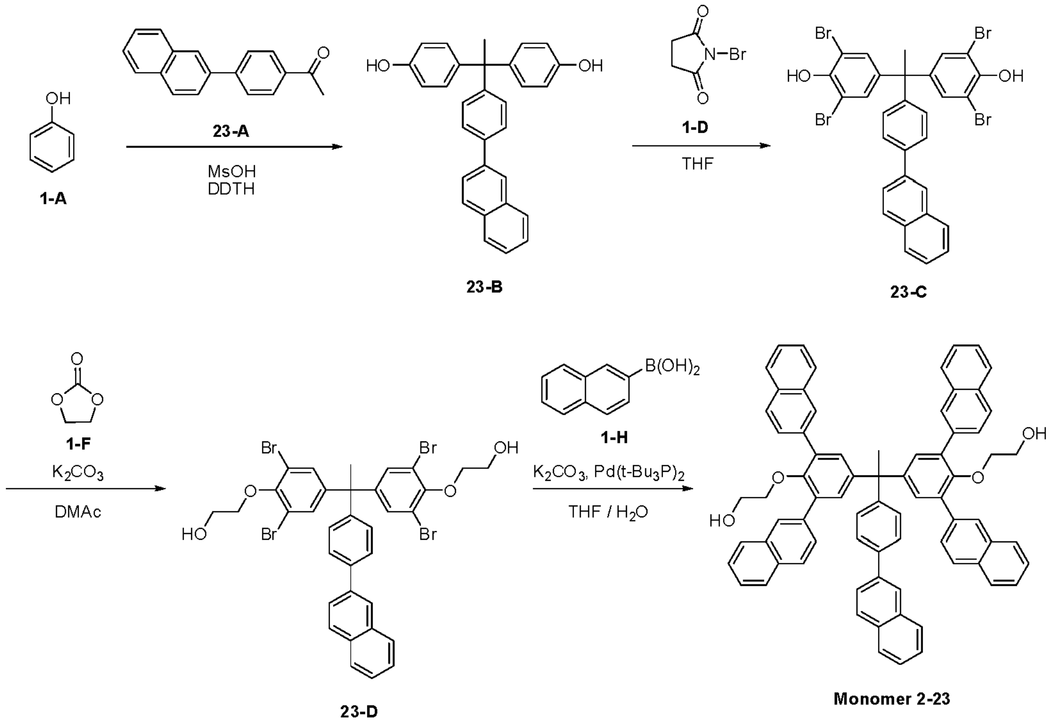


1-A 23-B 23-C 23-D Monomer 2-23

Monomer 2-23 was obtained in the same manner as in the synthesis of Monomer 2-1, except that Compound 23-A was used instead of Compound 1-B.
MS: $[M+H]^+$=1009

**<Synthesis of Monomer 2-24>**

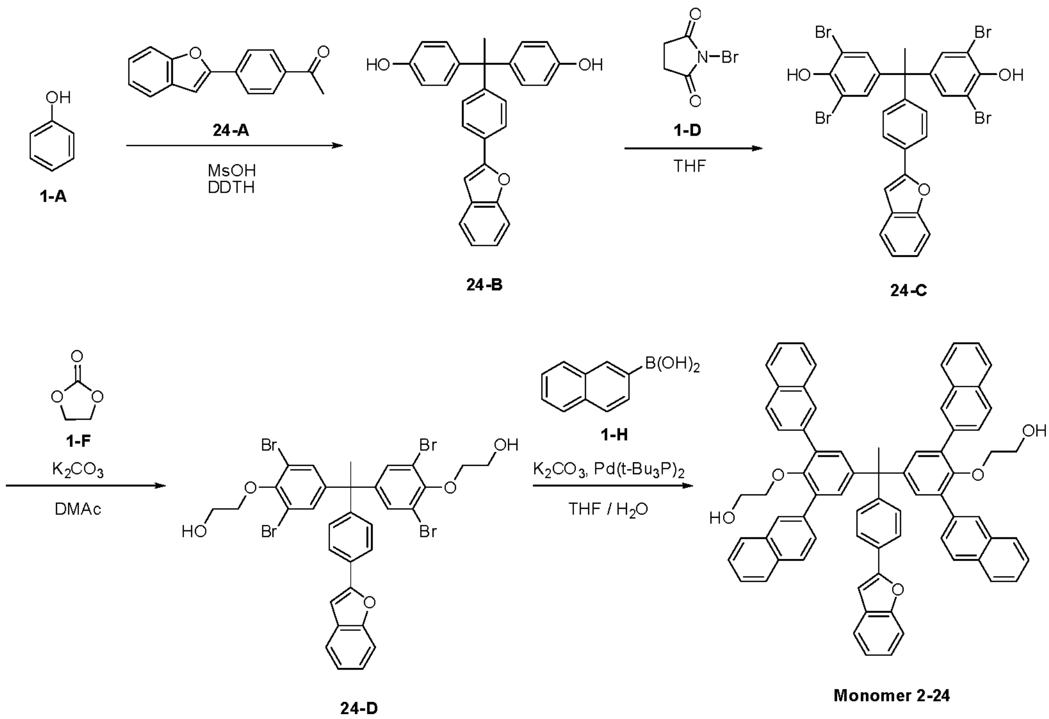


1-A 24-B 24-C 24-D Monomer 2-24

Monomer 2-24 was obtained in the same manner as in the synthesis of Monomer 2-1, except that Compound 24-A was used instead of Compound 1-B.

MS: $[M+H]^+=999$

**<Synthesis of Monomer 2-25>**

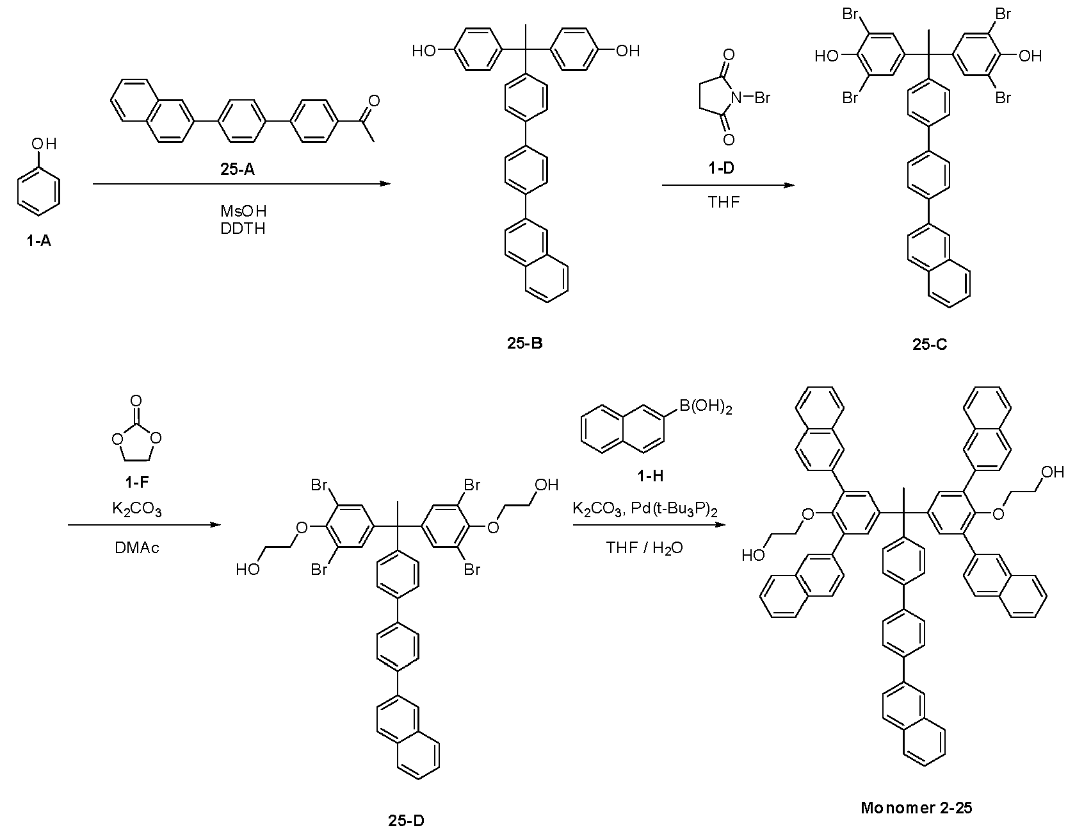


Monomer 2-25 was obtained in the same manner as in the synthesis of Monomer 2-1, except that Compound 25-A was used instead of Compound 1-B.
MS: $[M+H]^+=1085$

**<Synthesis of Monomer 3-1>**

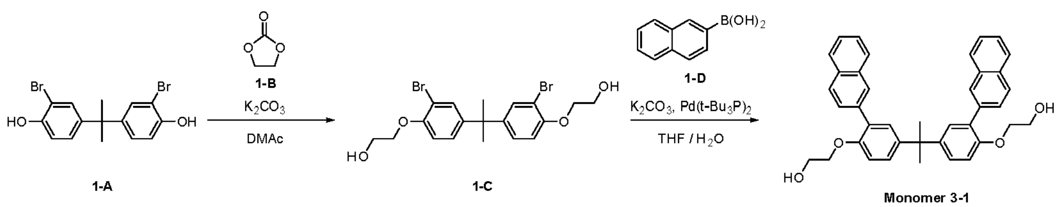


1) Synthesis of Compound 1-C

100.0 g (183 mmol, 1.0 eq) of Compound 1-A, 48.57 g (552 mmol, 3.0 eq) of Compound 1-B, and 20.33 g (147 mmol, 0.80 eq) of $K_2CO_3$ were dissolved in 400 g of dimethylacetamide (DMAc), and the resulting solution was stirred in an oil bath at 120°C for 2 hours. After cooling, a solid was precipitated by adding water thereto, and then filtered. The obtained solid was purified by column chromatography through ethyl acetate (EA) and dichloromethane (DCM), and then precipitated in n-hexane (n-Hex) to obtain 60 g of Compound 1-C as a white solid.

2) Synthesis of Monomer 3-1

50.0 g (79 mmol, 1.0 eq) of Compound 1-C and 56.06 g (326 mmol, 4.12 eq) of Compound 1-D were dissolved in 510 g of tetrahydrofuran (THF), and the resulting solution was stirred in an oil bath at 80°C for 30 minutes. After 56.86 g (411 mmol, 5.20 eq) of $K_2CO_3$ was dissolved in 489 mL of water, the solution was added dropwise thereto for 10 minutes while maintaining the internal temperature of the solution at 50°C or higher. 0.20 g (0.4 mmol, 0.005 eq) of a Pd(t-$Bu_3$P)$_2$ catalyst was added thereto at an internal temperature of 60°C. After being stirred for 1 hour, the mixture was washed with ethyl acetate (EA)/$H_2O$ to separate the organic layer, and the solvent was concentrated under vacuum. Purification was performed by column chromatography through n-hexane (n-Hex) and dichloromethane (DCM), and then precipitated in n-hexane (n-Hex) to obtain 58 g of Monomer 3-1 as a white solid.
MS: $[M+H]^+$=820

**<Synthesis of Monomer 3-2>**

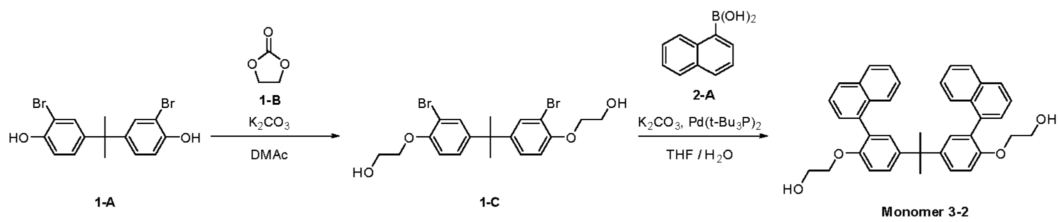


Monomer 3-2 was obtained in the same manner as in the synthesis of Monomer 3-1, except that Compound 2-A was used instead of Compound 1-D.
MS: $[M+H]^+$=568

**<Synthesis of Monomer 3-3>**

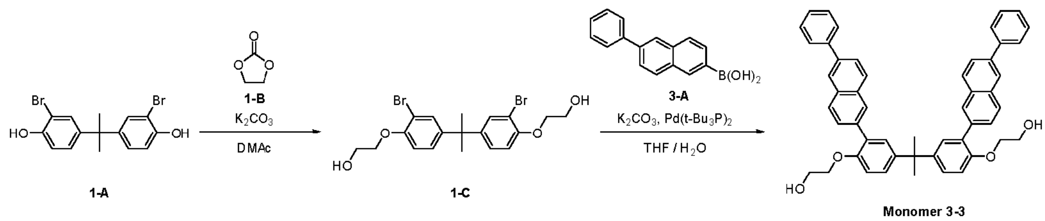


Monomer 3-3 was obtained in the same manner as in the synthesis of Monomer 3-1, except that Compound 3-A was used instead of Compound 1-D.
MS: $[M+H]^+$=720

**<Synthesis of Monomer 3-4>**

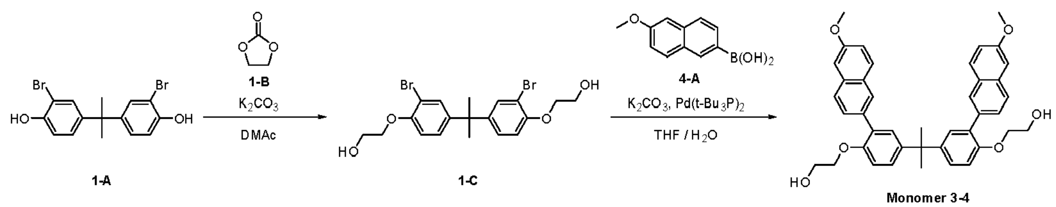


Monomer 3-4 was obtained in the same manner as in the synthesis of Monomer 3-1, except that Compound 4-A was used instead of Compound 1-D.

MS: [M+H]+=628

**<Synthesis of Monomer 3-5>**

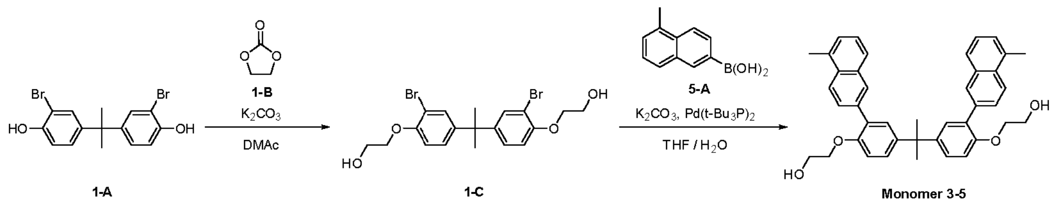


Monomer 3-5 was obtained in the same manner as in the synthesis of Monomer 3-1, except that Compound 5-A was used instead of Compound 1-D.
MS: [M+H]+=596

**<Synthesis of Monomer 3-6>**

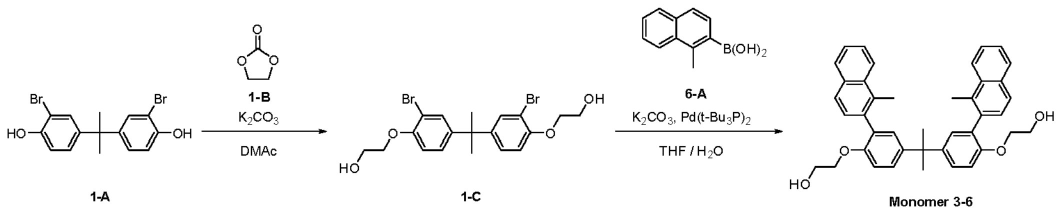


Monomer 3-6 was obtained in the same manner as in the synthesis of Monomer 3-1, except that Compound 6-A was used instead of Compound 1-D.
MS: [M+H]+=596

**<Synthesis of Monomer 3-7>**

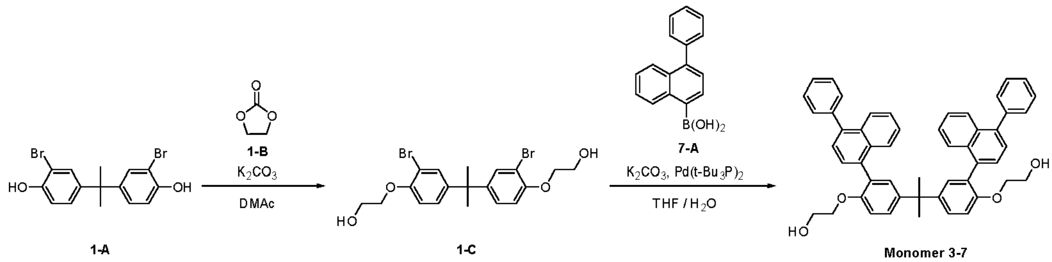


Monomer 3-7 was obtained in the same manner as in the synthesis of Monomer 3-1, except that Compound 7-A was used instead of Compound 1-D.
MS: [M+H]+=720

**<Synthesis of Monomer 3-8>**

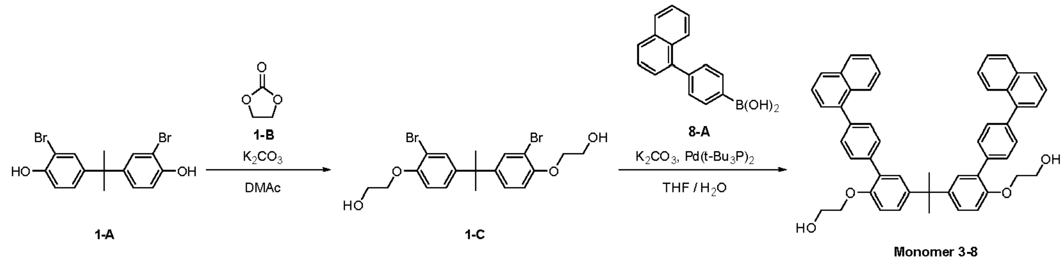


Monomer 3-8 was obtained in the same manner as in the synthesis of Monomer 3-1, except that Compound 8-A was used instead of Compound 1-D.
MS: $[M+H]^+$=652

**<Synthesis of Monomer 3-9>**

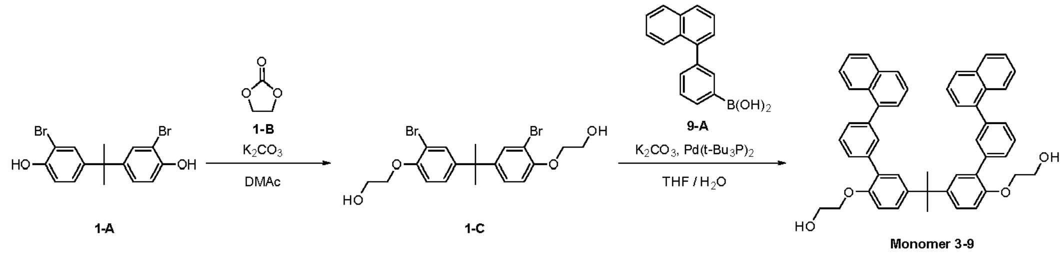


Monomer 3-9 was obtained in the same manner as in the synthesis of Monomer 3-1, except that Compound 9-A was used instead of Compound 1-D.
MS: $[M+H]^+$=720

**<Synthesis of Monomer 3-10>**

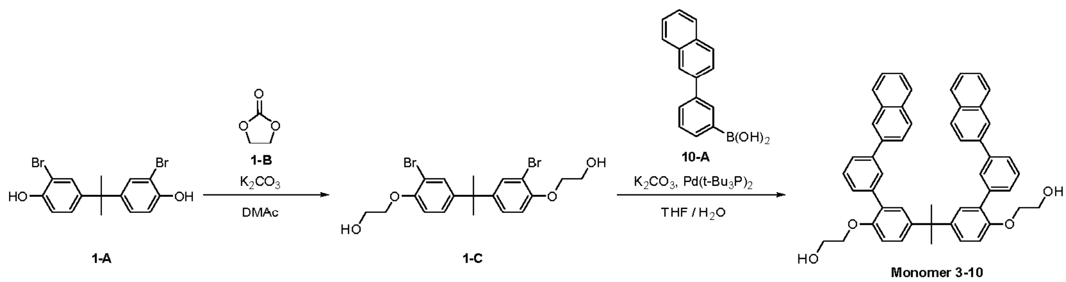


Monomer 3-10 was obtained in the same manner as in the synthesis of Monomer 3-1, except that Compound 10-A was used instead of Compound 1-D.
MS: $[M+H]^+$=720

**<Synthesis of Monomer 3-11>**

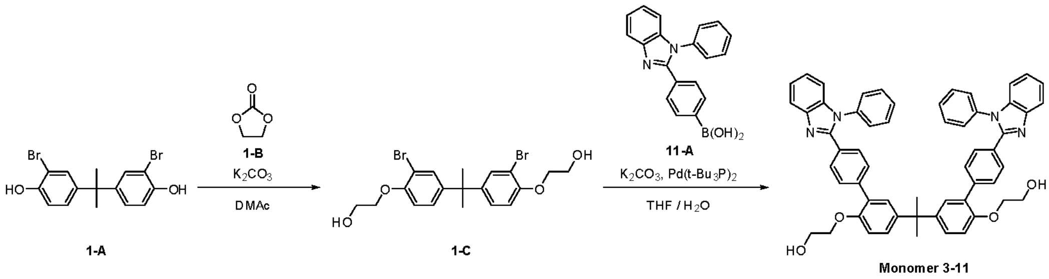


Monomer 3-11 was obtained in the same manner as in the synthesis of Monomer 3-1, except that Compound 11-A was used instead of Compound 1-D.
MS: $[M+H]^+$=852

**<Synthesis of Monomer 3-12>**

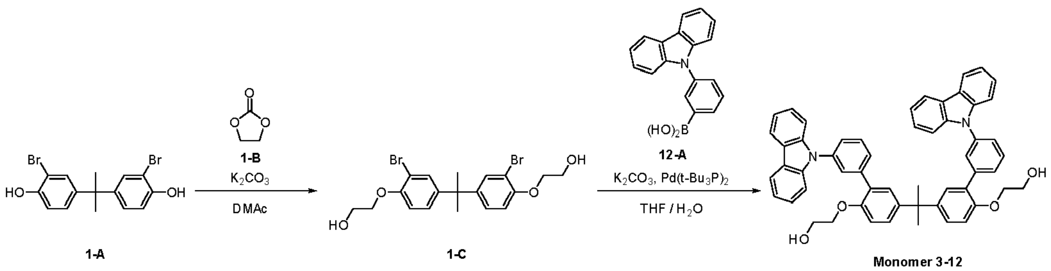


Monomer 3-12 was obtained in the same manner as in the synthesis of Monomer 3-1, except that Compound 12-A was used instead of Compound 1-D.
MS: $[M+H]^+$=798

**<Synthesis of Monomer 3-13>**

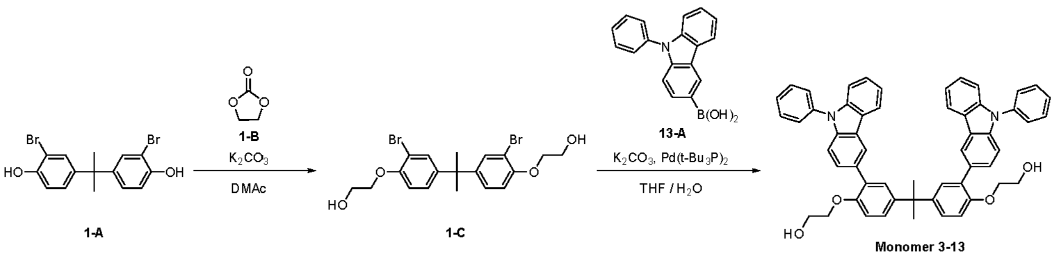


Monomer 3-13 was obtained in the same manner as in the synthesis of Monomer 3-1, except that Compound 13-A was used instead of Compound 1-D.
MS: $[M+H]^+$=798

**<Synthesis of Monomer 3-14>**

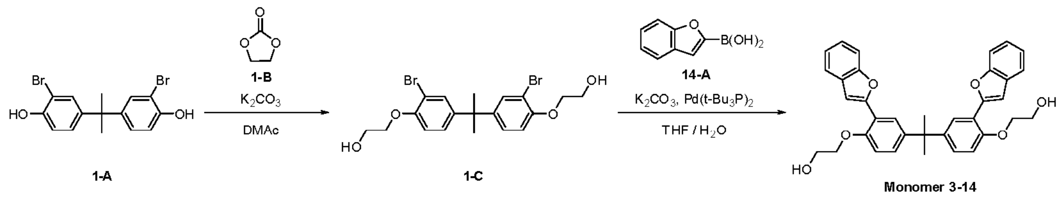


Monomer 3-14 was obtained in the same manner as in the synthesis of Monomer 3-1, except that Compound 14-A was used instead of Compound 1-D.
MS: $[M+H]^+$=548

**<Synthesis of Monomer 3-15>**

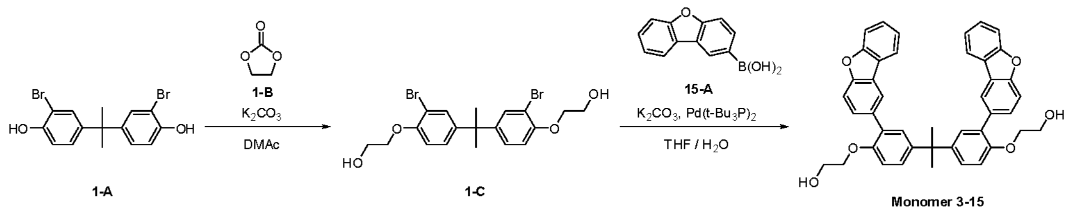


Monomer 3-15 was obtained in the same manner as in the synthesis of Monomer 3-1, except that Compound 15-A was used instead of Compound 1-D.
MS: $[M+H]^+$=648

**<Synthesis of Monomer 3-16>**

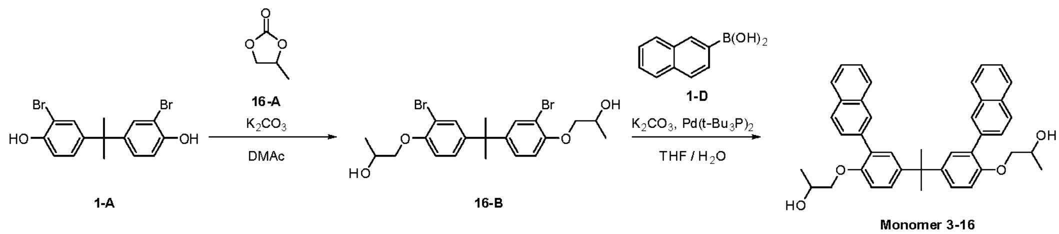


Monomer 3-16 was obtained in the same manner as in the synthesis of Monomer 3-1, except that Compound 16-A was used instead of Compound 1-B.
MS: $[M+H]^+$=848

**<Synthesis of Monomer 3-17>**

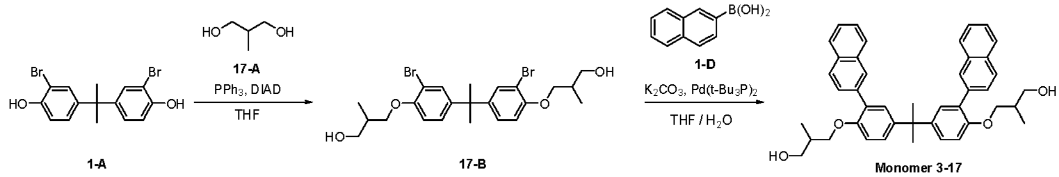


1) Synthesis of Compound 17-B

35.3g (91.5 mmol, 1.0 eq) of Compound 1-A, 17.32 g (192 mmol, 2.1 eq) of Compound 17-A, and 50.36 g (192

mmol, 2.1 eq) of $PPh_3$ were dissolved in 2,000 g of tetrahydrofuran (THF), and 38.82 g (192 mmol, 2.1 eq) of diisopropyl azodicarboxylate was added thereto for 70 minutes. After the addition, the resulting mixture was stirred at room temperature for 12 hours. Thereafter, the mixture was purified by column chromatography through ethyl acetate (EA) and dichloromethane (DCM) after concentration, and then precipitated in n-hexane (n-Hex) to obtain 29g of Compound 17-B as a white solid.

2) Synthesis of Monomer 3-17

Monomer 3-17 was obtained in the same manner as in the synthesis of Monomer 3-1.
MS: $[M+H]^+$=624

**<Synthesis of Monomer 3-18>**

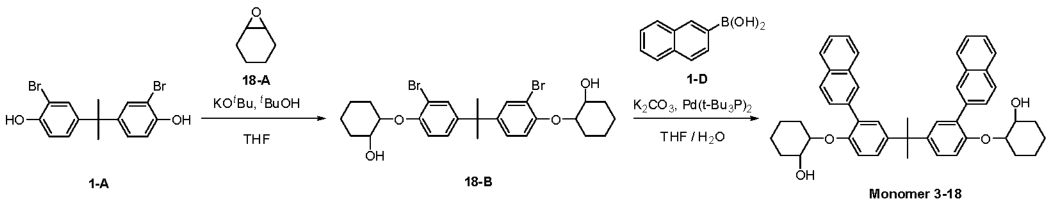


1-A 18-B Monomer 3-18

1) Synthesis of Compound 18-B

35.3g (91.5 mmol, 1.0 eq) of Compound 1-A was dissolved in 500 g of tetrahydrofuran (THF) and 500 g of $^t$BuOH, and 21.56 g (192 mmol, 2.1 eq) of KOtBu was added thereto. After the addition, 18.84 g (192 mmol, 2.0 eq) of Compound 18-A was slowly added thereto. Thereafter, the temperature was raised and the mixture was stirred under reflux for 72 hours. After cooling, the mixture was purified by column chromatography through ethyl acetate (EA) and dichloromethane (DCM) after concentration, and then precipitated in n-hexane (n-Hex) to obtain 32g of Compound 18-B as a white solid.

2) Synthesis of Monomer 3-18

Monomer 3-18 was obtained in the same manner as in the synthesis of Monomer 3-1.
MS: $[M+H]^+$=676

**<Synthesis of Monomer 3-19>**

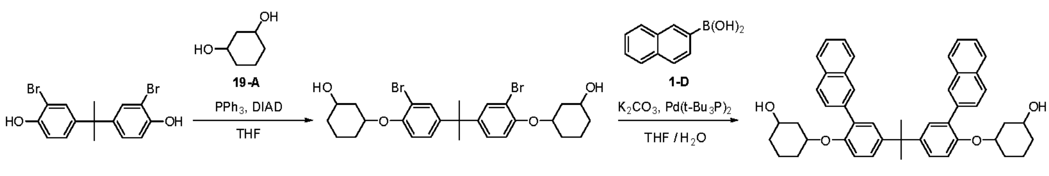


1-A 19-B Monomer 3-19

Monomer 3-19 was obtained in the same manner as in the synthesis of Monomer 3-17, except that Compound 19-A was used instead of Compound 17-A.
MS: $[M+H]^+$=676

**<Synthesis of Monomer 3-20>**

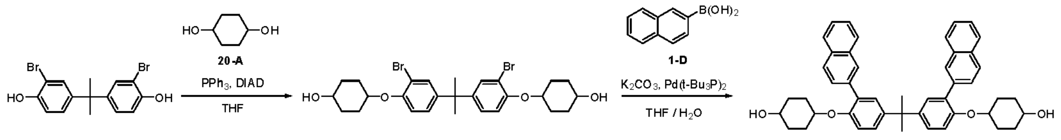


1-A 20-B Monomer 3-20

Monomer 3-20 was obtained in the same manner as in the synthesis of Monomer 3-17, except that Compound 19-A was used instead of Compound 17-A.
MS: $[M+H]^+$=676

**<Synthesis of Monomer 3-21>**

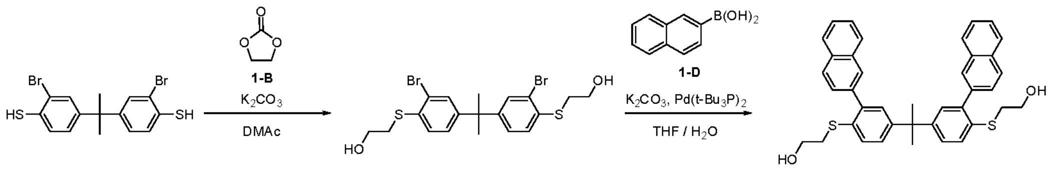


21-A 21-B Monomer 3-21

Monomer 3-21 was obtained in the same manner as in the synthesis of Monomer 3-1, except that Compound 21-A was used instead of Compound 1-A.
MS: $[M+H]^+$=600

**<Synthesis of Monomer 3-22>**

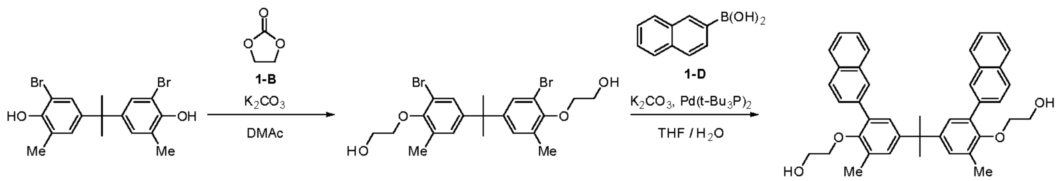


22-A 22-B Monomer 3-22

Monomer 3-22 was obtained in the same manner as in the synthesis of Monomer 3-1, except that Compound 22-A was used instead of Compound 1-A.
MS: $[M+H]^+$=596

**<Synthesis of Monomer 3-23>**

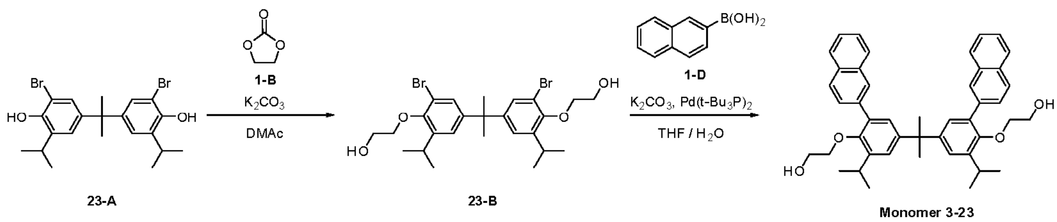


23-A 23-B Monomer 3-23

Monomer 3-23 was obtained in the same manner as in the synthesis of Monomer 3-1, except that Compound 23-A was used instead of Compound 1-A.
MS: $[M+H]^+$=652

**<Synthesis of Monomer 3-24>**

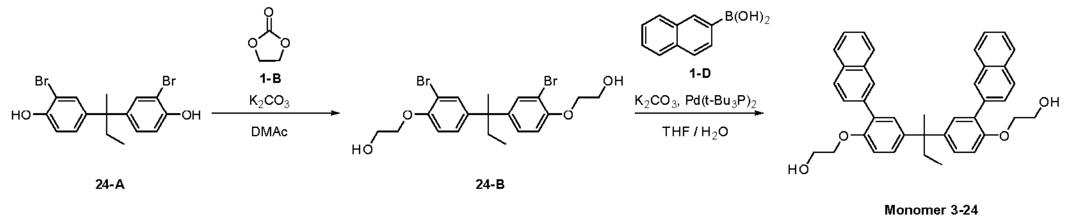


Monomer 3-24 was obtained in the same manner as in the synthesis of Monomer 3-1, except that Compound 24-A was used instead of Compound 1-A.
MS: $[M+H]^+$=582

**<Synthesis of Monomer 3-25>**

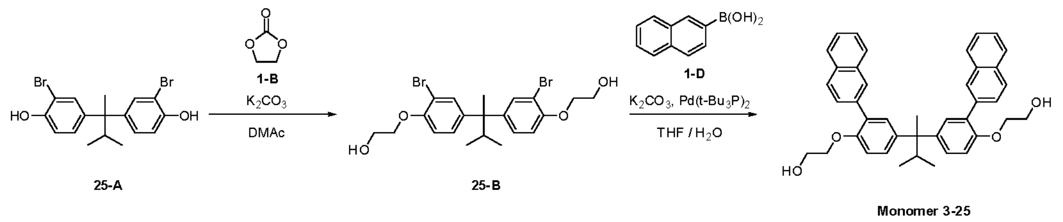


Monomer 3-25 was obtained in the same manner as in the synthesis of Monomer 3-1, except that Compound 25-A was used instead of Compound 1-A.
MS: $[M+H]^+$=596

**<Synthesis of Monomer 4-1>**

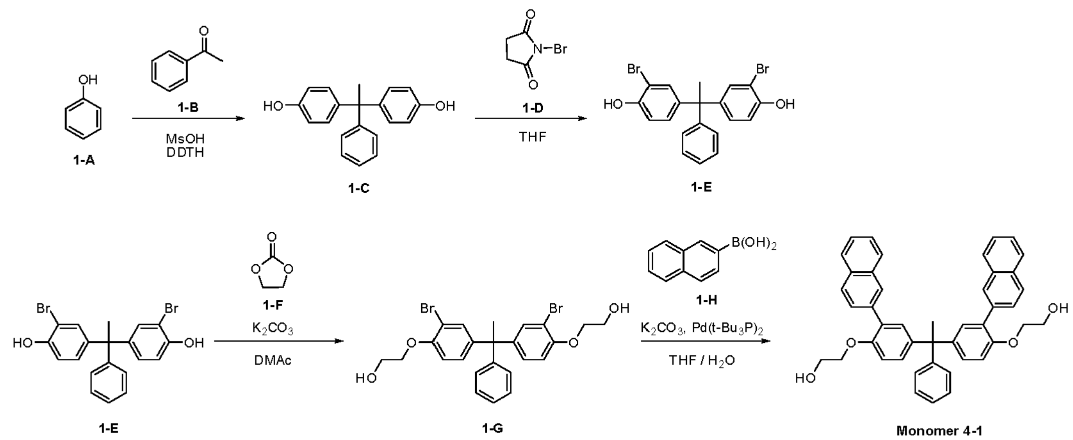


1) Synthesis of Compound 1-C

After 94.0 g (999 mmol, 6.0 eq) of Compound 1-A, 20.0 g (166 mmol, 1.0 eq) of Compound 1-B, 8.0 g (83 mmol, 0.50 eq) of methanesulfonic acid (MsOH), and 4.25 g (21 mmol, 0.126 eq) of dodecyl mercaptan (DDTH) were put into a flask, the resulting mixture was stirred in an oil bath at 35°C for 72 hours. After cooling, the mixture was concentrated under vacuum to remove Compound 1-A, and then the obtained solid was purified by column chromatography through ethyl acetate (EA) and dichloromethane (DCM), and then precipitated in n-hexane (n-Hex) to obtain 35 g of Compound 1-C as a

white solid.

2) Synthesis of Compound 1-E

20.0 g (68.9 mmol, 1.0 eq) of Compound 1-C and 25.74g (144.5mmol, 2.1 eq) of Compound 1-D were dissolved in 200 g of tetrahydrofuran (THF), and the resulting solution was stirred at room temperature for 5 hours. Thereafter, the solution was concentrated under vacuum to completely remove the solvent, and then the obtained solid was purified by column chromatography through ethyl acetate (EA) and dichloromethane (DCM), and then precipitated in n-hexane (n-Hex) to obtain 19 g of Compound 1-E as a white solid.

3) Synthesis of Compound 1-G

82.0g (183 mmol, 1.0 eq) of Compound 1-E, 48.57 g (552 mmol, 3.0 eq) of Compound 1-F, and 20.33 g (147 mmol, 0.80 eq) of $K_2CO_3$ were dissolved in 400 g of dimethylacetamide (DMAc), and the resulting solution was stirred in an oil bath at 120°C for 2 hours. After cooling, a solid was precipitated by adding water thereto, and then filtered. The obtained solid was purified by column chromatography through ethyl acetate (EA) and dichloromethane (DCM), and then precipitated in n-hexane (n-Hex) to obtain 59g of Compound 1-G as a white solid.

4) Synthesis of Monomer 4-1

42.36 g (79 mmol, 1.0 eq) of Compound 1-G and 28.03 g (163 mmol, 2.06 eq) of Compound 1-H were dissolved in 205 g of tetrahydrofuran (THF), and the resulting solution was stirred in an oil bath at 80°C for 30 minutes. After 28.44 g (206 mmol, 2.60 eq) of $K_2CO_3$ was dissolved in 245 mL of water, the solution was added dropwise thereto for 10 minutes while maintaining the internal temperature of the solution at 50°C or higher. 0.20 g (0.4 mmol, 0.005 eq) of a $Pd(t\text{-}Bu_3P)_2$ catalyst was added thereto at an internal temperature of 60°C. After being stirred for 1 hour, the mixture was washed with ethyl acetate (EA)/$H_2O$ to separate the organic layer, and the solvent was concentrated under vacuum. Purification was performed by column chromatography through n-hexane (n-Hex) and dichloromethane (DCM), and then precipitated in n-hexane (n-Hex) to obtain 30g of Monomer 4-1 as a white solid.
MS: $[M+H]^+$=630

**<Synthesis of Monomer 4-2>**

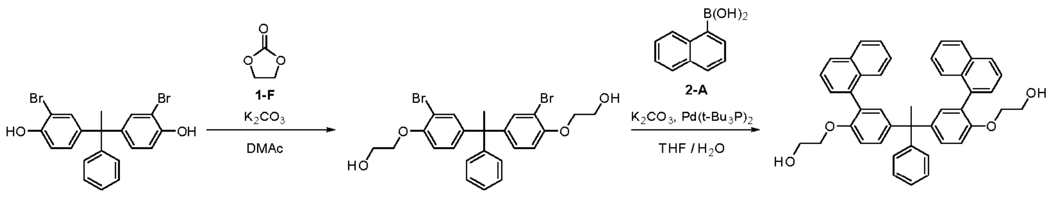


1-E 1-G Monomer 4-2

Monomer 4-2 was obtained in the same manner as in the synthesis of Monomer 4-1, except that Compound 2-A was used instead of Compound 1-H.
MS: $[M+H]^+$=630

**<Synthesis of Monomer 4-3>**

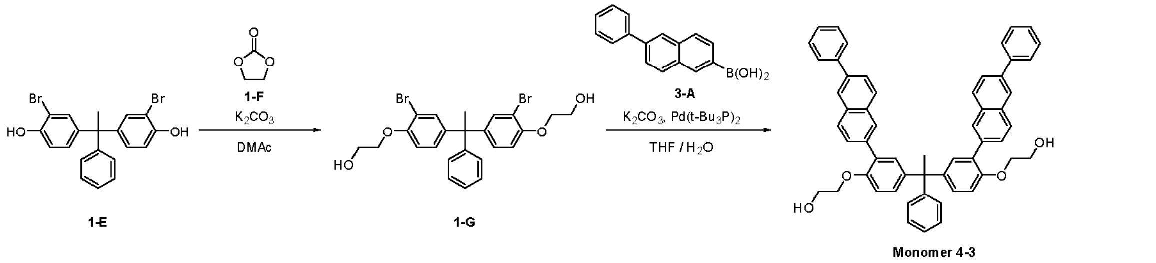


1-E 1-G Monomer 4-3

Monomer 4-3 was obtained in the same manner as in the synthesis of Monomer 4-1, except that Compound 3-A was used instead of Compound 1-H.
MS: $[M+H]^+$=782

### <Synthesis of Monomer 4-4>

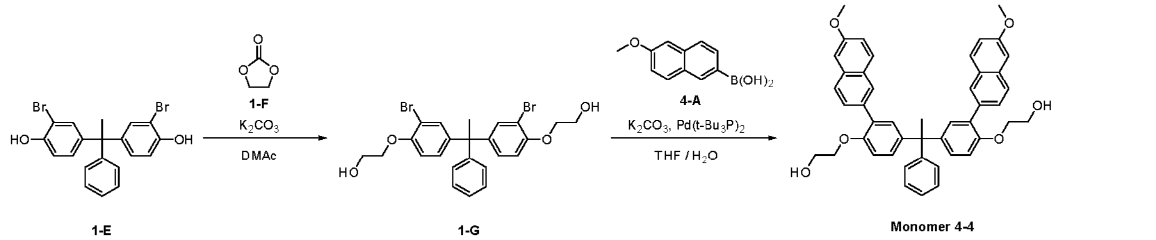


1-E 1-G Monomer 4-4

Monomer 4-4 was obtained in the same manner as in the synthesis of Monomer 4-1, except that Compound 4-A was used instead of Compound 1-H.
MS: $[M+H]^+$=690

### <Synthesis of Monomer 4-5>

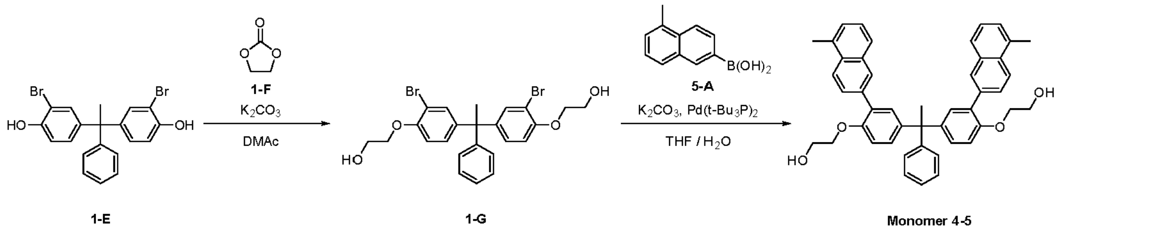


1-E 1-G Monomer 4-5

Monomer 4-5 was obtained in the same manner as in the synthesis of Monomer 4-1, except that Compound 5-A was used instead of Compound 1-H.
MS: $[M+H]^+$=658

### <Synthesis of Monomer 4-6>

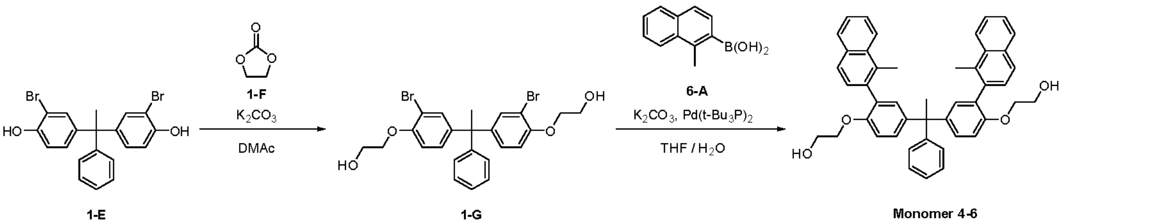


1-E 1-G Monomer 4-6

Monomer 4-6 was obtained in the same manner as in the synthesis of Monomer 4-1, except that Compound 6-A was used instead of Compound 1-H.
MS: $[M+H]^+$=658

**<Synthesis of Monomer 4-7>**

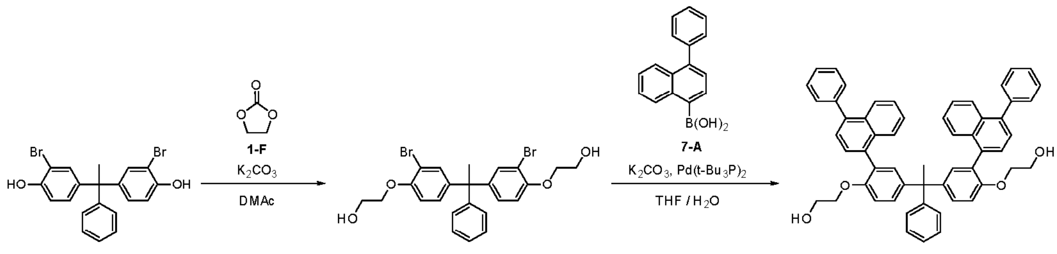


1-E 1-G Monomer 4-7

Monomer 4-7 was obtained in the same manner as in the synthesis of Monomer 4-1, except that Compound 7-A was used instead of Compound 1-H.
MS: $[M+H]^+$=782

**<Synthesis of Monomer 4-8>**

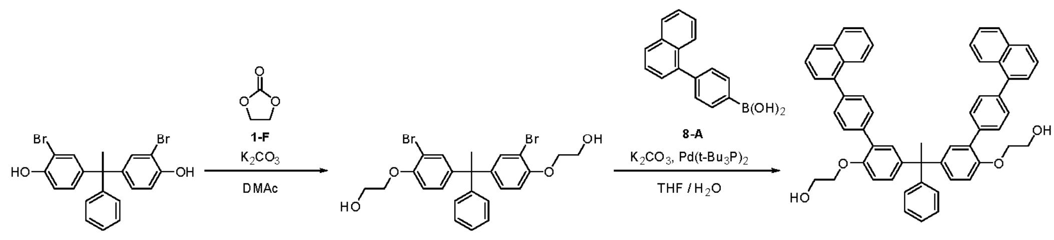


1-E 1-G Monomer 4-8

Monomer 4-8 was obtained in the same manner as in the synthesis of Monomer 4-1, except that Compound 8-A was used instead of Compound 1-H.
MS: $[M+H]^+$=714

**<Synthesis of Monomer 4-9>**

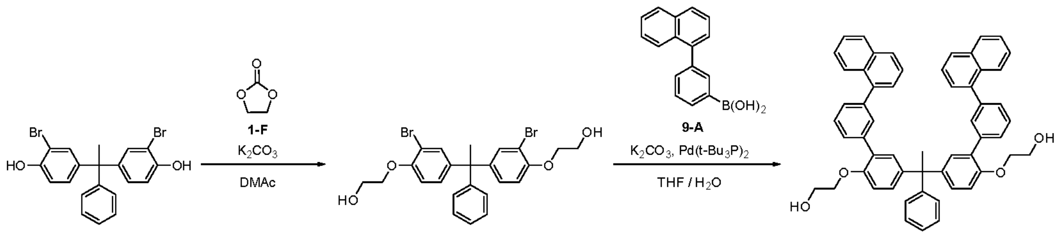


1-E 1-G Monomer 4-9

Monomer 4-9 was obtained in the same manner as in the synthesis of Monomer 4-1, except that Compound 9-A was used instead of Compound 1-H.
MS: $[M+H]^+$=782

**<Synthesis of Monomer 4-10>**

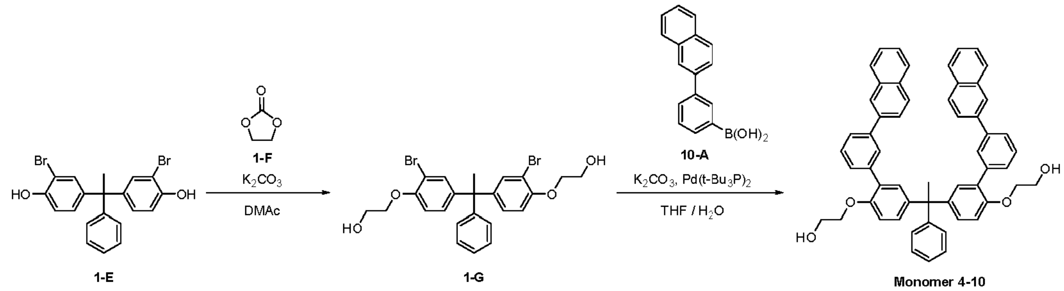


Monomer 4-10 was obtained in the same manner as in the synthesis of Monomer 4-1, except that Compound 10-A was used instead of Compound 1-H.
MS: $[M+H]^+$=782

**<Synthesis of Monomer 4-11>**

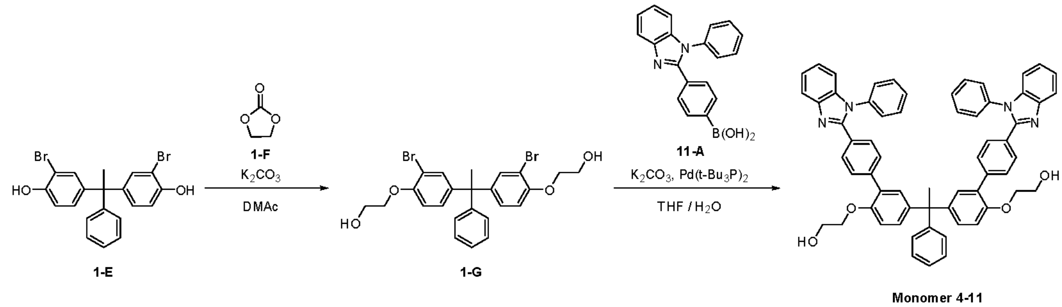


Monomer 4-11 was obtained in the same manner as in the synthesis of Monomer 4-1, except that Compound 11-A was used instead of Compound 1-H.
MS: $[M+H]^+$=914

**<Synthesis of Monomer 4-12>**

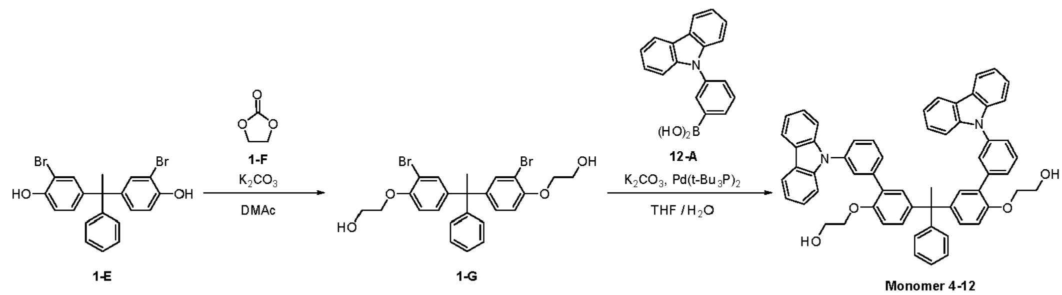


Monomer 4-12 was obtained in the same manner as in the synthesis of Monomer 4-1, except that Compound 12-A was used instead of Compound 1-H.
MS: $[M+H]^+$=860

**<Synthesis of Monomer 4-13>**

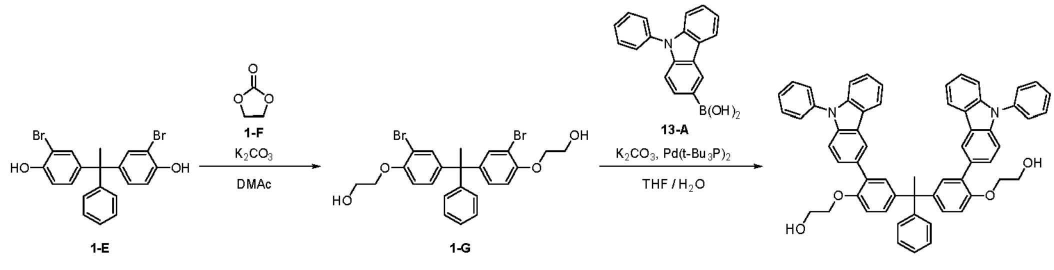


1-E 1-G Monomer 4-13

Monomer 4-13 was obtained in the same manner as in the synthesis of Monomer 4-1, except that Compound 13-A was used instead of Compound 1-H.
MS: $[M+H]^+$=860

**<Synthesis of Monomer 4-14>**

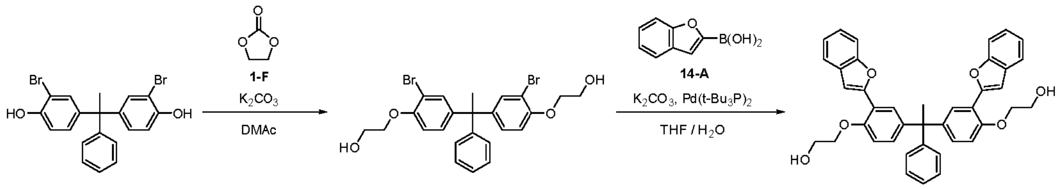


1-E 1-G Monomer 4-14

Monomer 4-14 was obtained in the same manner as in the synthesis of Monomer 4-1, except that Compound 14-A was used instead of Compound 1-H.
MS: $[M+H]^+$=610

**<Synthesis of Monomer 4-15>**

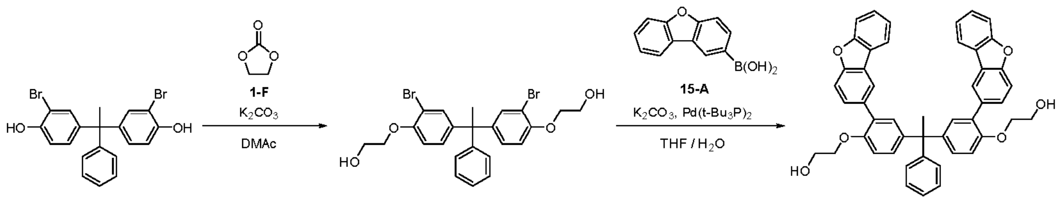


1-E 1-G Monomer 4-15

Monomer 4-15 was obtained in the same manner as in the synthesis of Monomer 4-1, except that Compound 15-A was used instead of Compound 1-H.
MS: $[M+H]^+$=710

**<Synthesis of Monomer 4-16>**

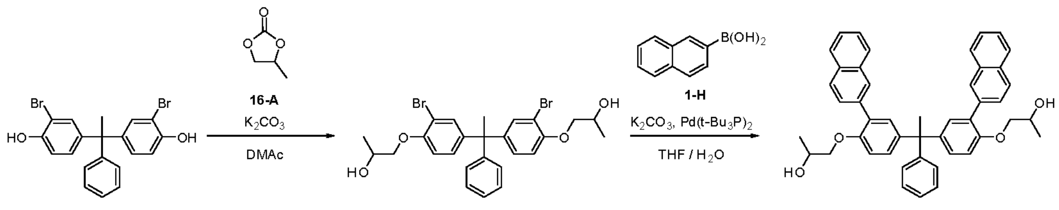


1-E 16-B Monomer 4-16

Monomer 4-16 was obtained in the same manner as in the synthesis of Monomer 4-1, except that Compound 16-A was used instead of Compound 1-F.
MS: $[M+H]^+$=658

**<Synthesis of Monomer 4-17>**

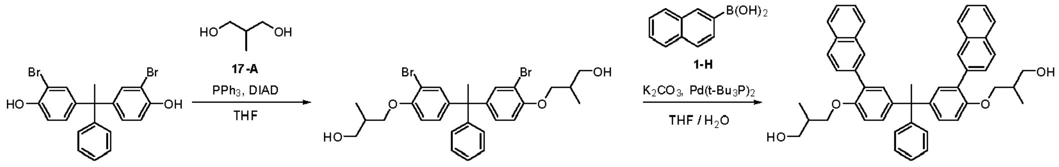


1-E 17-B Monomer 4-17

1) Synthesis of Compound 17-B

41.0g (91.5 mmol, 1.0 eq) of Compound 1-E, 17.32 g (192 mmol, 2.1 eq) of Compound 17-A, and 50.36 g (192 mmol, 2.1 eq) of $PPh_3$ were dissolved in 2,000 g of tetrahydrofuran (THF), and 38.82 g (192 mmol, 2.1 eq) of diisopropyl azodicarboxylate was added thereto for 70 minutes. After the addition, the resulting mixture was stirred at room temperature for 12 hours. Thereafter, the mixture was purified by column chromatography through ethyl acetate (EA) and dichloromethane (DCM) after concentration, and then precipitated in n-hexane (n-Hex) to obtain 32g of Compound 17-B as a white solid.

2) Synthesis of Monomer 4-17

Monomer 4-17 was obtained in the same manner as in the synthesis of Monomer 1.
MS: $[M+H]^+$=686

**<Synthesis of Monomer 4-18>**

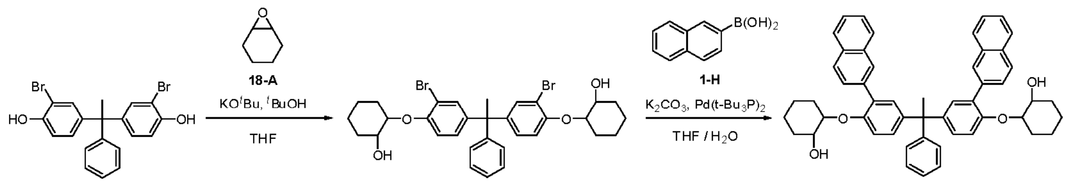


1-E 18-B Monomer 4-18

1) Synthesis of Compound 18-B

41.0g (91.5 mmol, 1.0 eq) of Compound 1-E was dissolved in 500 g of tetrahydrofuran (THF) and 500 g of $^t$BuOH, and 21.56 g (192 mmol, 2.1 eq) of KOtBu was added thereto. After the addition, 18.84 g (192 mmol, 2.0 eq) of Compound 18-A was slowly added thereto. Thereafter, the temperature was raised and the mixture was stirred under reflux for 72 hours. After cooling, the mixture was purified by column chromatography through ethyl acetate (EA) and dichloromethane (DCM) after concentration, and then precipitated in n-hexane (n-Hex) to obtain 35g of Compound 18-B as a white solid.

2) Synthesis of Monomer 4-18

Monomer 4-18 was obtained in the same manner as in the synthesis of Monomer 1. MS: $[M+H]^+$=738

**<Synthesis of Monomer 4-19>**

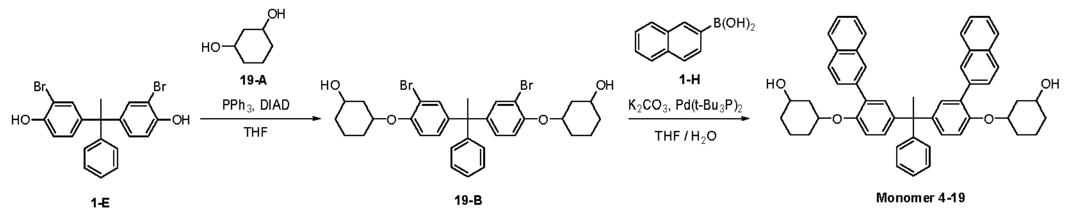


Monomer 4-19 was obtained in the same manner as in the synthesis of Monomer 4-17, except that Compound 19-A was used instead of Compound 17-A.
MS: $[M+H]^+$=738

**<Synthesis of Monomer 4-20>**

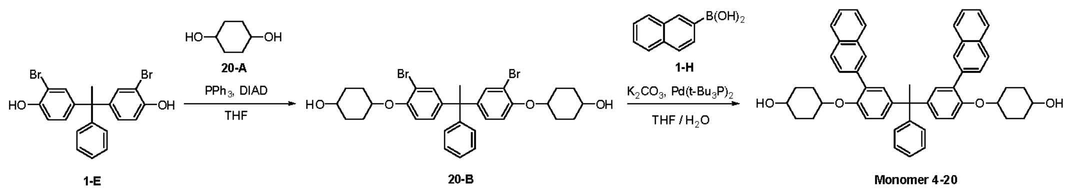


Monomer 4-20 was obtained in the same manner as in the synthesis of Monomer 4-17, except that Compound 20-A was used instead of Compound 17-A.
MS: $[M+H]^+$=738

**<Synthesis of Monomer 4-21>**

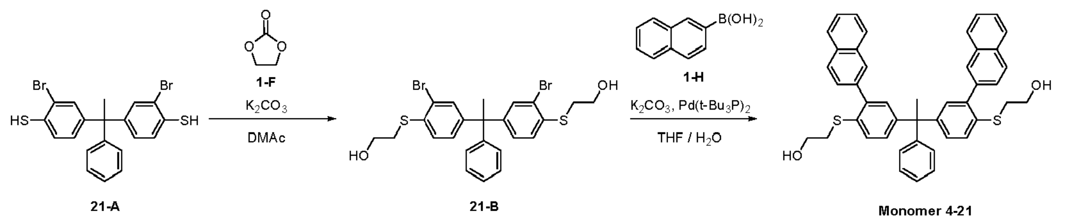


Monomer 4-21 was obtained in the same manner as in the synthesis of Monomer 4-1, except that Compound 21-A was used instead of Compound 1-E.
MS: $[M+H]^+$=662

**<Synthesis of Monomer 4-22>**

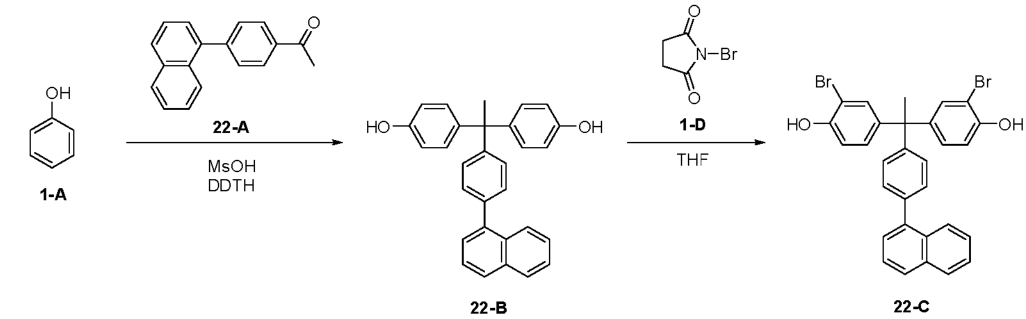


1-A 22-B 22-C

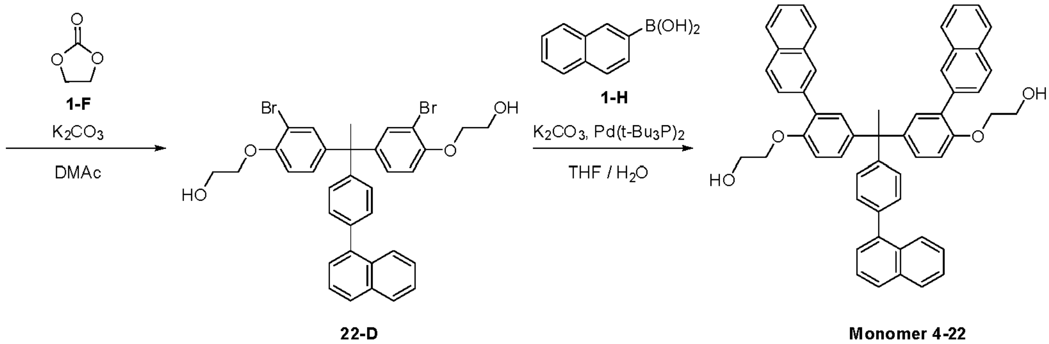


22-D Monomer 4-22

Monomer 4-22 was obtained in the same manner as in the synthesis of Monomer 4-1, except that Compound 22-A was used instead of Compound 1-B.
MS: $[M+H]^+$=756

## <Synthesis of Monomer 4-23>

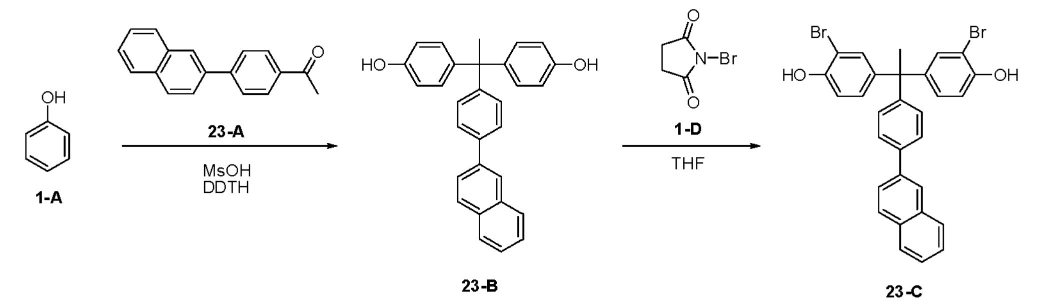


1-A 23-B 23-C

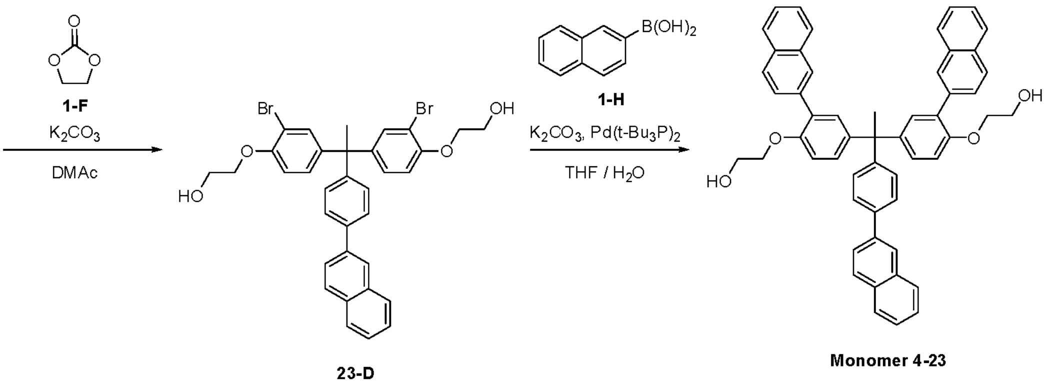


23-D Monomer 4-23

Monomer 4-23 was obtained in the same manner as in the synthesis of Monomer 4-1, except that Compound 23-A was used instead of Compound 1-B.

MS: $[M+H]^+$=756

**<Synthesis of Monomer 4-24>**

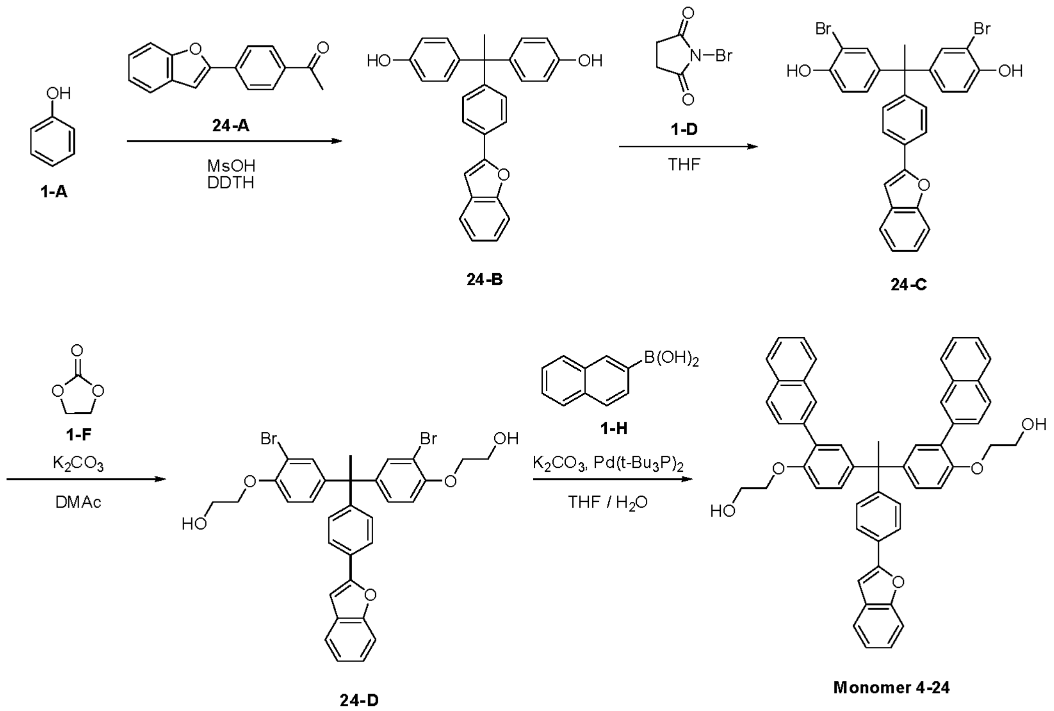


Monomer 4-24 was obtained in the same manner as in the synthesis of Monomer 4-1, except that Compound 24-A was used instead of Compound 1-B.
MS: $[M+H]^+$=746

**<Synthesis of Monomer 4-25>**

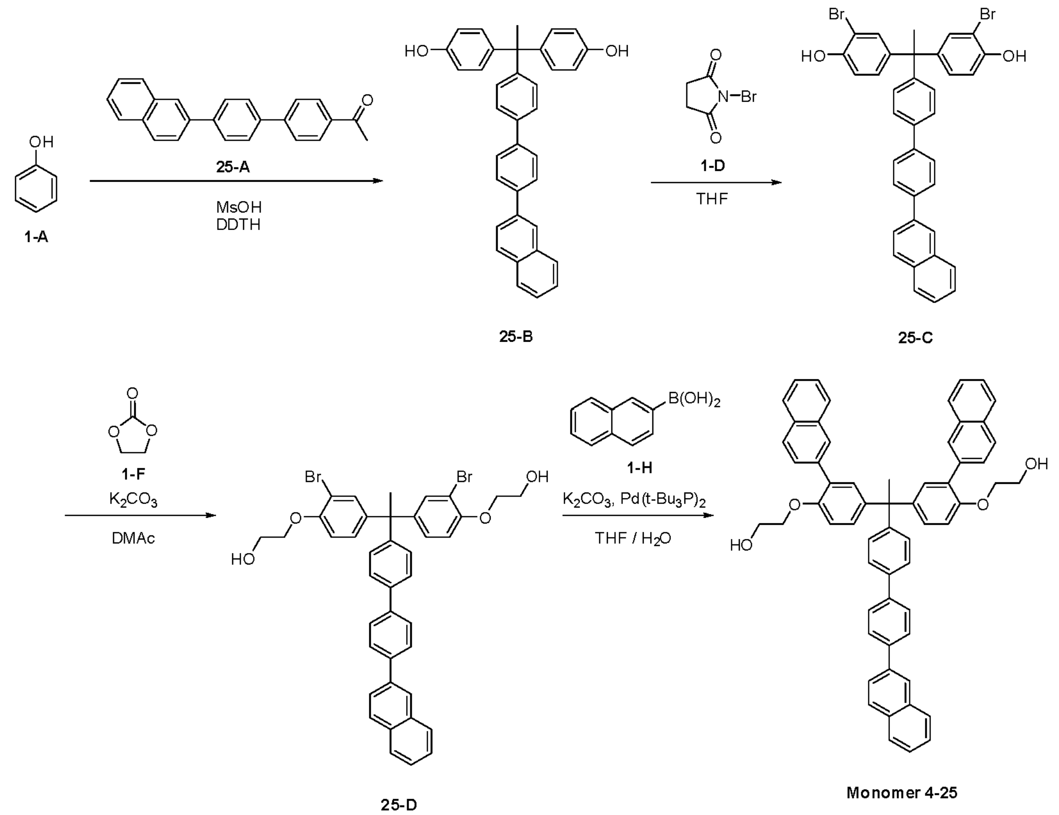


1-A 25-B 25-C 25-D Monomer 4-25

Monomer 4-25 was obtained in the same manner as in the synthesis of Monomer 4-1, except that Compound 25-A was used instead of Compound 1-B.
MS: $[M+H]^+$=832

## <Synthesis of Monomer 4-26>

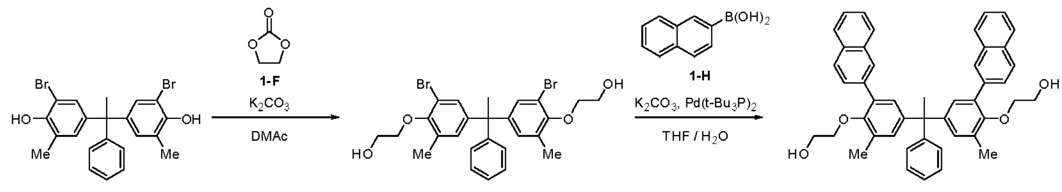


26-A 26-B Monomer 4-26

Monomer 4-26 was obtained in the same manner as in the synthesis of Monomer 4-1, except that Compound 26-A was used instead of Compound 1-E.
MS: $[M+H]^+$=658

## <Synthesis of Monomer 4-27>

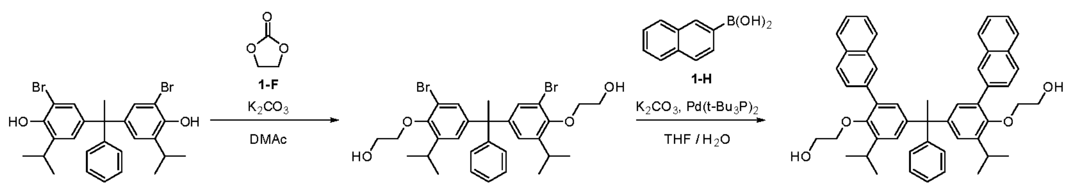


27-A 27-B Monomer 4-27

Monomer 4-27 was obtained in the same manner as in the synthesis of Monomer 4-1, except that Compound 27-A was used instead of Compound 1-E.
MS: $[M+H]^+$=658

**<Monomer C1>**

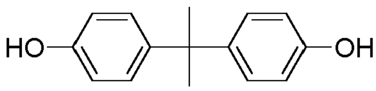

**Monomer C1**

Comparative Example Monomer C1 was obtained from TCI Chemical or Alfa Aesar.

**<Synthesis of Monomer C2>**

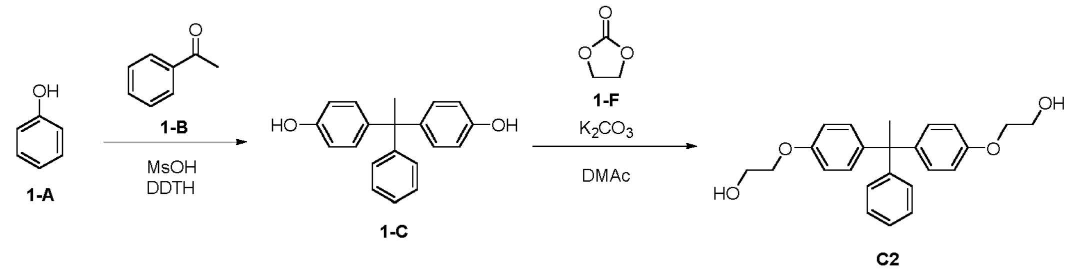


Monomer C2 was obtained in the same manner as in the synthesis of Monomer 2-1.

**<Synthesis of Monomer C3>**

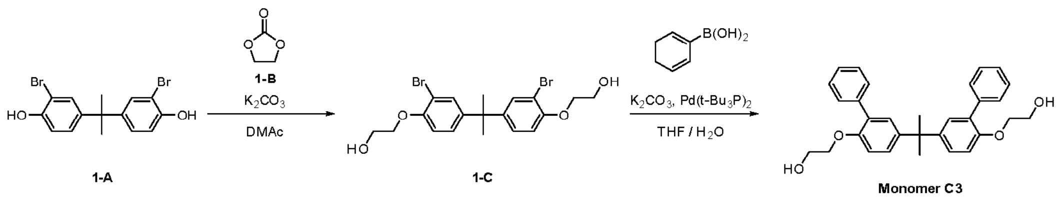


Monomer C3 was obtained in the same manner as in the synthesis of Monomer 3-1, except that phenylboronic acid was used instead of Compound 1-D.
MS: $[M+H]^+$=468

**<Synthesis of Monomer C4>**

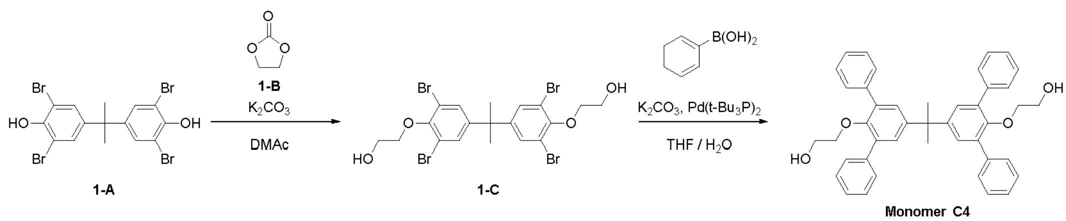

Monomer C4 was obtained in the same manner as in the synthesis of Monomer 3-1, except that phenylboronic acid was used instead of Compound 1-D.
MS: $[M+H]^+$=620.79

**<Examples>**

<Preparation of Polyester Resin 1-1>

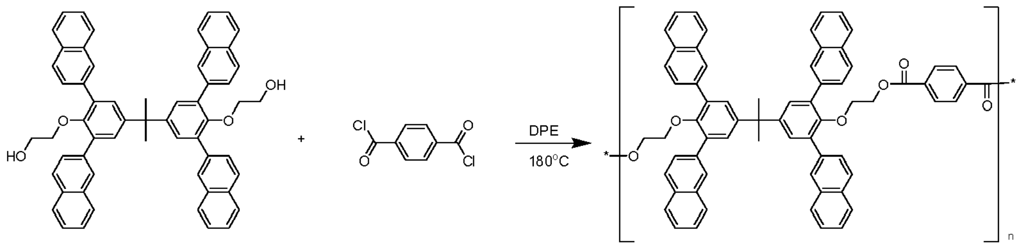


Monomer 1-1 Polyester resin 1-1

1.45 g (1.77 mmol, 1.0 eq) of Monomer 1-1 and 0.36 g (1.77 mmol, 1.0 eq) of terephthaloyl chloride were dissolved in 4.1 g of diphenyl ether (DPE), and the resulting solution was reacted in an oil bath at 180°C for 6 hours. Chloric acid (HCl) gas was generated as the reaction proceeded, and a nitrogen substitution device and a chloric acid gas capturing device were installed to remove the gas. After the reaction, the resulting product was cooled to 100°C, 15 g of dimethylacetamide (DMAc) was added thereto, and the resulting mixture was precipitated through methanol (methyl alcohol) to prepare Polyester Resin 1.

**<Preparation of Polyester Resins 1-2 to 1-23>**

Polyester Resins 1-2 to 1-23 were prepared in the same manner as in the method of preparing Polyester Resin 1, except that Monomers 1-2 to 1-23 were used instead of Monomer 1-1.

**<Preparation of Polyester Resin 2-1>**

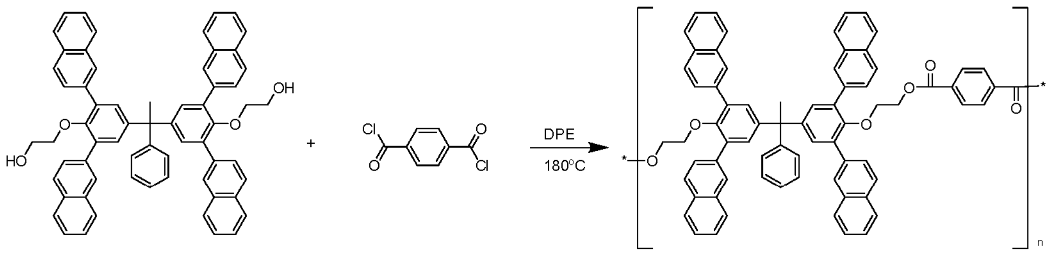


Monomer 2-1 Polyester resin 2-1

1.56 g (1.77 mmol, 1.0 eq) of Monomer 2-1 and 0.36 g (1.77 mmol, 1.0 eq) of terephthaloyl chloride were dissolved in 4.1 g of diphenyl ether (DPE), and the resulting solution was reacted in an oil bath at 180°C for 6 hours. Chloric acid (HCl) gas was generated as the reaction proceeded, and a nitrogen substitution device and a chloric acid gas capturing device were installed to remove the gas. After the reaction, the resulting product was cooled to 100°C, 15 g of dimethylacetamide (DMAc) was added thereto, and the resulting mixture was precipitated through methanol (methyl alcohol) to prepare Polyester Resin 2-1.

**<Preparation of Polyester Resins 2-2 to 2-25>**

Polyester Resins 2-2 to 2-25 were prepared in the same manner as in the method of preparing Polyester Resin 2-1, except that Monomers 2-2 to 2-25 were used instead of Monomer 2-1.

<Preparation of Polyester Resin 3-1>

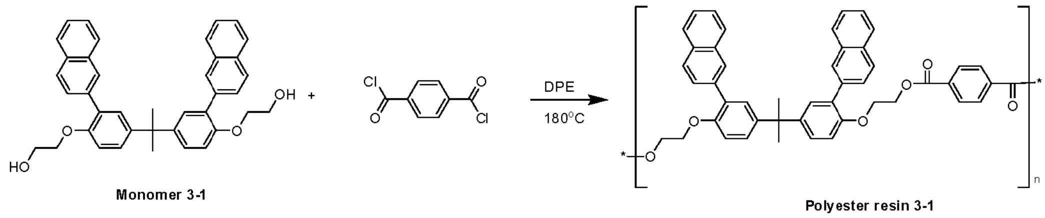


Monomer 3-1

Polyester resin 3-1

1.00g (1.77 mmol, 1.0 eq) of Monomer 3-1 and 0.36 g (1.77 mmol, 1.0 eq) of terephthaloyl chloride were dissolved in 4.1 g of diphenyl ether (DPE), and the resulting solution was reacted in an oil bath at 180°C for 6 hours. Chloric acid (HCl) gas was generated as the reaction proceeded, and a nitrogen substitution device and a chloric acid gas capturing device were installed to remove the gas. After the reaction, the resulting product was cooled to 100°C, 15 g of dimethylacetamide (DMAc) was added thereto, and the resulting mixture was precipitated through methanol (methyl alcohol) to prepare Polyester Resin 3-1.

<Preparation of Polyester Resins 3-2 to 3-25>

Polyester Resins 3-2 to 3-25 were prepared in the same manner as in the method of preparing Polyester Resin 3-1, except that Monomers 3-2 to 3-25 were used instead of Monomer 3-1.

<Preparation of Polyester Resin 4-1>

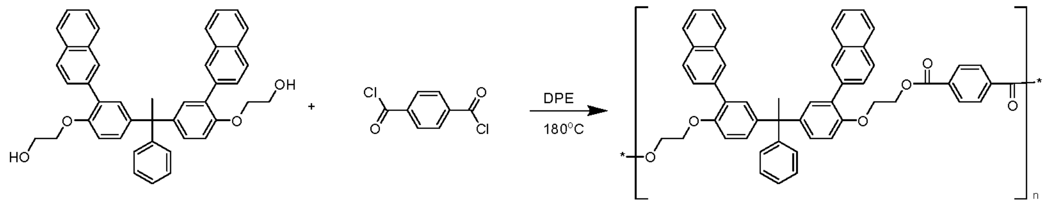


Monomer 4-1

Polyester resin 4-1

1.11 g (1.77 mmol, 1.0 eq) of Monomer 4-1 and 0.36 g (1.77 mmol, 1.0 eq) of terephthaloyl chloride were dissolved in 4.1 g of diphenyl ether (DPE), and the resulting solution was reacted in an oil bath at 180°C for 6 hours. Chloric acid (HCl) gas was generated as the reaction proceeded, and a nitrogen substitution device and a chloric acid gas capturing device were installed to remove the gas. After the reaction, the resulting product was cooled to 100°C, 15 g of dimethylacetamide (DMAc) was added thereto, and the resulting mixture was precipitated through methanol (methyl alcohol) to prepare Polyester Resin 4-1.

<Preparation of Polyester Resins 4-2 to 4-27>

Polyester Resins 4-2 to 4-27 were prepared in the same manner as in the method of preparing Polyester Resin 4-1, except that Monomers 4-2 to 4-27 were used instead of Monomer 4-1.

<Preparation of Comparative Example Resin PE1>

Comparative Example Resin PE1 was prepared in the same manner as in the method of preparing Polyester Resin 1-1, 2-1, 3-1 or 4-1, except that Monomer C1 was used instead of Monomer 1-1, 2-1, 3-1 or 4-1.

<Preparation of Comparative Example Resin PE2>

Comparative Example Resin PE2 was prepared in the same manner as in the method of preparing Polyester Resin 1-1, 2-1, 3-1 or 4-1, except that Monomer C2 was used instead of Monomer 1-1, 2-1, 3-1 or 4-1.

**<Preparation of Comparative Example Resin PE3>**

Comparative Example Resin PE3 was prepared in the same manner as in the method of preparing Polyester Resin 1-1, 2-1, 3-1 or 4-1, except that Monomer C3 was used instead of Monomer 1-1, 2-1, 3-1 or 4-1.

**<Preparation of Comparative Example Resin PE4>**

Comparative Example Resin PE4 was prepared in the same manner as in the method of preparing Polyester Resin 1-1, 2-1, 3-1 or 4-1, except that Monomer C4 was used instead of Monomer 1-1, 2-1, 3-1 or 4-1.

**<Preparation of Polycarbonate Resin 1-1>**

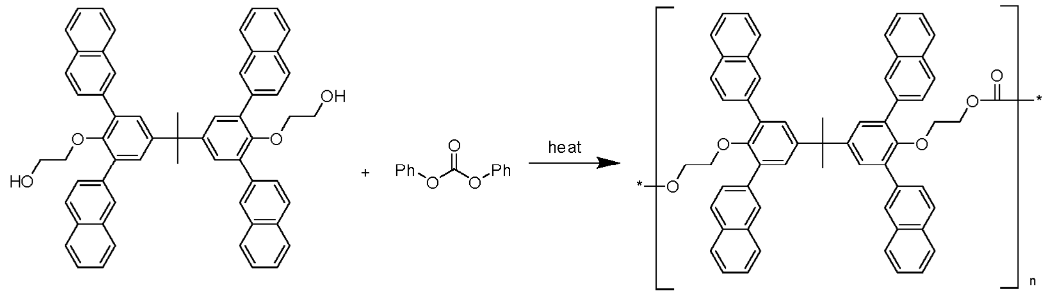


Monomer 1-1

Polycarbonate resin 1-1

As raw materials, 246.3 g (0.3 mol, 1 eq) of Monomer 1-1, 67.5 g (0.315 mol, 1.05 eq) of diphenyl carbonate (hereinafter sometimes abbreviated as "DPC"), and 0.37 mg ($4.4 \times 10^{-6}$ mol, 0.000015 eq) of sodium bicarbonate were put into a reactor, melted, and reacted at 250°C for 5 hours. As the reaction proceeded, phenol was generated as a by-product, and the degree of decompression was adjusted up to 1 Torr to remove the phenol. After completion of the reaction, the molten resin of a polymer polymerized by blowing nitrogen into the reactor to create a normal pressure atmosphere was taken out, whereby Polycarbonate Resin 1-1 was obtained.

**<Preparation of Polycarbonate Resins 1-2 to 1-23>**

Polycarbonate Resins 1-2 to 1-23 were prepared in the same manner as in the method of preparing Polycarbonate Resin 1-1, except that Monomers 1-2 to 1-23 were used instead of Monomer 1-1.

**<Preparation of Polycarbonate Resin 2-1>**

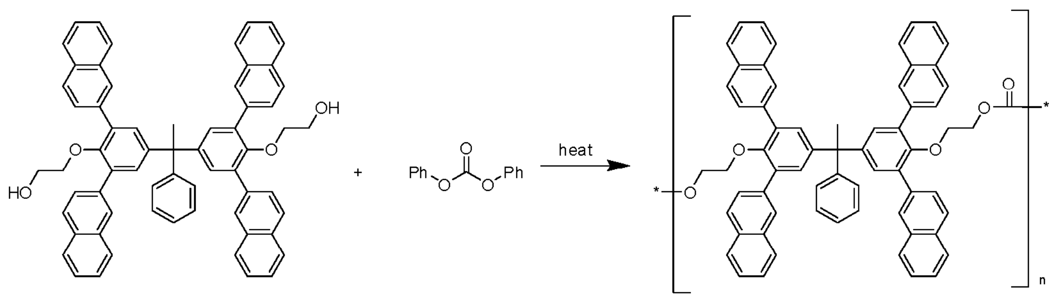


Monomer 2-1

Polycarbonate resin 2-1

As raw materials, 246.3 g (0.3 mol, 1 eq) of Monomer 2-1, 67.5 g (0.315 mol, 1.05 eq) of diphenyl carbonate (hereinafter sometimes abbreviated as "DPC"), and 0.37 mg ($4.4 \times 10^{-6}$ mol, 0.000015 eq) of sodium bicarbonate were put into a reactor, melted, and reacted at 250°C for 5 hours. As the reaction proceeded, phenol was generated as a by-product, and the degree of decompression was adjusted up to 1 Torr to remove the phenol. After completion of the reaction, the molten resin of a polymer polymerized by blowing nitrogen into the reactor to create a normal pressure atmosphere was

taken out, whereby Polycarbonate Resin 2-1 was obtained.

**<Preparation of Polycarbonate Resins 2-2 to 2-25>**

Polycarbonate Resins 2-2 to 2-25 were prepared in the same manner as in the method of preparing Polycarbonate Resin 2-1, except that Monomers 2-2 to 2-25 were used instead of Monomer 2-1.

**<Preparation of Polycarbonate Resin 3-1>**

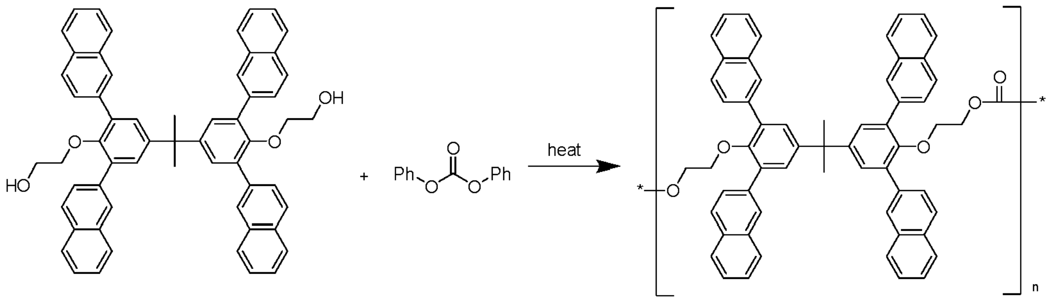


Monomer 3-1

Polycarbonate resin 3-1

As raw materials, 246.3 g (0.3 mol, 1 eq) of Monomer 3-1, 67.5 g (0.315 mol, 1.05 eq) of diphenyl carbonate (hereinafter sometimes abbreviated as "DPC"), and 0.37 mg (4.4 × $10^{-6}$ mol, 0.000015 eq) of sodium bicarbonate were put into a reactor, melted, and reacted at 250°C for 5 hours. As the reaction proceeded, phenol was generated as a by-product, and the degree of decompression was adjusted up to 1 Torr to remove the phenol. After completion of the reaction, the molten resin of a polymer polymerized by blowing nitrogen into the reactor to create a normal pressure atmosphere was taken out, whereby Polycarbonate Resin 3-1 was obtained.

**<Preparation of Polycarbonate Resins 3-2 to 3-25>**

Polycarbonate Resins 3-2 to 3-25 were prepared in the same manner as in the method of preparing Polycarbonate Resin 3-1, except that Monomers 3-2 to 3-25 were used instead of Monomer 3-1.

**<Preparation of Polycarbonate Resin 4-1>**

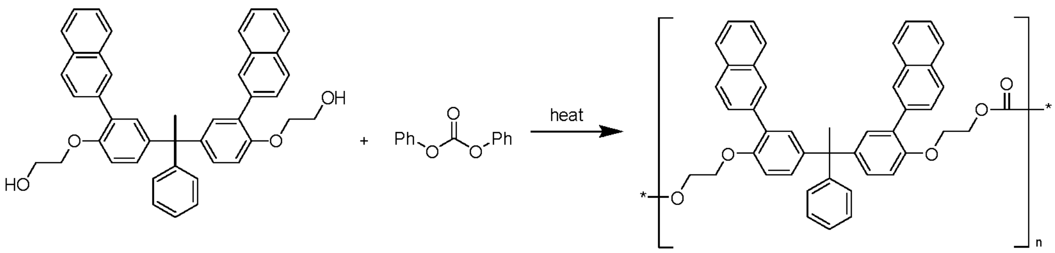


Monomer 4-1

Polycarbonate 4-1

As raw materials, 189.2g (0.3 mol, 1 eq) of Monomer 4-1, 67.5 g (0.315 mol, 1.05 eq) of diphenyl carbonate (hereinafter sometimes abbreviated as "DPC"), and 0.37 mg (4.4 × $10^{-6}$ mol, 0.000015 eq) of sodium bicarbonate were put into a reactor, melted, and reacted at 250°C for 5 hours. As the reaction proceeded, phenol was generated as a by-product, and the degree of decompression was adjusted up to 1 Torr to remove the phenol. After completion of the reaction, the molten resin of a polymer polymerized by blowing nitrogen into the reactor to create a normal pressure atmosphere was taken out, whereby Polycarbonate Resin 4-1 was obtained.

**<Preparation of Polycarbonate Resins 4-2 to 4-27>**

Polycarbonate Resins 4-2 to 4-27 were prepared in the same manner as in the method of preparing Polycarbonate Resin 4-1, except that Monomers 4-2 to 4-27 were used instead of Monomer 4-1.

**<Preparation of Comparative Example Resin PC1>**

Comparative Example Resin PC1 was prepared in the same manner as in the method of preparing Polycarbonate Resin 1-1, 2-1, 3-1 or 4-1, except that Monomer C1 was used instead of Monomer 1-1, 2-1, 3-1 or 4-1.

**<Preparation of Comparative Example Resin PC2>**

Comparative Example Resin PC2 was prepared in the same manner as in the method of preparing Polycarbonate Resin 1-1, 2-1, 3-1 or 4-1, except that Monomer C2 was used instead of Monomer 1-1, 2-1, 3-1 or 4-1.

**<Preparation of Comparative Example Resin PC3>**

Comparative Example Resin PC3 was prepared in the same manner as in the method of preparing Polycarbonate Resin 1-1, 2-1, 3-1 or 4-1, except that Monomer C3 was used instead of Monomer 1-1, 2-1, 3-1 or 4-1.

**<Preparation of Comparative Example Resin PC4>**

Comparative Example Resin PC4 was prepared in the same manner as in the method of preparing Polycarbonate Resin 1-1, 2-1, 3-1 or 4-1, except that Monomer C4 was used instead of Monomer 1-1, 2-1, 3-1 or 4-1.

**<Experimental Example>**

The molecular weight and molecular weight distribution of the polymerized resin sample were confirmed through gel permeation chromatography (GPC), and a thermogram was obtained using a differential scanning calorimeter (DSC) to investigate the thermal characteristics. After a film was formed to measure the refractive index and the Abbe's Number, a result value according to the wavelength of light was obtained using an ellipsometer.

For the molecular weight through gel permeation chromatography (GPC), results were obtained by injecting a solution produced by dissolving the resin sample in tetrahydrofuran (THF, stabilized with butylated hydroxytoluene (BHT)) as a solvent at a concentration of 1.0 mg/1 ml, and filtering the dissolved resin sample with a syringe filter, and measuring the molecular weight at 40°C, and the results are each shown in the following Tables 1 to 8. A Waters RI detector was used, and two Agilent PLgel MIXED-B columns were used.

A differential scanning calorimeter (DSC) was measured to determine the glass transition temperature (Tg) of the resin. A glass transition temperature (Tg) was obtained on a graph obtained by heating a 5.5 mg to 8.5 mg of the resin sample to 270°C under $N_2$ flow, cooling the resin sample, and then scanning the resin sample while heating the resin sample at a heating rate of 10°C/min during the second heating, and the glass transition temperature (Tg) is each shown in the following Tables 1 to 8.

In order to measure the refractive index and Abbe's Number of the resin, a polymer solution prepared by dissolving a resin powder sample obtained by polymerization in a solvent dimethylacetamide in an amount of 10 wt% based on the total weight of the polymer solution was applied onto a silicon wafer at a rotation speed of 220 rpm by spin coating to form a film having a thickness of 20 μm, and then the resulting values according to the wavelength of light were obtained at 20°C using an ellipsometer, and are each shown in the following Tables 1 to 8. Specifically, the refractive index was measured at a wavelength of 589 nm, and the Abbe's Number was obtained by the following Equation by measuring the refractive index ($n_D$, $n_F$, and $n_C$) at a wavelength of D (589 nm), F (486 nm), and C (656 nm), respectively.

$$\text{Abbe's Number} = (n_D - 1)/(n_F - n_C)$$

[Table 1]

| | Polyester resin | Mn (g/mol) | Mw (g/mol) | Tg(°C) | Refractive index (589nm) | Abbe's Number |
|---|---|---|---|---|---|---|
| Example 1-1 | 1-1 | 15200 | 27500 | 157 | 1.710 | 14.2 |

(continued)

| | Polyester resin | Mn (g/mol) | Mw (g/mol) | Tg(°C) | Refractive index (589nm) | Abbe's Number |
|---|---|---|---|---|---|---|
| Example 1-2 | 1-2 | 16500 | 29700 | 159 | 1.704 | 14.7 |
| Example 1-3 | 1-3 | 14600 | 26300 | 153 | 1.714 | 14.2 |
| Example 1-4 | 1-4 | 15100 | 27400 | 156 | 1.689 | 17.6 |
| Example 1-5 | 1-5 | 15200 | 27200 | 152 | 1.702 | 14.7 |
| Example 1-6 | 1-6 | 14600 | 26300 | 158 | 1.700 | 14.8 |
| Example 1-7 | 1-7 | 14800 | 26600 | 164 | 1.717 | 14.0 |
| Example 1-8 | 1-8 | 12800 | 23100 | 149 | 1.670 | 18.1 |
| Example 1-9 | 1-9 | 16700 | 30100 | 157 | 1.715 | 14.1 |
| Example 1-10 | 1-10 | 14500 | 26100 | 155 | 1.713 | 14.1 |
| Example 1-11 | 1-11 | 16100 | 28900 | 234 | 1.738 | 12.2 |
| Example 1-12 | 1-12 | 16600 | 29900 | 209 | 1.748 | 12.7 |
| Example 1-13 | 1-13 | 14600 | 26300 | 209 | 1.748 | 12.7 |
| Example 1-14 | 1-14 | 16100 | 28900 | 183 | 1.710 | 14.2 |
| Example 1-15 | 1-15 | 15200 | 27400 | 184 | 1.704 | 14.7 |
| Example 1-16 | 1-16 | 15100 | 27200 | 148 | 1.696 | 15.2 |
| Example 1-17 | 1-17 | 14800 | 26600 | 139 | 1.694 | 15.3 |
| Example 1-18 | 1-18 | 12800 | 23000 | 155 | 1.692 | 15.4 |
| Example 1-19 | 1-19 | 15800 | 28400 | 154 | 1.693 | 15.3 |
| Example 1-20 | 1-20 | 14600 | 26600 | 153 | 1.694 | 15.3 |
| Example 1-21 | 1-21 | 16800 | 31000 | 144 | 1.710 | 14.2 |
| Example 1-22 | 1-22 | 16500 | 28900 | 167 | 1.705 | 14.7 |
| Example 1-23 | 1-23 | 16200 | 28800 | 170 | 1.701 | 14.7 |
| Comparative Example 1-1 | PE1 | 18500 | 33300 | 152 | 1.582 | 34.1 |
| Comparative Example 1-2 | PE2 | 17000 | 31300 | 166 | 1.581 | 34.1 |
| Comparative Example 1-3 | PE3 | 14100 | 25400 | 151 | 1.635 | 23.3 |
| Comparative Example 1-4 | PE4 | 16900 | 30500 | 142 | 1.660 | 20.3 |

[Table 2]

| | Polycarbonate resin | Mn (g/mol) | Mw (g/mol) | Tg(°C) | Refractive index (589nm) | Abbe's Number |
|---|---|---|---|---|---|---|
| Example 2-1 | 1-1 | 18200 | 32700 | 153 | 1.714 | 14.0 |
| Example 2-2 | 1-2 | 17500 | 31500 | 158 | 1.708 | 14.5 |
| Example 2-3 | 1-3 | 15600 | 28100 | 151 | 1.717 | 14.0 |
| Example 2-4 | 1-4 | 17600 | 31600 | 151 | 1.689 | 17.6 |
| Example 2-5 | 1-5 | 17400 | 31400 | 148 | 1.704 | 14.7 |
| Example 2-6 | 1-6 | 17300 | 31100 | 155 | 1.702 | 14.7 |
| Example 2-7 | 1-7 | 18400 | 33100 | 163 | 1.721 | 13.9 |
| Example 2-8 | 1-8 | 18300 | 32900 | 148 | 1.667 | 20.2 |

(continued)

| | Polycarbonate resin | Mn (g/mol) | Mw (g/mol) | Tg(°C) | Refractive index (589nm) | Abbe's Number |
|---|---|---|---|---|---|---|
| Example 2-9 | 1-9 | 16800 | 30200 | 154 | 1.718 | 14.0 |
| Example 2-10 | 1-10 | 15700 | 31700 | 152 | 1.715 | 14.0 |
| Example 2-11 | 1-11 | 18300 | 32900 | 236 | 1.747 | 12.7 |
| Example 2-12 | 1-12 | 17700 | 31900 | 211 | 1.757 | 12.2 |
| Example 2-13 | 1-13 | 18000 | 32400 | 210 | 1.757 | 12.2 |
| Example 2-14 | 1-14 | 16700 | 33100 | 188 | 1.714 | 14.0 |
| Example 2-15 | 1-15 | 17800 | 32600 | 187 | 1.706 | 14.6 |
| Example 2-16 | 1-16 | 17400 | 31400 | 144 | 1.702 | 14.7 |
| Example 2-17 | 1-17 | 16900 | 30400 | 133 | 1.696 | 15.2 |
| Example 2-18 | 1-18 | 17200 | 31400 | 153 | 1.693 | 15.3 |
| Example 2-19 | 1-19 | 16800 | 28300 | 149 | 1.694 | 15.3 |
| Example 2-20 | 1-20 | 17400 | 31200 | 149 | 1.695 | 15.2 |
| Example 2-21 | 1-21 | 16600 | 31000 | 144 | 1.717 | 14.0 |
| Example 2-22 | 1-22 | 16600 | 29900 | 163 | 1.710 | 14.2 |
| Example 2-23 | 1-23 | 17000 | 31200 | 166 | 1.707 | 14.6 |
| Comparative Example 2-1 | PC1 | 18900 | 32200 | 150 | 1.586 | 34.0 |
| Comparative Example 2-2 | PC2 | 18700 | 35400 | 167 | 1.585 | 34.0 |
| Comparative Example 2-3 | PC3 | 15400 | 27800 | 152 | 1.641 | 23.1 |
| Comparative Example 2-4 | PC4 | 19200 | 34700 | 145 | 1.662 | 20.2 |

In Tables 1 and 2, Mn means the number average molecular weight, Mw means the weight average molecular weight, and the refractive index is a value measured at a wavelength of 589 nm.

According to Tables 1 and 2, the resins of Examples 1-1 to 1-23 and 2-1 to 2-23 comprise the unit of Chemical Formula 1 according to exemplary embodiments of the present specification, and may improve the refractive index by increasing the electron density of a bisphenol A core structure, particularly when substituted with an electron-rich substituent such as R1 to R4 of the benzene ring of the bisphenol A core structure.

In contrast, since the resins according to Comparative Examples 1-1 to 1-4 and 2-1 to 2-4 do not have electron-rich substituents on the benzene ring of the bisphenol A core structure, it can be confirmed that the refractive indices are much lower than those of the resins of Examples 1-1 to 1-23 and 2-1 to 2-23 comprising the unit of Chemical Formula 1 according to an exemplary embodiment of the present specification.

In order to appropriately apply the resin according to exemplary embodiments of the present specification to a molded article such as an optical lens, a high refractive index is preferentially required, and in the case of Comparative Examples 1-1 to 1-4 and 2-1 to 2-4, the refractive index is very low even though the Abbe's Number is higher than those of Examples 1-1 to 1-23 and 2-1 to 2-23, so that it could be confirmed that Examples 1-1 to 1-23 and 2-1 to 2-23 are better than Comparative Examples 1-1 to 1-4 and 2-1 to 2-4 as optical materials.

[Table 3]

| | Polyester resin | Mn (g/mol) | Mw (g/mol) | Tg(°C) | Refractive index (589nm) | Abbe's Number |
|---|---|---|---|---|---|---|
| Example 3-1 | 2-1 | 18500 | 33300 | 162 | 1.699 | 15.1 |
| Example 3-2 | 2-2 | 15200 | 27100 | 163 | 1.703 | 14.6 |
| Example 3-3 | 2-3 | 18000 | 32300 | 162 | 1.712 | 14.2 |
| Example 3-4 | 2-4 | 17500 | 31500 | 165 | 1.688 | 17.6 |

(continued)

| | Polyester resin | Mn (g/mol) | Mw (g/mol) | Tg(°C) | Refractive index (589nm) | Abbe's Number |
|---|---|---|---|---|---|---|
| Example 3-5 | 2-5 | 15400 | 27800 | 159 | 1.700 | 15.1 |
| Example 3-6 | 2-6 | 15900 | 28700 | 162 | 1.699 | 15.1 |
| Example 3-7 | 2-7 | 16200 | 29100 | 166 | 1.715 | 14.1 |
| Example 3-8 | 2-8 | 16700 | 30100 | 160 | 1.698 | 15.1 |
| Example 3-9 | 2-9 | 16600 | 29900 | 165 | 1.713 | 14.2 |
| Example 3-10 | 2-10 | 16800 | 30000 | 163 | 1.711 | 14.2 |
| Example 3-11 | 2-11 | 15500 | 27900 | 234 | 1.734 | 13.4 |
| Example 3-12 | 2-12 | 15600 | 28100 | 211 | 1.744 | 12.7 |
| Example 3-13 | 2-13 | 18500 | 33300 | 210 | 1.744 | 12.7 |
| Example 3-14 | 2-14 | 17900 | 32300 | 186 | 1.697 | 15.1 |
| Example 3-15 | 2-15 | 17300 | 31100 | 186 | 1.702 | 14.6 |
| Example 3-16 | 2-16 | 15500 | 28000 | 153 | 1.695 | 15.4 |
| Example 3-17 | 2-17 | 16500 | 29700 | 147 | 1.693 | 15.4 |
| Example 3-18 | 2-18 | 16600 | 29900 | 159 | 1.691 | 15.5 |
| Example 3-19 | 2-19 | 16900 | 30200 | 159 | 1.692 | 15.4 |
| Example 3-20 | 2-20 | 16500 | 29800 | 159 | 1.693 | 15.4 |
| Example 3-21 | 2-21 | 15900 | 28700 | 150 | 1.708 | 14.7 |
| Example 3-22 | 2-22 | 16000 | 28800 | 165 | 1.708 | 14.7 |
| Example 3-23 | 2-23 | 16900 | 30500 | 164 | 1.707 | 14.7 |
| Example 3-24 | 2-24 | 17300 | 31100 | 170 | 1.703 | 14.8 |
| Example 3-25 | 2-25 | 17900 | 32200 | 164 | 1.709 | 14.3 |
| Comparative Example 3-1 | PE1 | 18500 | 33300 | 152 | 1.582 | 34.1 |
| Comparative Example 3-2 | PE2 | 17000 | 31300 | 166 | 1.581 | 34.1 |
| Comparative Example 3-3 | PE3 | 14100 | 25400 | 151 | 1.635 | 23.3 |
| Comparative Example 3-4 | PE4 | 16900 | 30500 | 142 | 1.660 | 20.3 |

[Table 4]

| | Polycarbonate resin | Mn (g/mol) | Mw (g/mol) | Tg(°C) | Refractive index (589nm) | Abbe's Number |
|---|---|---|---|---|---|---|
| Example 4-1 | 2-1 | 16000 | 28800 | 163 | 1.706 | 14.7 |
| Example 4-2 | 2-2 | 17200 | 31000 | 164 | 1.708 | 14.7 |
| Example 4-3 | 2-3 | 18500 | 33300 | 160 | 1.714 | 14.2 |
| Example 4-4 | 2-4 | 18900 | 34000 | 162 | 1.689 | 17.5 |
| Example 4-5 | 2-5 | 16900 | 30500 | 158 | 1.702 | 15.1 |
| Example 4-6 | 2-6 | 15500 | 27700 | 163 | 1.701 | 15.1 |
| Example 4-7 | 2-7 | 16500 | 29700 | 167 | 1.719 | 14.1 |
| Example 4-8 | 2-8 | 15600 | 28100 | 158 | 1.705 | 14.7 |
| Example 4-9 | 2-9 | 16200 | 29200 | 163 | 1.716 | 14.2 |

(continued)

| | Polycarbonate resin | Mn (g/mol) | Mw (g/mol) | Tg(°C) | Refractive index (589nm) | Abbe's Number |
|---|---|---|---|---|---|---|
| Example 4-10 | 2-10 | 16600 | 29900 | 161 | 1.713 | 14.2 |
| Example 4-11 | 2-11 | 16700 | 30000 | 237 | 1.740 | 12.8 |
| Example 4-12 | 2-12 | 17300 | 31200 | 212 | 1.752 | 12.5 |
| Example 4-13 | 2-13 | 17800 | 32100 | 213 | 1.751 | 12.5 |
| Example 4-14 | 2-14 | 15900 | 28700 | 191 | 1.704 | 15.0 |
| Example 4-15 | 2-15 | 16600 | 29900 | 189 | 1.707 | 15.0 |
| Example 4-16 | 2-16 | 16800 | 30200 | 154 | 1.702 | 15.1 |
| Example 4-17 | 2-17 | 15500 | 27900 | 145 | 1.696 | 15.2 |
| Example 4-18 | 2-18 | 15700 | 28300 | 159 | 1.692 | 15.3 |
| Example 4-19 | 2-19 | 16000 | 28800 | 159 | 1.694 | 15.3 |
| Example 4-20 | 2-20 | 16600 | 29900 | 159 | 1.694 | 15.3 |
| Example 4-21 | 2-21 | 16500 | 29700 | 150 | 1.714 | 14.2 |
| Example 4-22 | 2-22 | 15900 | 28600 | 165 | 1.710 | 14.5 |
| Example 4-23 | 2-23 | 15500 | 27900 | 164 | 1.709 | 14.5 |
| Example 4-24 | 2-24 | 17300 | 31200 | 172 | 1.706 | 14.7 |
| Example 4-25 | 2-25 | 17800 | 32000 | 163 | 1.711 | 14.6 |
| Comparative Example 4-1 | PC1 | 18900 | 32200 | 150 | 1.586 | 34.0 |
| Comparative Example 4-2 | PC2 | 18700 | 35400 | 167 | 1.585 | 34.0 |
| Comparative Example 4-3 | PC3 | 15400 | 27800 | 152 | 1.641 | 23.1 |
| Comparative Example 4-4 | PC4 | 19200 | 34700 | 145 | 1.662 | 20.2 |

In Tables 3 and 4, Mn means the number average molecular weight, Mw means the weight average molecular weight, and the refractive index is a value measured at a wavelength of 589 nm.

According to Tables 3 and 4, the resins of Examples 3-1 to 3-25 and 4-1 to 4-25 comprise the unit of Chemical Formula 1 according to exemplary embodiments of the present specification, and may improve the refractive index by increasing the electron density of a bisphenol A core structure, particularly when substituted with an electron-rich substituent such as R1 to R4 of the benzene ring of the bisphenol A core structure.

In contrast, since the resins according to Comparative Examples 3-1 to 3-4 and 4-1 to 4-4 do not have electron-rich substituents on the benzene ring of the bisphenol A core structure, it can be confirmed that the refractive indices are much lower than those of the resins of Examples 3-1 to 3-25 and 4-1 to 4-25 comprising the unit of Chemical Formula 1 according to an exemplary embodiment of the present specification.

In order to appropriately apply the resin according to exemplary embodiments of the present specification to a molded article such as an optical lens, a high refractive index is preferentially required, and in the case of Comparative Examples 3-1 to 3-4 and 4-1 to 4-4, the refractive index is very low even though the Abbe's Number is higher than those of Examples 3-1 to 3-25 and 4-1 to 4-25, so that it could be confirmed that Examples 3-1 to 3-25 and 4-1 to 4-25 are better than Comparative Examples 3-1 to 3-4 and 4-1 to 4-4 as optical materials.

[Table 5]

| | Polyester resin | Mn (g/mol) | Mw (g/mol) | Tg(°C) | Refractive index (589nm) | Abbe's Number |
|---|---|---|---|---|---|---|
| Example 5-1 | 3-1 | 16100 | 28900 | 137 | 1.675 | 18.6 |
| Example 5-2 | 3-2 | 17300 | 31200 | 140 | 1.678 | 18.6 |
| Example 5-3 | 3-3 | 16600 | 29900 | 140 | 1.692 | 17.5 |

(continued)

| | Polyester resin | Mn (g/mol) | Mw (g/mol) | Tg(°C) | Refractive index (589nm) | Abbe's Number |
|---|---|---|---|---|---|---|
| Example 5-4 | 3-4 | 16500 | 29700 | 138 | 1.666 | 20.2 |
| Example 5-5 | 3-5 | 16200 | 29200 | 135 | 1.677 | 18.6 |
| Example 5-6 | 3-6 | 16500 | 28800 | 140 | 1.675 | 18.6 |
| Example 5-7 | 3-7 | 15400 | 27800 | 146 | 1.695 | 17.4 |
| Example 5-8 | 3-8 | 15500 | 28000 | 134 | 1.693 | 17.5 |
| Example 5-9 | 3-9 | 17400 | 31300 | 142 | 1.693 | 17.5 |
| Example 5-10 | 3-10 | 18300 | 33000 | 140 | 1.691 | 17.6 |
| Example 5-11 | 3-11 | 17300 | 31200 | 208 | 1.713 | 14.1 |
| Example 5-12 | 3-12 | 16500 | 29700 | 190 | 1.719 | 14.0 |
| Example 5-13 | 3-13 | 16900 | 30500 | 190 | 1.719 | 14.0 |
| Example 5-14 | 3-14 | 17200 | 31000 | 159 | 1.676 | 18.6 |
| Example 5-15 | 3-15 | 16800 | 29900 | 162 | 1.678 | 18.6 |
| Example 5-16 | 3-16 | 16600 | 30000 | 125 | 1.668 | 20.2 |
| Example 5-17 | 3-17 | 15300 | 27700 | 114 | 1.665 | 20.2 |
| Example 5-18 | 3-18 | 16500 | 29700 | 138 | 1.661 | 20.3 |
| Example 5-19 | 3-19 | 16400 | 29600 | 134 | 1.664 | 20.2 |
| Example 5-20 | 3-20 | 19300 | 34800 | 133 | 1.665 | 20.2 |
| Example 5-21 | 3-21 | 16500 | 29700 | 122 | 1.690 | 17.6 |
| Example 5-22 | 3-22 | 16000 | 28800 | 142 | 1.671 | 18.7 |
| Example 5-23 | 3-23 | 16300 | 29300 | 132 | 1.656 | 21.0 |
| Example 5-24 | 3-24 | 17200 | 29300 | 147 | 1.670 | 18.7 |
| Example 5-25 | 3-25 | 17800 | 31100 | 151 | 1.667 | 20.2 |
| Comparative Example 5-1 | PE1 | 18500 | 33300 | 152 | 1.582 | 34.1 |
| Comparative Example 5-2 | PE2 | 17000 | 31300 | 166 | 1.581 | 34.1 |
| Comparative Example 5-3 | PE3 | 14100 | 25400 | 151 | 1.635 | 23.3 |
| Comparative Example 5-4 | PE4 | 16900 | 30500 | 142 | 1.660 | 20.3 |

[Table 6]

| | Polycarbonate resin | Mn (g/mol) | Mw (g/mol) | Tg(°C) | Refractive index (589nm) | Abbe's Number |
|---|---|---|---|---|---|---|
| Example 6-1 | 3-1 | 18400 | 33100 | 131 | 1.672 | 18.6 |
| Example 6-2 | 3-2 | 17300 | 31200 | 134 | 1.677 | 18.6 |
| Example 6-3 | 3-3 | 17800 | 32000 | 137 | 1.696 | 17.4 |
| Example 6-4 | 3-4 | 16600 | 29900 | 130 | 1.661 | 20.3 |
| Example 6-5 | 3-5 | 16500 | 29800 | 131 | 1.671 | 18.6 |
| Example 6-6 | 3-6 | 16100 | 29000 | 132 | 1.671 | 18.6 |
| Example 6-7 | 3-7 | 16900 | 30500 | 141 | 1.701 | 14.7 |
| Example 6-8 | 3-8 | 15400 | 27800 | 131 | 1.698 | 15.2 |

(continued)

| | Polycarbonate resin | Mn (g/mol) | Mw (g/mol) | Tg(°C) | Refractive index (589nm) | Abbe's Number |
|---|---|---|---|---|---|---|
| Example 6-9 | 3-9 | 16000 | 28800 | 140 | 1.698 | 15.2 |
| Example 6-10 | 3-10 | 16100 | 28900 | 137 | 1.695 | 15.2 |
| Example 6-11 | 3-11 | 15500 | 27900 | 209 | 1.721 | 14.0 |
| Example 6-12 | 3-12 | 16700 | 30100 | 193 | 1.731 | 13.5 |
| Example 6-13 | 3-13 | 1850 | 33400 | 193 | 1.731 | 13.5 |
| Example 6-14 | 3-14 | 19300 | 34800 | 160 | 1.686 | 17.6 |
| Example 6-15 | 3-15 | 16500 | 29700 | 163 | 1.682 | 17.7 |
| Example 6-16 | 3-16 | 16700 | 30200 | 118 | 1.675 | 18.6 |
| Example 6-17 | 3-17 | 15800 | 28500 | 107 | 1.670 | 18.9 |
| Example 6-18 | 3-18 | 16600 | 29900 | 130 | 1.664 | 20.2 |
| Example 6-19 | 3-19 | 16900 | 30400 | 126 | 1.658 | 20.1 |
| Example 6-20 | 3-20 | 17300 | 31200 | 125 | 1.659 | 20.1 |
| Example 6-21 | 3-21 | 16900 | 30400 | 119 | 1.691 | 17.6 |
| Example 6-22 | 3-22 | 16200 | 29100 | 135 | 1.675 | 18.6 |
| Example 6-23 | 3-23 | 17100 | 30800 | 123 | 1.658 | 21.0 |
| Example 6-24 | 3-24 | 16000 | 28800 | 141 | 1.668 | 20.2 |
| Example 6-25 | 3-25 | 15800 | 27200 | 148 | 1.667 | 20.2 |
| Comparative Example 6-1 | PC1 | 18900 | 32200 | 150 | 1.586 | 34.0 |
| Comparative Example 6-2 | PC2 | 18700 | 35400 | 167 | 1.585 | 34.0 |
| Comparative Example 6-3 | PC3 | 15400 | 27800 | 152 | 1.641 | 23.1 |
| Comparative Example 6-4 | PC4 | 19200 | 34700 | 145 | 1.662 | 20.2 |

In Tables 5 and 6, Mn means the number average molecular weight, Mw means the weight average molecular weight, and the refractive index is a value measured at a wavelength of 589 nm.

According to Tables 5 and 6, the resins of Examples 5-1 to 5-25 and 6-1 to 6-25 comprises the unit of Chemical Formula 1 according to exemplary embodiments of the present specification, and may improve the refractive index by increasing the electron density of a bisphenol A core structure, particularly when R1 and R3 of the benzene ring of the bisphenol A core structure are substituted with an electron-rich substituent, such as an aryl group having 10 or more carbon atoms, or a heteroaryl group.

In contrast, since the resins according to Comparative Examples 5-1 to 5-4 and 6-1 to 6-4 do not have electron-rich substituents on the benzene ring of the bisphenol A core structure, it can be confirmed that the refractive indices are much lower than those of the resins of Examples 5-1 to 5-25 and 6-1 to 6-25 comprising the unit of Chemical Formula 1 according to an exemplary embodiment of the present specification.

In order to appropriately apply the resin according to exemplary embodiments of the present specification to a molded article such as an optical lens, a high refractive index is preferentially required, and in the case of Comparative Examples 5-1 to 5-4 and 6-1 to 6-4, the refractive index is very low even though the Abbe's Number is higher than those of Examples 5-1 to 5-25 and 6-1 to 6-25, so that it could be confirmed that Examples 5-1 to 5-25 and 6-1 to 6-25 are better than Comparative Examples 5-1 to 5-4 and 6-1 to 6-4 as optical materials.

[Table 7]

| | Polyester resin | Mn (g/mol) | Mw (g/mol) | Tg(°C) | Refractive index (589nm) | Abbe's Number |
|---|---|---|---|---|---|---|
| Example 7-1 | 4-1 | 16700 | 30100 | 148 | 1.674 | 18.6 |

(continued)

| | Polyester resin | Mn (g/mol) | Mw (g/mol) | Tg(°C) | Refractive index (589nm) | Abbe's Number |
|---|---|---|---|---|---|---|
| Example 7-2 | 4-2 | 15300 | 27700 | 149 | 1.677 | 18.6 |
| Example 7-3 | 4-3 | 16000 | 28900 | 151 | 1.690 | 17.6 |
| Example 7-4 | 4-4 | 17300 | 31200 | 151 | 1.670 | 18.6 |
| Example 7-5 | 4-5 | 18500 | 33300 | 146 | 1.675 | 18.3 |
| Example 7-6 | 4-6 | 15300 | 27600 | 149 | 1.673 | 18.6 |
| Example 7-7 | 4-7 | 16000 | 28800 | 154 | 1.693 | 15.6 |
| Example 7-8 | 4-8 | 16100 | 28900 | 155 | 1.690 | 15.2 |
| Example 7-9 | 4-9 | 16700 | 30100 | 154 | 1.691 | 15.2 |
| Example 7-10 | 4-10 | 14900 | 26900 | 153 | 1.689 | 16.6 |
| Example 7-11 | 4-11 | 17300 | 31200 | 215 | 1.710 | 14.5 |
| Example 7-12 | 4-12 | 17200 | 32100 | 194 | 1.715 | 14.2 |
| Example 7-13 | 4-13 | 16600 | 29900 | 193 | 1.714 | 14.2 |
| Example 7-14 | 4-14 | 16700 | 30100 | 168 | 1.670 | 18.6 |
| Example 7-15 | 4-15 | 15400 | 27800 | 169 | 1.677 | 18.2 |
| Example 7-16 | 4-16 | 16400 | 29600 | 135 | 1.668 | 19.9 |
| Example 7-17 | 4-17 | 17600 | 31700 | 127 | 1.665 | 20.2 |
| Example 7-18 | 4-18 | 18300 | 33000 | 147 | 1.662 | 20.5 |
| Example 7-19 | 4-19 | 15600 | 28100 | 147 | 1.665 | 20.2 |
| Example 7-20 | 4-20 | 15200 | 28200 | 147 | 1.665 | 20.2 |
| Example 7-21 | 4-21 | 14100 | 25700 | 132 | 1.686 | 17.6 |
| Example 7-22 | 4-22 | 16100 | 29000 | 152 | 1.688 | 17.6 |
| Example 7-23 | 4-23 | 16700 | 30100 | 151 | 1.690 | 17.6 |
| Example 7-24 | 4-24 | 16500 | 29700 | 150 | 1.689 | 17.6 |
| Example 7-25 | 4-25 | 16600 | 29800 | 165 | 1.692 | 15.1 |
| Example 7-26 | 4-26 | 17800 | 32100 | 151 | 1.671 | 18.6 |
| Example 7-27 | 4-27 | 17300 | 31200 | 143 | 1.668 | 20.2 |
| Comparative Example 7-1 | PE1 | 18500 | 33300 | 152 | 1.582 | 34.1 |
| Comparative Example 7-2 | PE2 | 17800 | 31200 | 164 | 1.583 | 34.1 |
| Comparative Example 7-3 | PE3 | 14100 | 25400 | 151 | 1.635 | 23.3 |
| Comparative Example 7-4 | PE4 | 16900 | 30500 | 142 | 1.660 | 20.3 |

[Table 8]

| | Polycarbonate resin | Mn (g/mol) | Mw (g/mol) | Tg(°C) | Refractive index (589nm) | Abbe's Number |
|---|---|---|---|---|---|---|
| Example 8-1 | 4-1 | 19800 | 35700 | 146 | 1.673 | 18.9 |
| Example 8-2 | 4-2 | 18400 | 33200 | 149 | 1.677 | 18.6 |
| Example 8-3 | 4-3 | 18500 | 33300 | 146 | 1.694 | 15.2 |
| Example 8-4 | 4-4 | 15400 | 27800 | 146 | 1.672 | 18.9 |

(continued)

| | Polycarbonate resin | Mn (g/mol) | Mw (g/mol) | Tg(°C) | Refractive index (589nm) | Abbe's Number |
|---|---|---|---|---|---|---|
| Example 8-5 | 4-5 | 14800 | 26700 | 143 | 1.673 | 18.9 |
| Example 8-6 | 4-6 | 16100 | 28900 | 147 | 1.673 | 18.8 |
| Example 8-7 | 4-7 | 16700 | 30200 | 153 | 1.699 | 15.1 |
| Example 8-8 | 4-8 | 16500 | 29700 | 147 | 1.690 | 17.6 |
| Example 8-9 | 4-9 | 16600 | 29900 | 149 | 1.696 | 15.2 |
| Example 8-10 | 4-10 | 16700 | 30100 | 148 | 1.693 | 15.2 |
| Example 8-11 | 4-11 | 16500 | 29800 | 214 | 1.713 | 14.2 |
| Example 8-12 | 4-12 | 15900 | 28700 | 196 | 1.725 | 13.5 |
| Example 8-13 | 4-13 | 15300 | 27700 | 196 | 1.725 | 13.5 |
| Example 8-14 | 4-14 | 16100 | 29000 | 170 | 1.675 | 18.6 |
| Example 8-15 | 4-15 | 15900 | 28700 | 172 | 1.682 | 17.8 |
| Example 8-16 | 4-16 | 16600 | 29900 | 135 | 1.668 | 20.2 |
| Example 8-17 | 4-17 | 17300 | 31200 | 121 | 1.661 | 20.5 |
| Example 8-18 | 4-18 | 16700 | 30100 | 145 | 1.657 | 21.5 |
| Example 8-19 | 4-19 | 17200 | 31100 | 141 | 1.660 | 20.5 |
| Example 8-20 | 4-20 | 16900 | 30500 | 140 | 1.661 | 20.5 |
| Example 8-21 | 4-21 | 15400 | 27800 | 131 | 1.689 | 16.6 |
| Example 8-22 | 4-22 | 18500 | 33300 | 155 | 1.690 | 17.6 |
| Example 8-23 | 4-23 | 17000 | 30700 | 155 | 1.693 | 16.6 |
| Example 8-24 | 4-24 | 15900 | 28700 | 153 | 1.692 | 16.6 |
| Example 8-25 | 4-25 | 15000 | 27000 | 168 | 1.695 | 16.6 |
| Example 8-26 | 4-26 | 16400 | 29600 | 151 | 1.690 | 17.6 |
| Example 8-27 | 4-27 | 15400 | 27800 | 137 | 1.691 | 17.5 |
| Comparative Example 8-1 | PC1 | 18100 | 32200 | 150 | 1.586 | 34.0 |
| Comparative Example 8-2 | PC2 | 17300 | 31200 | 165 | 1.585 | 34.0 |
| Comparative Example 8-3 | PC3 | 15400 | 27800 | 152 | 1.641 | 23.1 |
| Comparative Example 8-4 | PC4 | 19200 | 34700 | 145 | 1.662 | 20.2 |

In Tables 7 and 8, Mn means the number average molecular weight, Mw means the weight average molecular weight, and the refractive index is a value measured at a wavelength of 589 nm.

According to Tables 7 and 8, the resins of Examples 7-1 to 7-27 and 8-1 to 8-27 comprise the unit of Chemical Formula 1 according to exemplary embodiments of the present specification, and may improve the refractive index by increasing the electron density of a bisphenol A core structure, particularly when R1, R3 and R12 of the benzene ring of the bisphenol A core structure are substituted with an electron-rich substituent, such as an aryl group, or a heteroaryl group.

In contrast, since the resins according to Comparative Examples 7-1 to 7-4 and 8-1 to 8-4 do not have electron-rich substituents on the benzene ring of the bisphenol A core structure, it can be confirmed that the refractive indices are much lower than those of the resins of Examples 7-1 to 7-27 and 8-1 to 8-27 comprising the unit of Chemical Formula 1 according to an exemplary embodiment of the present specification.

In order to appropriately apply the resin according to exemplary embodiments of the present specification to a molded article such as an optical lens, a high refractive index is preferentially required, and in the case of Comparative Examples 7-1 to 7-4 and 8-1 to 8-4, the refractive index is very low even though the Abbe's Number is higher than those of Examples 7-1 to 7-27 and 8-1 to 8-27, so that it could be confirmed that Examples 7-1 to 7-27 and 8-1 to 8-27 are better than

Comparative Examples 7-1 to 7-4 and 8-1 to 8-4 as optical materials.
Specific embodiments of the disclosure are:

[Embodiment 1] A resin comprising a unit of the following Chemical Formula 1:

[Chemical Formula 1]

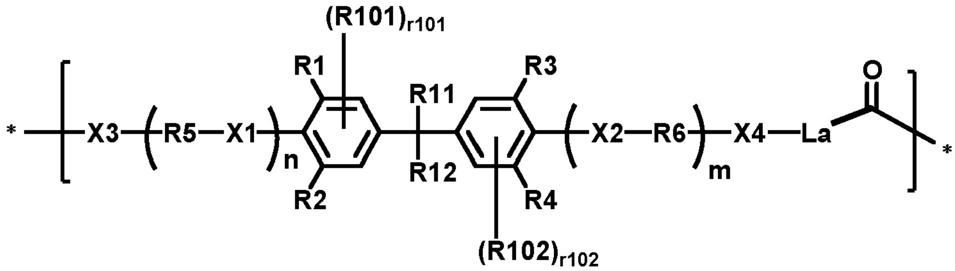

wherein, in Chemical Formula 1,

X1 to X4 are the same as or different from each other, and are each independently O or S,
at least one of R1 to R4 is a substituted or unsubstituted alkyl group; a substituted or unsubstituted aryl group; or a substituted or unsubstituted heteroaryl group, and the others are each independently hydrogen; a substituted or unsubstituted alkyl group; a substituted or unsubstituted aryl group; or a substituted or unsubstituted heteroaryl group,
R5 and R6 are the same as or different from each other, and are each independently a substituted or unsubstituted alkylene group; or a substituted or unsubstituted cycloalkylene group,
R11 and R12 are the same as or different from each other, and are each independently a substituted or unsubstituted alkyl group; a substituted or unsubstituted cycloalkyl group; a substituted or unsubstituted aryl group; or a substituted or unsubstituted heteroaryl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other,
La is a direct bond; or -C(=O)-L'-,
L' is a substituted or unsubstituted arylene group,
m and n are each 0 or 1, and
* means a moiety linked to the main chain of the resin.

[Embodiment 2] The resin of embodiment 1, wherein Chemical Formula 1 is any one of the following Chemical Formulae 1-1 to 1-4:

[Chemical Formula 1-1]

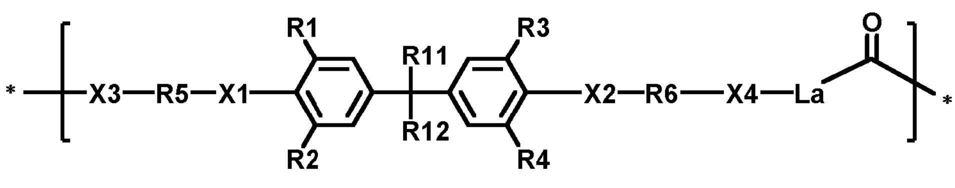

[Chemical Formula 1-2]

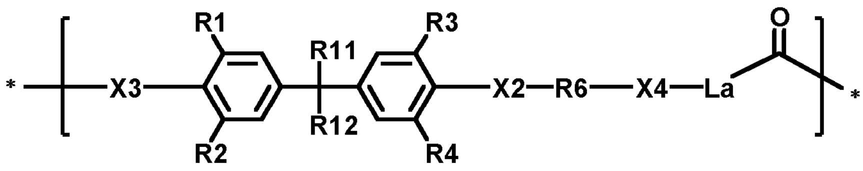

[Chemical Formula 1-3]

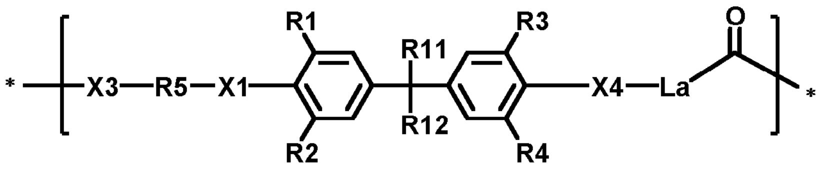

[Chemical Formula 1-4]

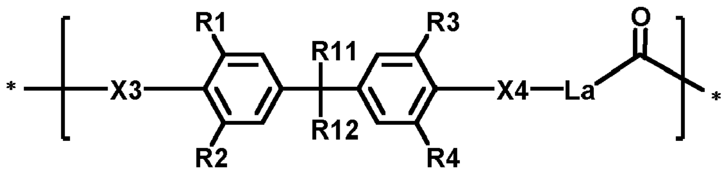

in Chemical Formulae 1-1 to 1-4,
the definitions of *, La, R1 to R6, R11, R12 and X1 to X4 are the same as those defined in Chemical Formula 1.
[Embodiment 3] The resin of embodiment 1, wherein Chemical Formula 1 is the following Chemical Formula 2:

[Chemical Formula 2]

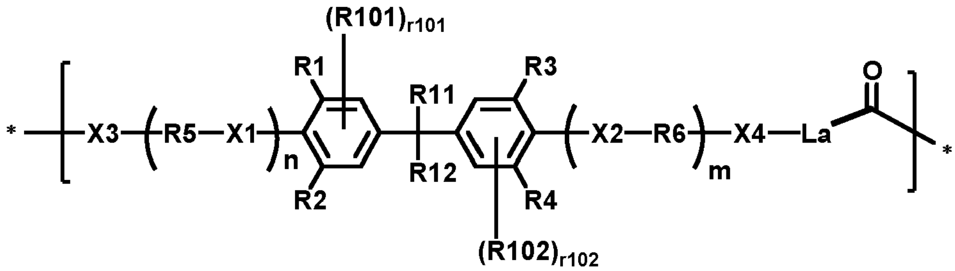

in Chemical Formula 2,

X1 to X4 are the same as or different from each other, and are each independently O or S,
R1 to R4 are the same as or different from each other, and are each independently a substituted or unsubstituted aryl group; or a substituted or unsubstituted heteroaryl group,
R5 and R6 are the same as or different from each other, and are each independently a substituted or unsubstituted alkylene group; or a substituted or unsubstituted cycloalkylene group,
R11 and R12 are the same as or different from each other, and are each independently a substituted or unsubstituted alkyl group; or a substituted or unsubstituted cycloalkyl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other,
La is a direct bond; or -C(=O)-L'-,

L' is a substituted or unsubstituted arylene group,
m and n are each 0 or 1, and
* means a moiety linked to the main chain of the resin.

[Embodiment 4] The resin of embodiment 1, wherein Chemical Formula 1 is the following Chemical Formula 3:

[Chemical Formula 3]

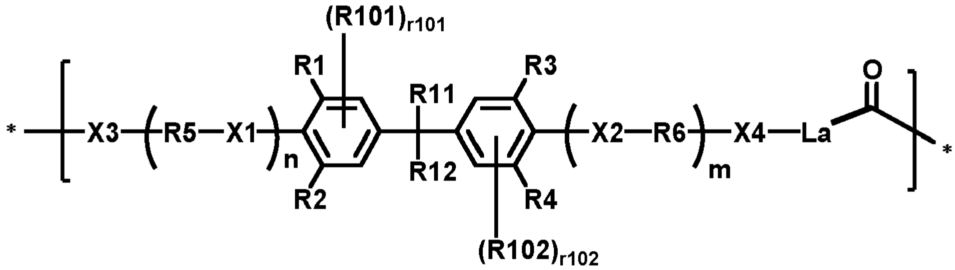

in Chemical Formula 3,

X1 to X4 are the same as or different from each other, and are each independently O or S,
R1 to R4 are the same as or different from each other, and are each independently a substituted or unsubstituted aryl group; or a substituted or unsubstituted heteroaryl group,
R5 and R6 are the same as or different from each other, and are each independently a substituted or unsubstituted alkylene group; or a substituted or unsubstituted cycloalkylene group,
R11 is a substituted or unsubstituted alkyl group; or a substituted or unsubstituted cycloalkyl group,
R12 is a substituted or unsubstituted aryl group; or a substituted or unsubstituted heteroaryl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other,
La is a direct bond; or -C(=O)-L'-,
L' is a substituted or unsubstituted arylene group,
m and n are each 0 or 1, and
* means a moiety linked to the main chain of the resin.

[Embodiment 5] The resin of embodiment 1, wherein Chemical Formula 1 is the following Chemical Formula 4:

[Chemical Formula 4]

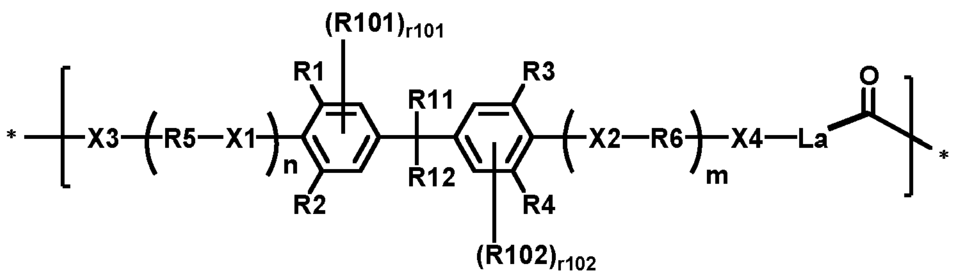

in Chemical Formula 4,

X1 to X4 are the same as or different from each other, and are each independently O or S,
R1 and R3 are the same as or different from each other, and are each independently a phenyl group which is substituted with one or more of a substituted or unsubstituted aryl group having 10 or more carbon atoms, a substituted or unsubstituted heteroaryl group, and a combination thereof; a substituted or unsubstituted aryl group having 10 or more carbon atoms; or a substituted or unsubstituted heteroaryl group,

R2 and R4 are the same as or different from each other, and are each independently hydrogen; or a substituted or unsubstituted alkyl group,
R5 and R6 are the same as or different from each other, and are each independently a substituted or unsubstituted alkylene group; or a substituted or unsubstituted cycloalkylene group,
R11 and R12 are the same as or different from each other, and are each independently a substituted or unsubstituted alkyl group; or a substituted or unsubstituted cycloalkyl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other,
La is a direct bond; or -C(=O)-L'-,
L' is a substituted or unsubstituted arylene group,
m and n are each 0 or 1, and
* means a moiety linked to the main chain of the resin.

[Embodiment 6] The resin of embodiment 1, wherein Chemical Formula 1 is the following Chemical Formula 5:

[Chemical Formula 5]

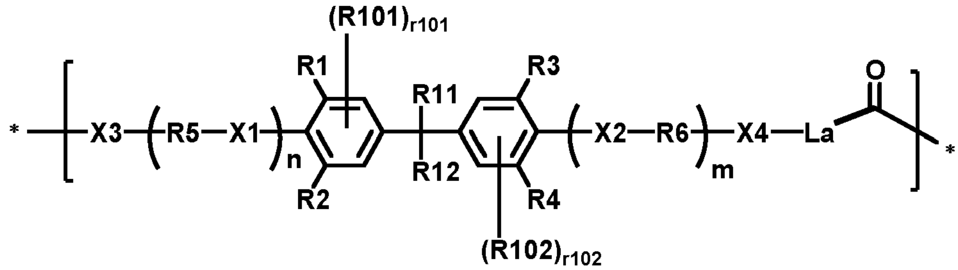

in Chemical Formula 5,

X1 to X4 are the same as or different from each other, and are each independently O or S,
R1 and R3 are the same as or different from each other, and are each independently a substituted or unsubstituted aryl group; or a substituted or unsubstituted heteroaryl group,
R2 and R4 are the same as or different from each other, and are each independently hydrogen; a substituted or unsubstituted alkyl group; or a substituted or unsubstituted cycloalkyl group,
R5 and R6 are the same as or different from each other, and are each independently a substituted or unsubstituted alkylene group; or a substituted or unsubstituted cycloalkylene group,
R11 is a substituted or unsubstituted alkyl group; or a substituted or unsubstituted cycloalkyl group,
R12 is a substituted or unsubstituted aryl group; or a substituted or unsubstituted heteroaryl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other,
La is a direct bond; or -C(=O)-L'-,
L' is a substituted or unsubstituted arylene group,
m and n are each 0 or 1, and
* means a moiety linked to the main chain of the resin.

[Embodiment 7] The resin of embodiment 3, wherein X1 to X4 are the same as or different from each other, and are each independently O; or S,

R1 to R4 are the same as or different from each other, and are each independently a monocyclic or polycyclic aryl group having 6 to 30 carbon atoms, which is unsubstituted or substituted with one or more of a straight-chained or branched alkyl group having 1 to 30 carbon atoms, a straight-chained or branched alkoxy group having 1 to 30 carbon atoms, a monocyclic or polycyclic aryloxy group having 6 to 30 carbon atoms, a monocyclic or polycyclic

aryl group having 6 to 30 carbon atoms, a polycyclic heteroaryl group having 6 to 30 carbon atoms, and a combination thereof; or a polycyclic heteroaryl group having 6 to 30 carbon atoms, which is unsubstituted or substituted with a monocyclic or polycyclic aryl group having 6 to 30 carbon atoms,

R5 and R6 are the same as or different from each other, and are each independently a straight-chained or branched alkylene group having 2 to 30 carbon atoms; or a monocyclic or polycyclic cycloalkylene group having 6 to 30 carbon atoms,

R11 and R12 are the same as or different from each other, and are each independently a straight-chained or branched alkyl group having 1 to 30 carbon atoms,

R101 and R102 are hydrogen,

La is a direct bond; or -C(=O)-L'-, and

L' is a monocyclic or polycyclic arylene group having 6 to 30 carbon atoms.

[Embodiment 8] The resin of embodiment 4, wherein X1 to X4 are the same as or different from each other, and are each independently O; or S,

R1 to R4 are the same as or different from each other, and are each independently a monocyclic or polycyclic aryl group having 6 to 30 carbon atoms, which is unsubstituted or substituted with one or more of a straight-chained or branched alkyl group having 1 to 30 carbon atoms, a straight-chained or branched alkoxy group having 1 to 30 carbon atoms, a monocyclic or polycyclic aryloxy group having 6 to 30 carbon atoms, a monocyclic or polycyclic aryl group having 6 to 30 carbon atoms, a polycyclic heteroaryl group having 6 to 30 carbon atoms, and a combination thereof; or a polycyclic heteroaryl group having 6 to 30 carbon atoms, which is unsubstituted or substituted with a monocyclic or polycyclic aryl group having 6 to 30 carbon atoms,

R5 and R6 are the same as or different from each other, and are each independently a straight-chained or branched alkylene group having 2 to 30 carbon atoms; or a monocyclic or polycyclic cycloalkylene group having 6 to 30 carbon atoms,

R11 is a straight-chained or branched alkyl group having 1 to 30 carbon atoms,

R12 is a monocyclic or polycyclic aryl group having 6 to 30 carbon atoms, which is unsubstituted or substituted with one or more of a monocyclic or polycyclic aryl group having 6 to 30 carbon atoms, a polycyclic heteroaryl group having 6 to 30 carbon atoms, and a combination thereof,

R101 and R102 are hydrogen,

La is a direct bond; or -C(=O)-L'-, and

L' is a monocyclic or polycyclic arylene group having 6 to 30 carbon atoms.

[Embodiment 9] The resin of embodiment 5, wherein X1 to X4 are the same as or different from each other, and are each independently O; or S,

R1 and R3 are the same as or different from each other, and are each independently a polycyclic aryl group having 10 to 30 carbon atoms, which is unsubstituted or substituted with a straight-chained or branched alkyl group having 1 to 30 carbon atoms, a straight-chained or branched alkoxy group having 1 to 30 carbon atoms, or a monocyclic or polycyclic aryl group having 6 to 30 carbon atoms; a phenyl group which is substituted with one or more of a monocyclic or polycyclic aryl group having 10 to 30 carbon atoms, a polycyclic heteroaryl group having 6 to 30 carbon atoms, which is unsubstituted or substituted with a monocyclic or polycyclic aryl group having 6 to 30 carbon atoms, and a combination thereof; or a polycyclic heteroaryl group having 6 to 30 carbon atoms, which is unsubstituted or substituted with a monocyclic or polycyclic aryl group having 6 to 30 carbon atoms,

R2 and R4 are the same as or different from each other, and are each independently hydrogen; or a substituted or unsubstituted straight-chained or branched alkyl group having 1 to 30 carbon atoms, which is unsubstituted or substituted with a monocyclic or polycyclic aryl group having 6 to 30 carbon atoms,

R5 and R6 are the same as or different from each other, and are each independently a straight-chained or branched alkylene group having 2 to 30 carbon atoms; or a monocyclic or polycyclic cycloalkylene group having 6 to 30 carbon atoms,

R11 and R12 are the same as or different from each other, and are each independently a straight-chained or branched alkyl group having 1 to 30 carbon atoms,

R101 and R102 are hydrogen,

La is a direct bond; or -C(=O)-L'-, and

L' is a monocyclic or polycyclic arylene group having 6 to 30 carbon atoms.

[Embodiment 10] The resin of embodiment 6, wherein X1 to X4 are the same as or different from each other, and are each independently O; or S,

R1 and R3 are the same as or different from each other, and are each independently a monocyclic or polycyclic aryl group having 6 to 30 carbon atoms, which is unsubstituted or substituted with one or more of a straight-chained or branched alkyl group having 1 to 30 carbon atoms, a straight-chained or branched alkoxy group having 1 to 30 carbon atoms, a monocyclic or polycyclic aryl group having 6 to 30 carbon atoms, a polycyclic heteroaryl group having 6 to 30 carbon atoms, and a combination thereof; or a polycyclic heteroaryl group having 6 to 30 carbon atoms, which is unsubstituted or substituted with a monocyclic or polycyclic aryl group having 6 to 30 carbon atoms,

R2 and R4 are the same as or different from each other, and are each independently hydrogen; or a straight-chained or branched alkyl group having 1 to 30 carbon atoms,

R5 and R6 are the same as or different from each other, and are each independently a straight-chained or branched alkylene group having 2 to 30 carbon atoms; or a monocyclic or polycyclic cycloalkylene group having 6 to 30 carbon atoms,

R11 is a straight-chained or branched alkyl group having 1 to 30 carbon atoms,

R12 is a monocyclic or polycyclic aryl group having 6 to 30 carbon atoms, which is unsubstituted or substituted with a monocyclic or polycyclic aryl group having 6 to 30 carbon atoms; or a polycyclic heteroaryl group having 6 to 30 carbon atoms,

R101 and R102 are hydrogen,

La is a direct bond; or -C(=O)-L'-, and

L' is a monocyclic or polycyclic arylene group having 6 to 30 carbon atoms.

[Embodiment 11] The resin of embodiment 1, wherein the resin has a weight average molecular weight (Mw) of 10,000 g/mol to 200,000 g/mol.

[Embodiment 12] The resin of embodiment 1, wherein a refractive index of the resin, which is measured at a wavelength of 589 nm is 1.60 to 1.80.

[Embodiment 13] The resin of embodiment 1, wherein the resin has a glass transition temperature (Tg) of 100°C to 260°C.

[Embodiment 14] The resin of embodiment 1, wherein the Abbe's Number of the resin, which is measured at a wavelength of 589 nm, 486 nm, and 656 nm, is 8 to 25.

[Embodiment 15] A compound of the following Chemical Formula 1a:

[Chemical Formula 1a]

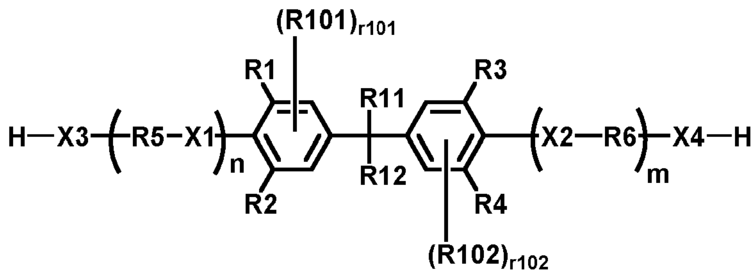

wherein, in Chemical Formula 1a,

X1 to X4 are the same as or different from each other, and are each independently O or S,

at least one of R1 to R4 is a substituted or unsubstituted alkyl group; a substituted or unsubstituted aryl group; or a substituted or unsubstituted heteroaryl group, and the others are each independently hydrogen; a substituted or unsubstituted alkyl group; a substituted or unsubstituted aryl group; or a substituted or unsubstituted heteroaryl group,

R5 and R6 are the same as or different from each other, and are each independently a substituted or unsubstituted alkylene group; or a substituted or unsubstituted cycloalkylene group,

R11 and R12 are the same as or different from each other, and are each independently a substituted or unsubstituted alkyl group; a substituted or unsubstituted cycloalkyl group; a substituted or unsubstituted aryl group; or a substituted or unsubstituted heteroaryl group,

R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,

r101 is 1 or 2, and when r101 is 2, two R101's are the same as or different from each other,

r102 is 1 or 2, and when r102 is 2, two R102's are the same as or different from each other, and
m and n are each 0 or 1.

[Embodiment 16] The compound of embodiment 15, wherein Chemical Formula 1a is the following Chemical Formula 2a:

[Chemical Formula 2a]

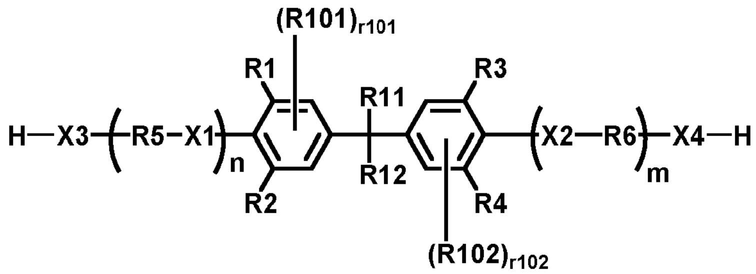

in Chemical Formula 2a,

X1 to X4 are the same as or different from each other, and are each independently O or S,
R1 to R4 are the same as or different from each other, and are each independently a substituted or unsubstituted aryl group; or a substituted or unsubstituted heteroaryl group,
R5 and R6 are the same as or different from each other, and are each independently a substituted or unsubstituted alkylene group; or a substituted or unsubstituted cycloalkylene group,
R11 and R12 are the same as or different from each other, and are each independently a substituted or unsubstituted alkyl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other, and
m and n are each 0 or 1.

[Embodiment 17] The compound of embodiment 15, wherein Chemical Formula 1a is the following Chemical Formula 3a:

[Chemical Formula 3a]

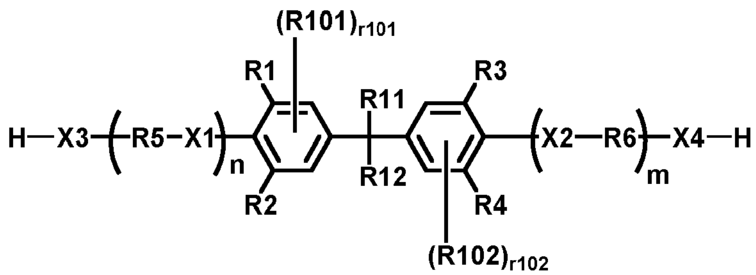

in Chemical Formula 3a,

X1 to X4 are the same as or different from each other, and are each independently O or S,
R1 to R4 are the same as or different from each other, and are each independently a substituted or unsubstituted aryl group; or a substituted or unsubstituted heteroaryl group,
R5 and R6 are the same as or different from each other, and are each independently a substituted or unsubstituted alkylene group; or a substituted or unsubstituted cycloalkylene group,
R11 is a substituted or unsubstituted alkyl group; or a substituted or unsubstituted cycloalkyl group,
R12 is a substituted or unsubstituted aryl group; or a substituted or unsubstituted heteroaryl group,
R101 and R102 are the same as or different from each other, and are each independently hydrogen; a substituted

or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other, and
m and n are each 0 or 1.

[Embodiment 18] The compound of embodiment 15, wherein Chemical Formula 1a is the following Chemical Formula 4a:

[Chemical Formula 4a]

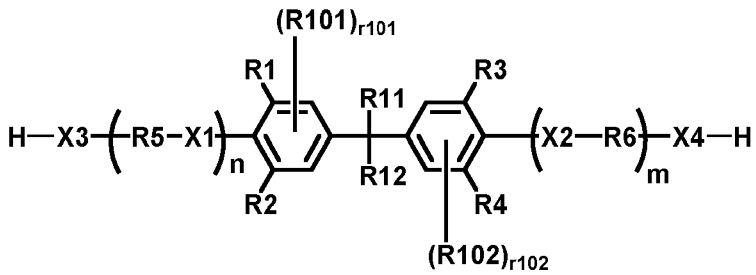

in Chemical Formula 4a,

X1 to X4 are the same as or different from each other, and are each independently O or S,
R1 and R3 are the same as or different from each other, and are each independently a phenyl group which is substituted with one or more of a substituted or unsubstituted aryl group having 10 or more carbon atoms, a substituted or unsubstituted heteroaryl group, and a combination thereof; a substituted or unsubstituted aryl group having 10 or more carbon atoms; or a substituted or unsubstituted heteroaryl group,
R2 and R4 are the same as or different from each other, and are each independently hydrogen; or a substituted or unsubstituted alkyl group,
R5 and R6 are the same as or different from each other, and are each independently a substituted or unsubstituted alkylene group; or a substituted or unsubstituted cycloalkylene group,
R11 and R12 are the same as or different from each other, and are each independently a substituted or unsubstituted alkyl group; or a substituted or unsubstituted cycloalkyl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other, and
m and n are each 0 or 1.

[Embodiment 19] The compound of embodiment 15, wherein Chemical Formula 1a is the following Chemical Formula 5a:

[Chemical Formula 5a]

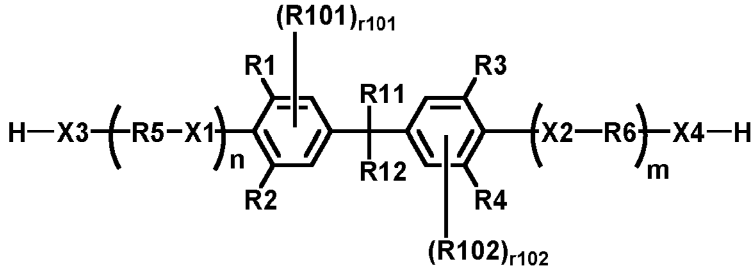

in Chemical Formula 5a,

X1 to X4 are the same as or different from each other, and are each independently O or S,

R1 and R3 are the same as or different from each other, and are each independently a substituted or unsubstituted aryl group; or a substituted or unsubstituted heteroaryl group,
R2 and R4 are the same as or different from each other, and are each independently hydrogen; a substituted or unsubstituted alkyl group; or a substituted or unsubstituted cycloalkyl group,
R5 and R6 are the same as or different from each other, and are each independently a substituted or unsubstituted alkylene group; or a substituted or unsubstituted cycloalkylene group,
R11 is a substituted or unsubstituted alkyl group; or a substituted or unsubstituted cycloalkyl group,
R12 is a substituted or unsubstituted aryl group; or a substituted or unsubstituted heteroaryl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other, and
m and n are each 0 or 1.

[Embodiment 20] The compound of embodiment 16, wherein Chemical Formula 2a is any one selected from the following compounds:

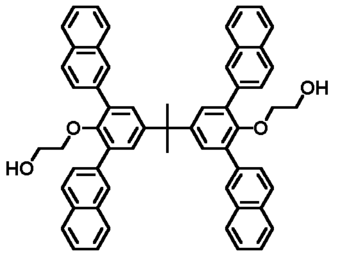
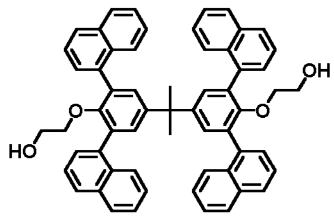
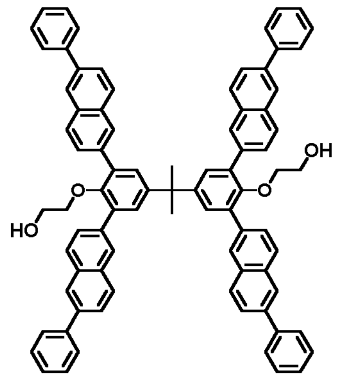
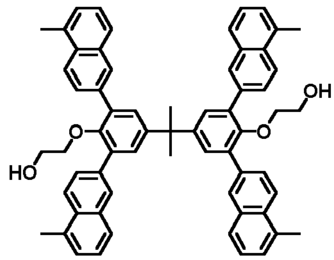
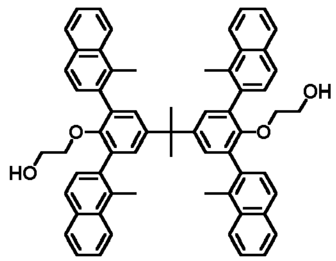
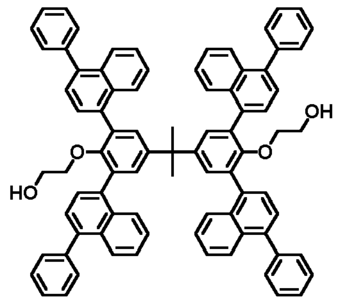
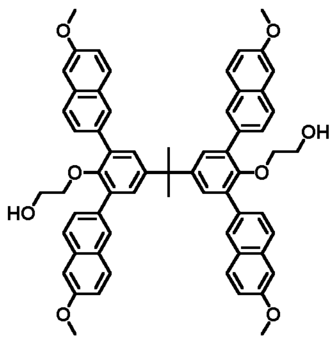

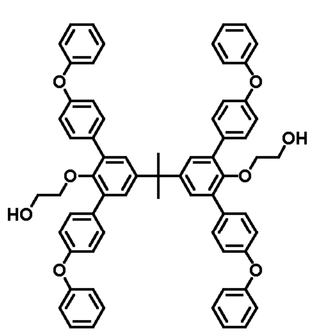
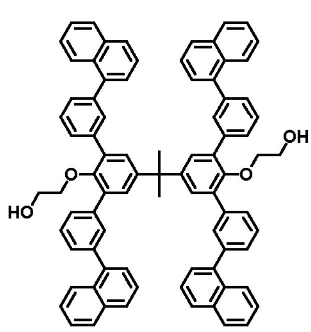
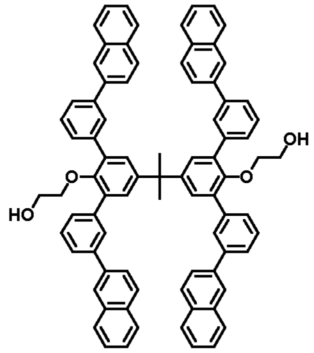

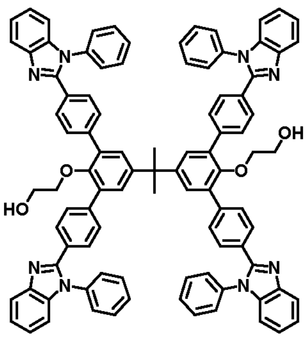
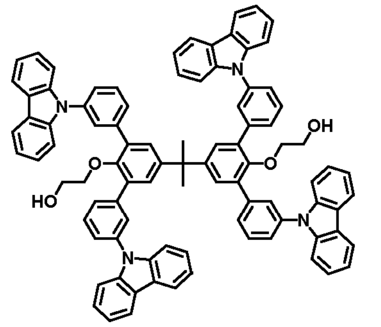
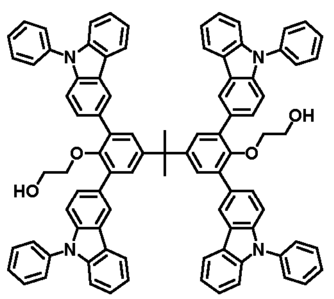

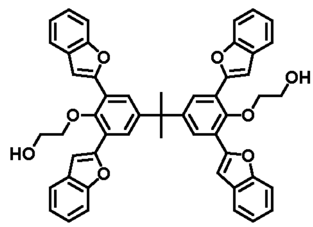
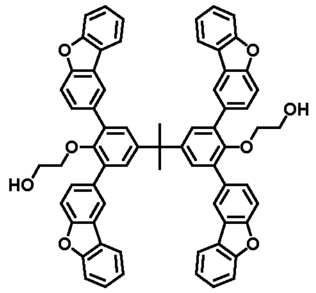

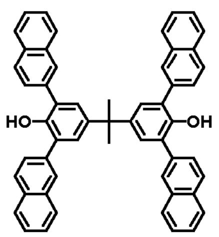
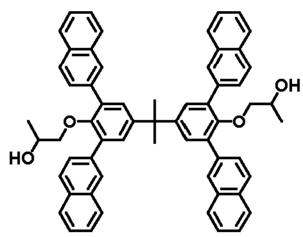
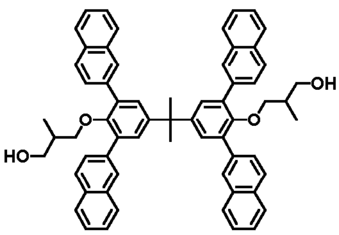

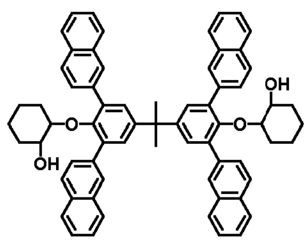
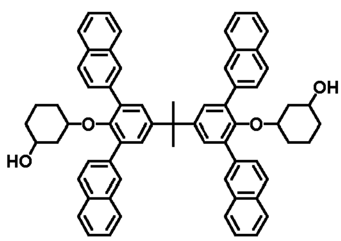
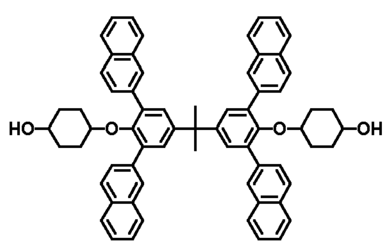

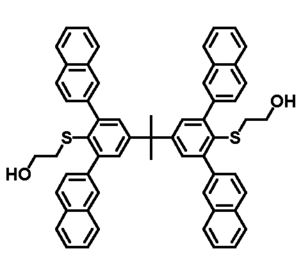
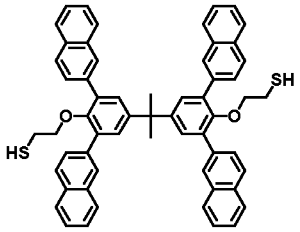
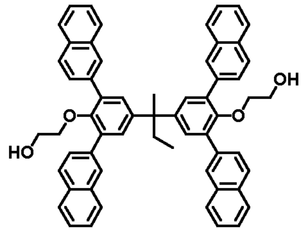

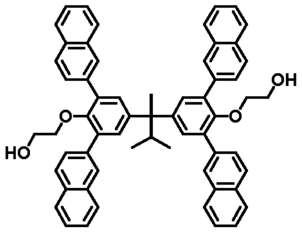

[Embodiment 21] The compound of embodiment 17, wherein Chemical Formula 3a is any one selected from the following compounds:

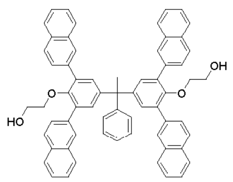
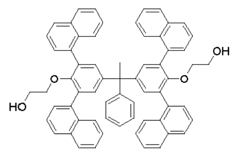
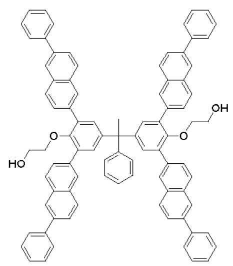
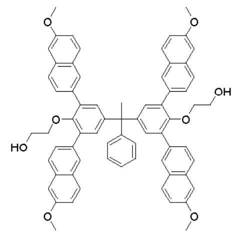

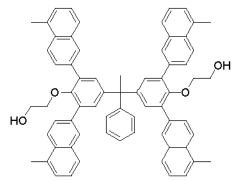
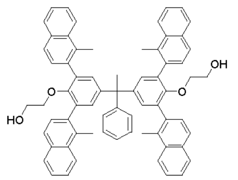
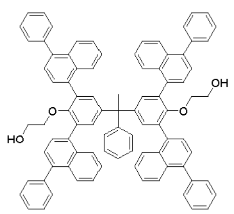

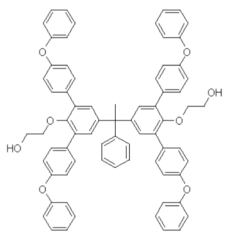
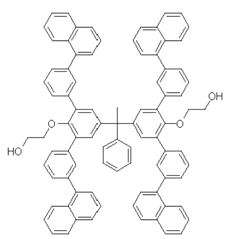
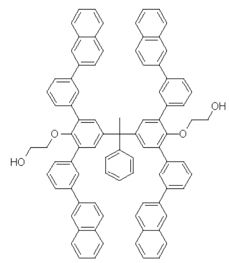
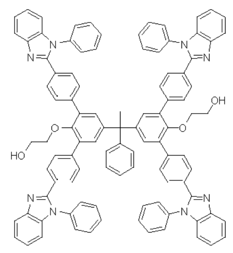

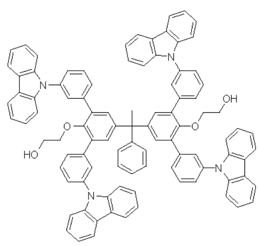
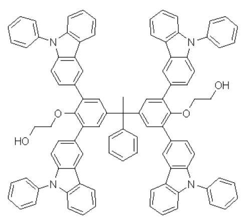
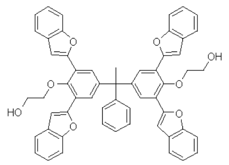
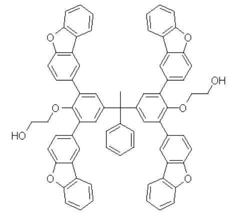

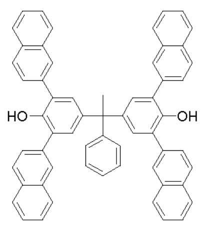
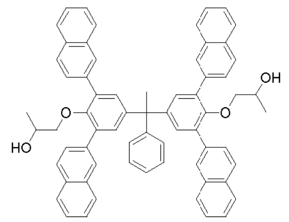
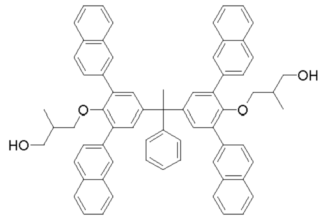

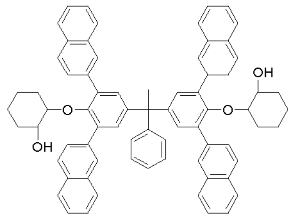
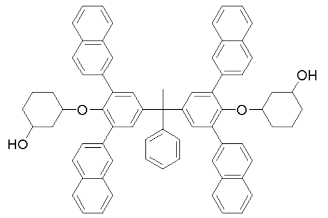
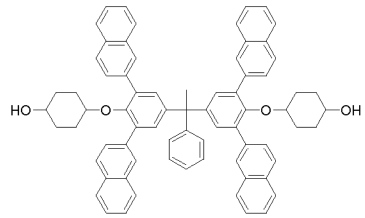

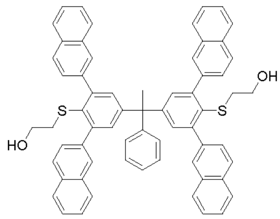
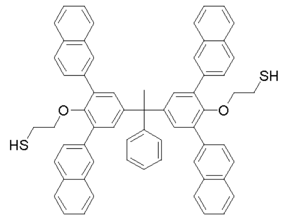

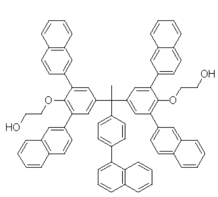
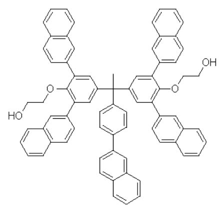
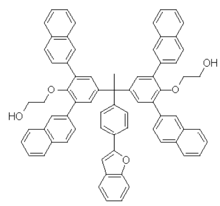
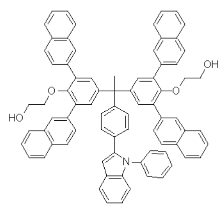

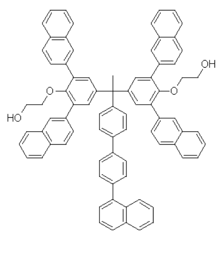
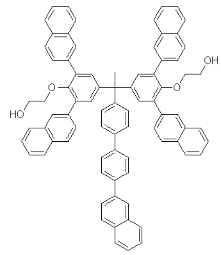
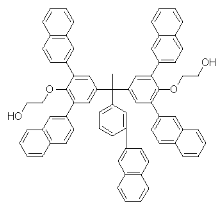

[Embodiment 22] The compound of embodiment 18, wherein Chemical Formula 4a is any one selected from the following compounds:

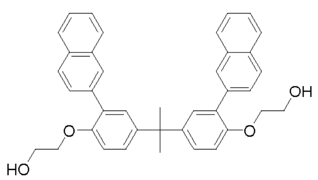
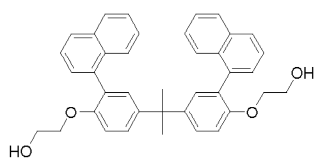
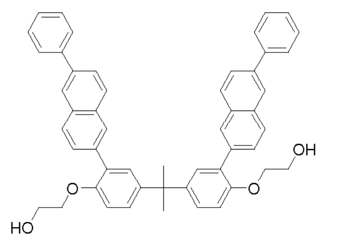

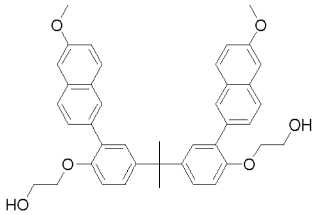
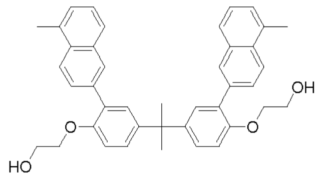
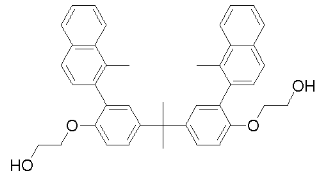
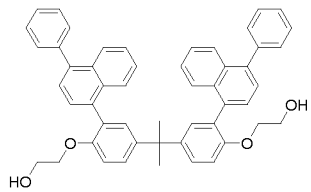
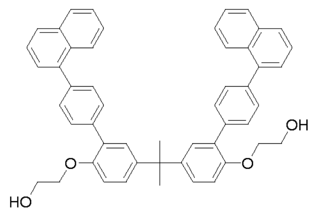
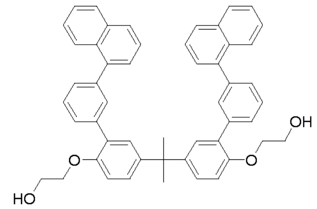
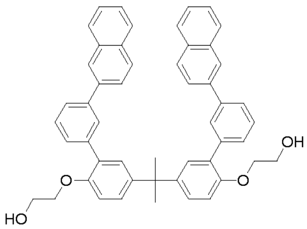
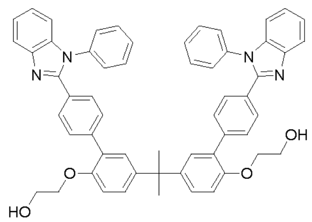
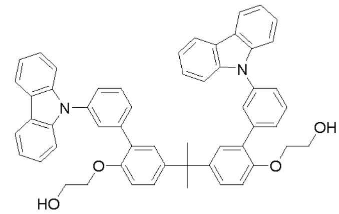
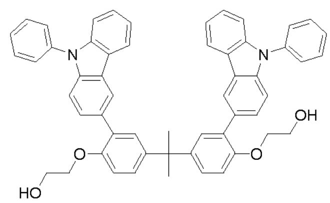
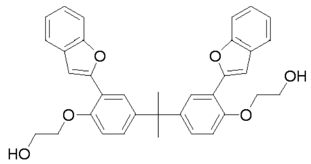
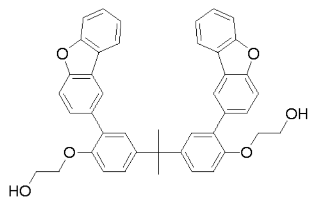
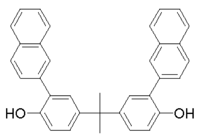
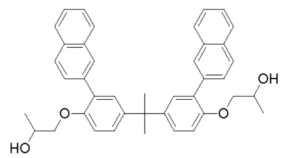
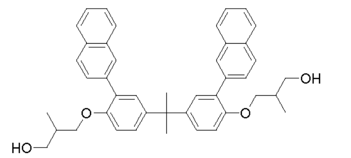
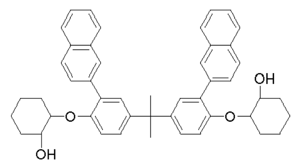
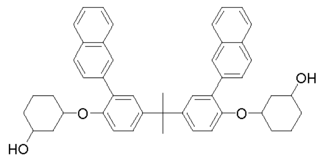
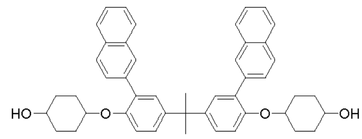
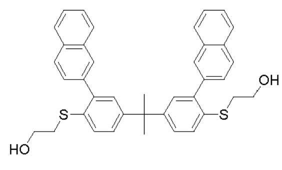
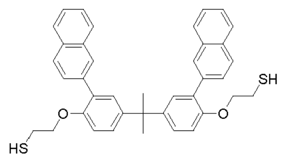

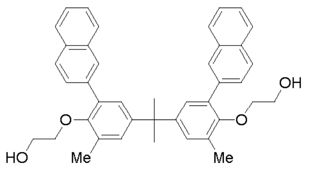
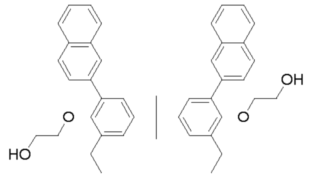
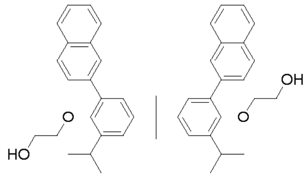

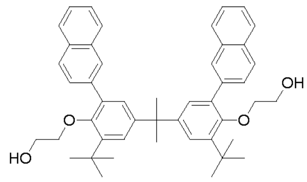
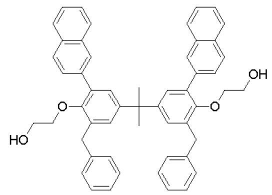

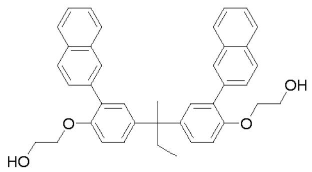
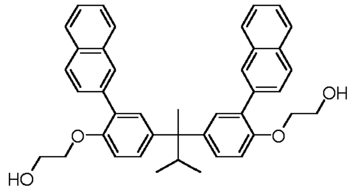
.

[Embodiment 23] The compound of embodiment 19, wherein Chemical Formula 5a is any one selected from the following compounds:

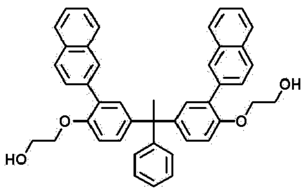
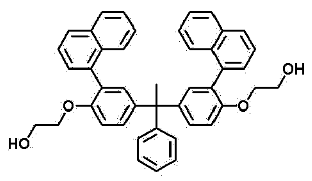
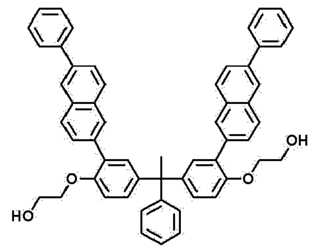

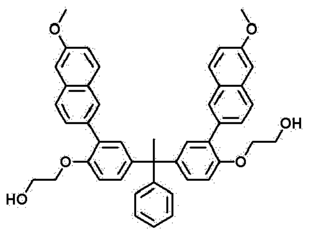
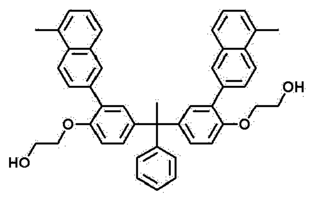
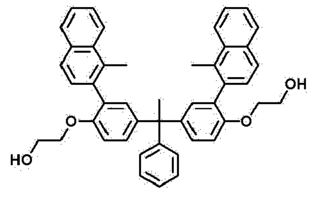

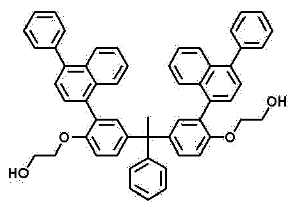
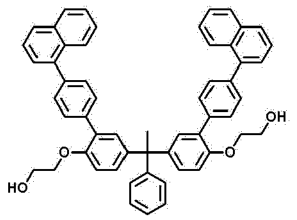
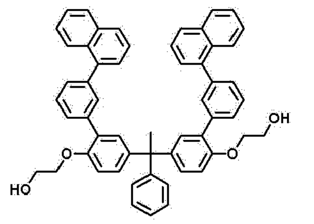

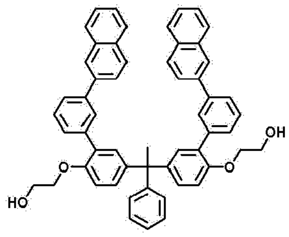
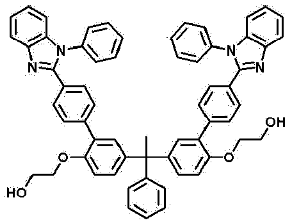
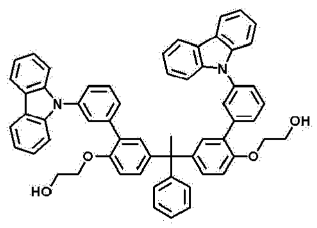

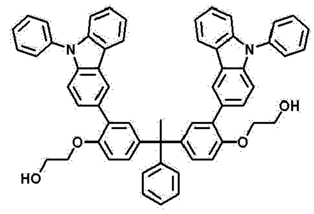
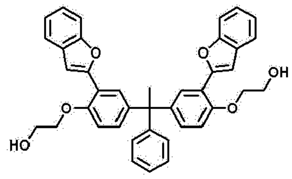
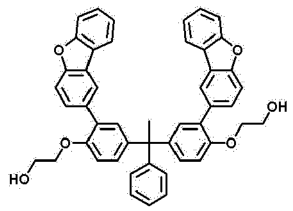

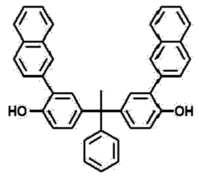
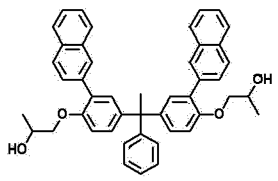
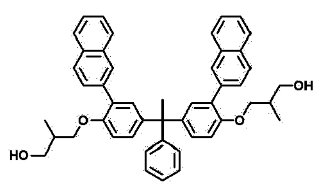

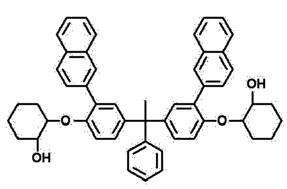
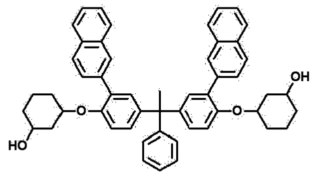
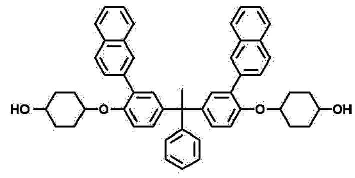

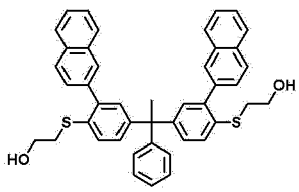
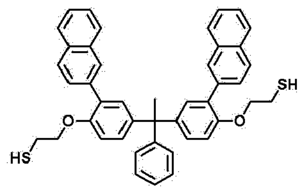
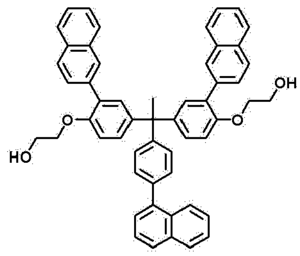

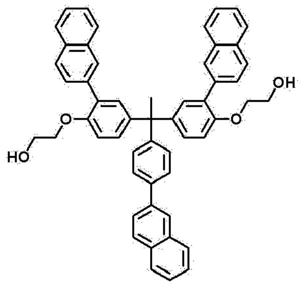
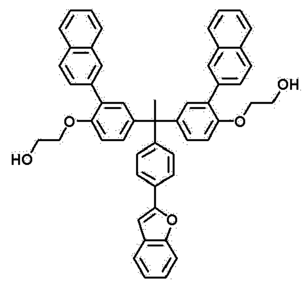
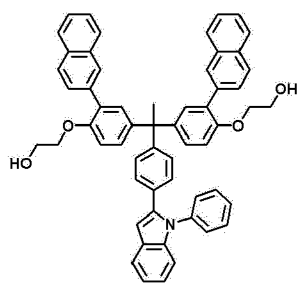

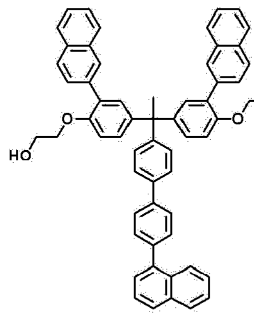

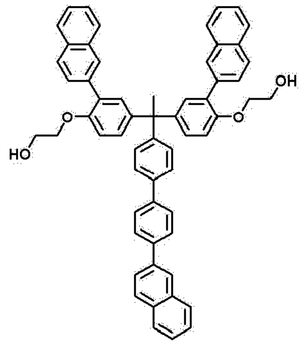

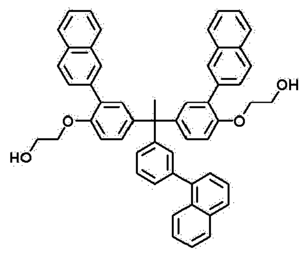

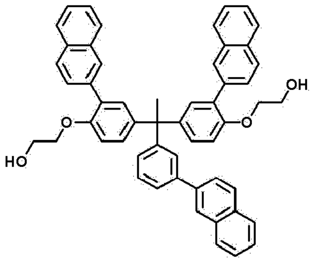

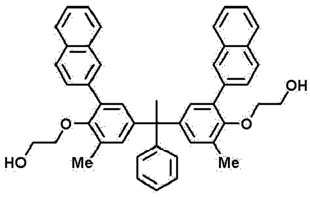

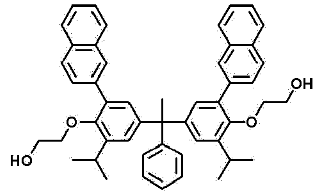

.

[Embodiment 24] A method for preparing the resin according to any one of embodiments 1 to 14, the method comprising:

polymerizing a composition for preparing a resin, which comprises a compound represented by the following Chemical Formula 1a; and a polyester precursor or a polycarbonate precursor:

[Chemical Formula 1a]

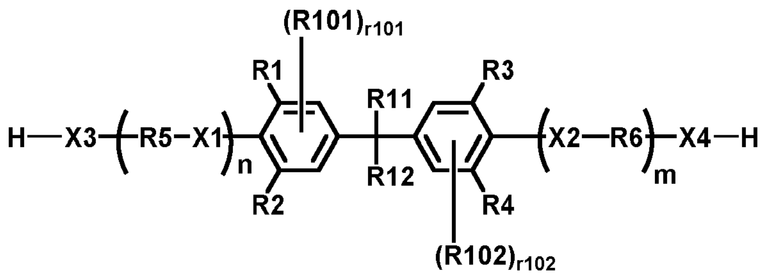

wherein, in Chemical Formula 1a,

X1 to X4 are the same as or different from each other, and are each independently O or S,

at least one of R1 to R4 is a substituted or unsubstituted alkyl group; a substituted or unsubstituted aryl group; or a substituted or unsubstituted heteroaryl group, and the others are each independently hydrogen; a substituted or unsubstituted alkyl group; a substituted or unsubstituted aryl group; or a substituted or unsubstituted heteroaryl group,

R5 and R6 are the same as or different from each other, and are each independently a substituted or unsubstituted alkylene group; or a substituted or unsubstituted cycloalkylene group,

R11 and R12 are the same as or different from each other, and are each independently a substituted or unsubstituted alkyl group; a substituted or unsubstituted cycloalkyl group; a substituted or unsubstituted aryl group; or a substituted or unsubstituted heteroaryl group,

R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,

r101 is 1 or 2, and when r101 is 2, two R101's are the same as or different from each other,

r102 is 1 or 2, and when r102 is 2, two R102's are the same as or different from each other, and

m and n are each 0 or 1.

[Embodiment 25] The method of embodiment 24, wherein the polyester precursor is the following Chemical Formula A, and the polycarbonate precursor is the following Chemical Formula B:

[Chemical Formula A]

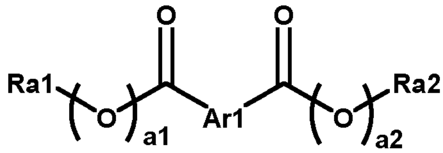

[Chemical Formula B]

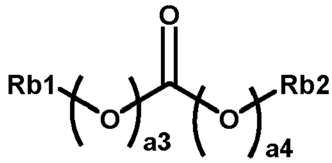

in Chemical Formulae A and B,

Ra1, Ra2, Rb1, and Rb2 are the same as or different from each other, and are each independently a halogen group; a substituted or unsubstituted alkyl group; or a substituted or unsubstituted aryl group,
Ar1 is a substituted or unsubstituted arylene group, and
a1 to a4 are each 0 or 1.

[Embodiment 26] A resin composition comprising the resin of any one of embodiments 1 to 14.
[Embodiment 27] A molded article comprising the resin composition of embodiment 26.
[Embodiment 28] The molded article of embodiment 27, wherein the molded article is an optical member.
[Embodiment 29] The molded article of embodiment 27, wherein the molded article is an optical lens.

## Claims

**1.** A resin comprising a unit of the following Chemical Formula 4:

[Chemical Formula 4]

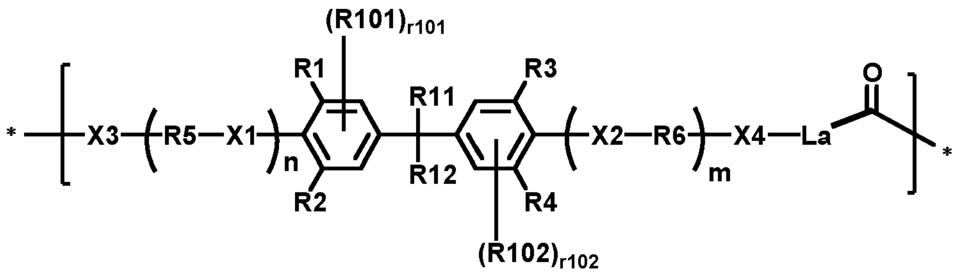

in Chemical Formula 4,

X1 to X4 are the same as or different from each other, and are each independently O or S,
R1 and R3 are the same as or different from each other, and are each independently a phenyl group which is substituted with one or more of a substituted or unsubstituted aryl group having 10 or more carbon atoms, a substituted or unsubstituted heteroaryl group, and a combination thereof; a substituted or unsubstituted aryl group having 10 or more carbon atoms; or a substituted or unsubstituted heteroaryl group,
R2 and R4 are the same as or different from each other, and are each independently hydrogen; or a substituted or

unsubstituted alkyl group,
R5 and R6 are the same as or different from each other, and are each independently a substituted or unsubstituted alkylene group; or a substituted or unsubstituted cycloalkylene group,
R11 and R12 are the same as or different from each other, and are each independently a substituted or unsubstituted alkyl group; or a substituted or unsubstituted cycloalkyl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other,
La is a direct bond; or -C(=O)-L'-,
L' is a substituted or unsubstituted arylene group,
m and n are each 0 or 1, and
* means a moiety linked to the main chain of the resin.

2. The resin of claim 1, wherein a refractive index of the resin, which is measured at a wavelength of 589 nm is 1.60 to 1.80, and
wherein the refractive index is determined as set out in the description.

3. The resin of claim 1, wherein the resin has a glass transition temperature (Tg) of 100°C to 260°C, and
wherein the glass transition temperature is determined as set out in the description.

4. The resin of claim 1, wherein the Abbe's Number of the resin, which is measured at a wavelength of 589 nm, 486 nm, and 656 nm, is 8 to 25, and
wherein the Abbe's Number is determined as set out in the description.

5. A compound of the following Chemical Formula 4a:

[Chemical Formula 4a]

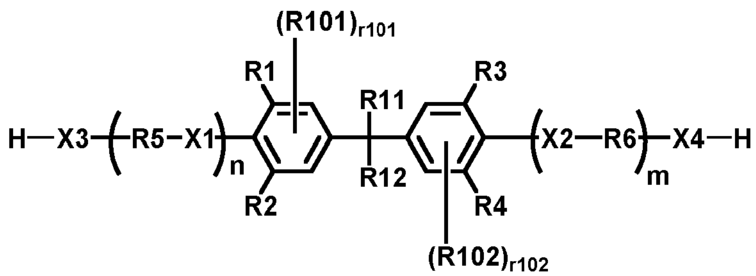

in Chemical Formula 4a,

X1 to X4 are the same as or different from each other, and are each independently O or S,
R1 and R3 are the same as or different from each other, and are each independently a phenyl group which is substituted with one or more of a substituted or unsubstituted aryl group having 10 or more carbon atoms, a substituted or unsubstituted heteroaryl group, and a combination thereof; a substituted or unsubstituted aryl group having 10 or more carbon atoms; or a substituted or unsubstituted heteroaryl group,
R2 and R4 are the same as or different from each other, and are each independently hydrogen; or a substituted or unsubstituted alkyl group,
R5 and R6 are the same as or different from each other, and are each independently a substituted or unsubstituted alkylene group; or a substituted or unsubstituted cycloalkylene group,
R11 and R12 are the same as or different from each other, and are each independently a substituted or unsubstituted alkyl group; or a substituted or unsubstituted cycloalkyl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other, and

m and n are each 0 or 1.

**6.** The compound of claim 5, wherein Chemical Formula 4a is any one selected from the following compounds:

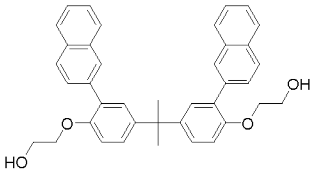

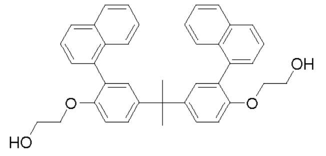

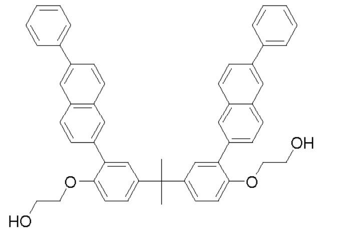

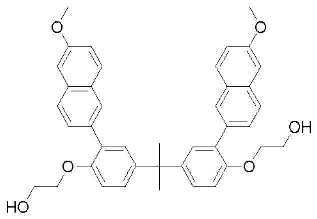

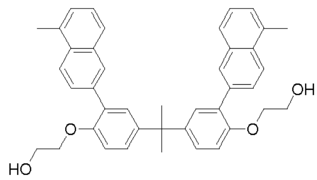

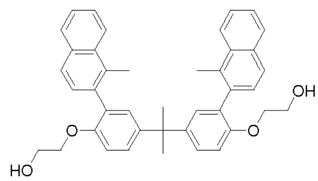

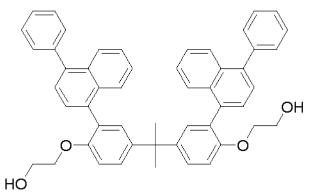

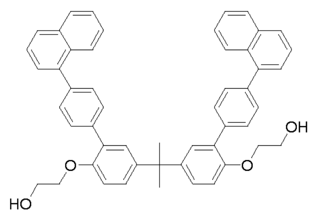

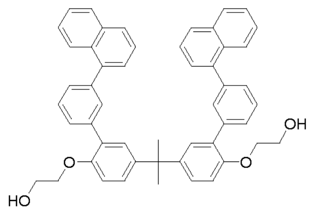

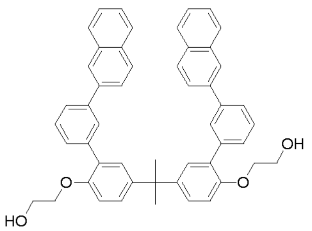

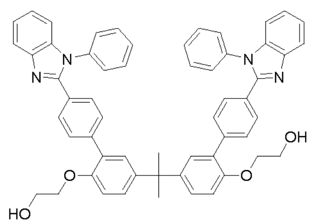

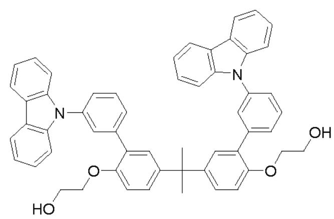

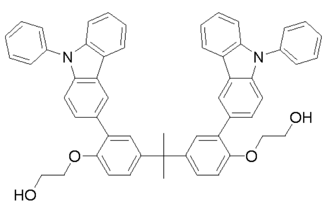

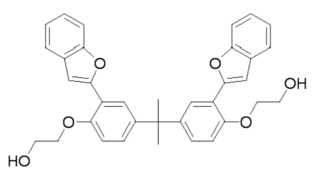

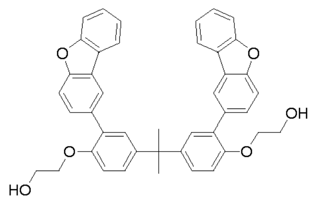

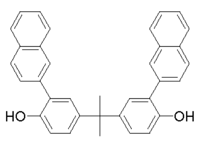

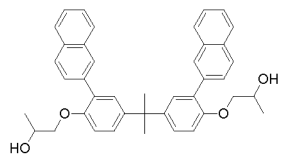

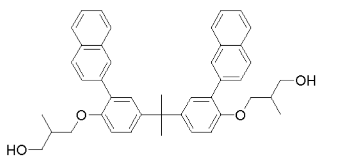

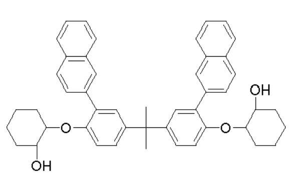
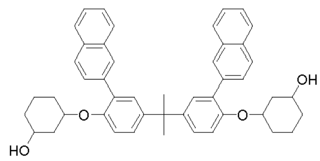
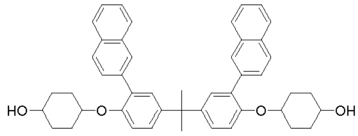

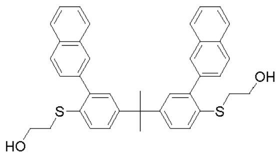
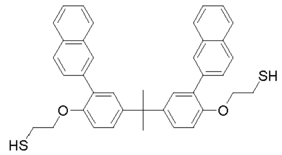

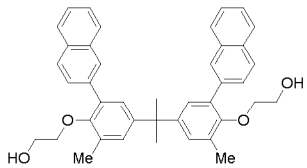
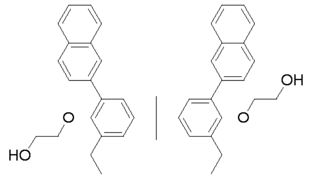
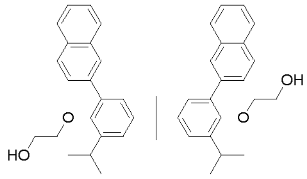

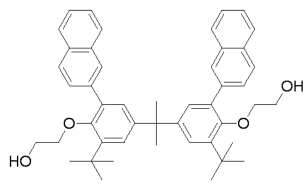
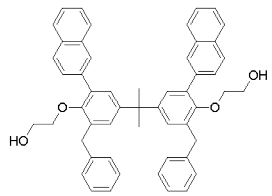

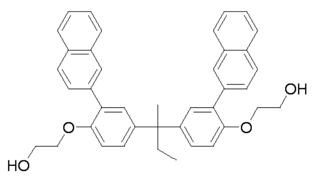
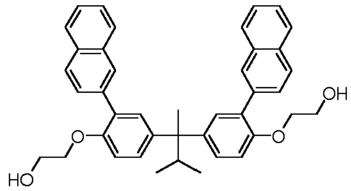
.

**7.** A method for preparing the resin according to any one of claims 1 to 4, the method comprising:

polymerizing a composition for preparing a resin, which comprises a compound represented by the following Chemical Formula 4a; and a polyester precursor or a polycarbonate precursor:

[Chemical Formula 4a]

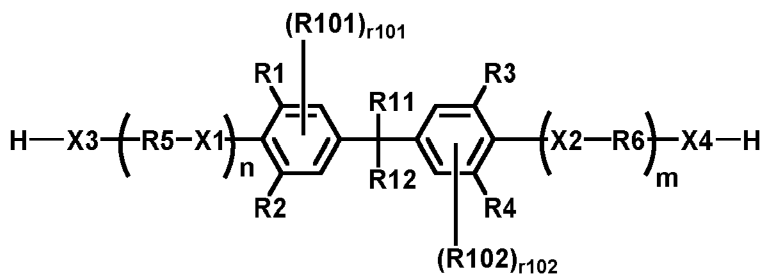

wherein, in Chemical Formula 4a,
X1 to X4 are the same as or different from each other, and are each independently O or S,
R1 and R3 are the same as or different from each other, and are each independently a phenyl group which is substituted with one or more of a substituted or unsubstituted aryl group having 10 or more carbon atoms, a substituted or unsubstituted heteroaryl group, and a combination thereof; a substituted or unsubstituted aryl

group having 10 or more carbon atoms; or a substituted or unsubstituted heteroaryl group,
R2 and R4 are the same as or different from each other, and are each independently hydrogen; or a substituted or unsubstituted alkyl group,
R5 and R6 are the same as or different from each other, and are each independently a substituted or unsubstituted alkylene group; or a substituted or unsubstituted cycloalkylene group,
R11 and R12 are the same as or different from each other, and are each independently a substituted or unsubstituted alkyl group; or a substituted or unsubstituted cycloalkyl group,
R101 and R102 are the same as or different from each other, and are each independently hydrogen; a substituted or unsubstituted alkyl group; a substituted or unsubstituted cycloalkyl group; a substituted or unsubstituted aryl group; or a substituted or unsubstituted heteroaryl group,
r101 is 1 or 2, and when r101 is 2, two R101's are the same as or different from each other,
r102 is 1 or 2, and when r102 is 2, two R102's are the same as or different from each other,
m and n are each 0 or 1.

**8.** The method of claim 7, wherein the polyester precursor is the following Chemical Formula A, and the polycarbonate precursor is the following Chemical Formula B:

[Chemical Formula A]

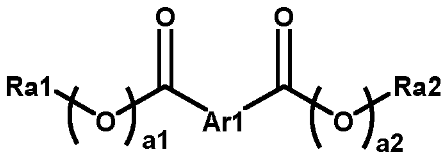

[Chemical Formula B]

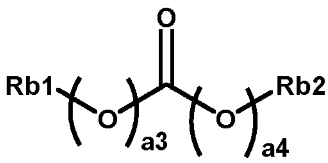

in Chemical Formulae A and B,

Ra1, Ra2, Rb1, and Rb2 are the same as or different from each other, and are each independently a halogen group; a substituted or unsubstituted alkyl group; or a substituted or unsubstituted aryl group,
Ar1 is a substituted or unsubstituted arylene group, and
a1 to a4 are each 0 or 1.

**9.** A resin composition comprising the resin of any one of claims 1 to 4.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020220082381
- KR 1020220082388
- KR 1020220082390
- KR 1020220082391